# EUROPÄISCHE PATENTSCHRIFT

(11) **EP 2 523 961 B1**
(45) Veröffentlichungstag und Bekanntmachung des Hinweises auf die Patenterteilung: **18.12.2013**
(21) Anmeldenummer: 11700403.6
(22) Anmeldetag: 12.01.2011
(51) Int. Cl.: C07D 498/04, A61K 31/519, A61P 9/00

(54) **2,5,7-SUBSTITUIERTE OXAZOLOPYRIMIDINDERIVATE**
2,5,7-SUBSTITUTED OXAZOLOPYRIMIDINE DERIVATIVES
DÉRIVÉS DE L'OXAZOLOPYRIMIDINE À SUBSTITUTION 2,5,7 EN TANT QU'AGONISTES DU RÉCEPTEUR EDG-1

(30) Priorität: 13.01.2010 EP 10305035
(43) Veröffentlichungstag der Anmeldung: 21.11.2012
(73) Patentinhaber: SANOFI, 75008 Paris (FR)
(72) Erfinder: KADEREIT, Dieter, 65926 Frankfurt am Main (DE); SCHAEFER, Matthias, 65926 Frankfurt am Main (DE); HACHTEL, Stephanie, 65926 Frankfurt am Main (DE); DIETRICH, Axel, 65926 Frankfurt am Main (DE); HUEBSCHLE, Thomas, 65926 Frankfurt am Main (DE); GILLE, Andreas, 65926 Frankfurt am Main (DE); HISS, Katrin, 65926 Frankfurt am Main (DE)
(74) Vertreter: Then, Johann
(86) Internationale Anmeldenummer: PCT/EP2011/050295
(87) Internationale Veröffentlichungsnummer: WO 2011/086075

(56) Entgegenhaltungen:
- WO-A1-2005/000833
- WO-A1-2009/154775
- WO-A2-2007/061458
- WO-A2-2007/109334

## Beschreibung

Die vorliegende Erfindung betrifft 2,5,7-Substituierte Oxazolopyrimidinderivate, sowie deren physiologisch akzeptable Salze.

Es sind bereits strukturähnliche Verbindungen im Stand der Technik beschrieben (siehe WO2009/154775), die zur Behandlung multipler Sklerose geeignet sind. Die Wirkungsweise dieser Verbindungen besteht darin, durch Aktivierung des EDG-1 Rezeptors eine Desensitisierung des EDG-1 Signalweges zu verursachen (sog. Superagonismus), der dann einem funktionellen Antagonismus des EDG-1-Signalweges gleichkommt. Systemisch bedeutet das, daß vor allem auf Lymphozyten der EDG-1 Signalweg dauerhaft unterdrückt wird, wodurch diese Zellen nicht mehr chemotaktisch dem S1 P Gradienten zwischen Blut und Lymphflüssigkeit folgen können. Dies bedingt, dass die betroffenen Lymphozyten nicht mehr das sekundäre lymphatische Gewebe verlassen können (verstärktes Homeing) und die Zahl der frei zirkulierenden Lymphozyten im Plasma stark gesenkt wird. Dieser Mangel an Lymphozyten im Plasma (Lymphopenie) bewirkt eine Immunsuppression, die zwingend für den Wirkmechanismus der in WO 2009/154775 beschriebenen EDG-1-Rezeptor-Modulatoren erforderlich ist.

Der vorliegenden Erfindung lag die Aufgabe zugrunde, Verbindungen zur Verfügung zustellen, die spezifisch zur Wundheilung und insbesondere zur Behandlung von Wundheilungsstörungen von Diabetes Patienten geeignet sind. Weiter war es wünschenswert Verbindungen zur Verfügung zustellen, die zur Behandlung des Diabetische Fußsyndroms (DFS) geeignet sind.
Weiter war es wünschenswert eine wiederholbare Aktivierung des EDG-1-Rezeptor Signalweges zu erreichen, was damit pharmakologisch eine persistente Aktivierung des EDG-1 Signalweges ermöglicht.

Die vorliegende Erfindung betrifft daher Oxazolopyrimidinverbindungen der Formel I. 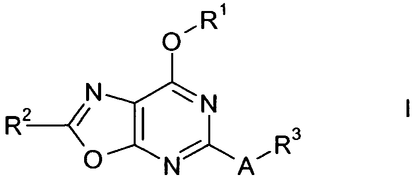 worin A, R¹, R² und R³ wie nachstehend definiert sind. Der Wirkmechanismus der Verbindungen der Formel I beruht also nicht auf Desensitisierung des EDG-1-Signalweges und steht somit dem in WO 2009/154775 beschriebenen Wirkmechanismus diametral gegenüber. Die Erfindung betrifft ferner Verfahren zur Herstellung von Verbindungen der Formel I, ihre Verwendung, insbesondere als Wirkstoff in Pharmazeutika, und pharmazeutische Zusammensetzungen, die sie enthalten.

Patienten mit Diabetes haben gegenüber gesunden Menschen eine verzögerte Wundheilung und eine erhöhte Infektionsrate, vor allem bei länger währender Hyperglykämie, zum Beispiel hervorgerufen durch eine schlechte Blutzuckereinstellung. Zu den Ursachen gehören Durchblutungsstörungen, vor allem im Bereich der kleinen Gefäße, die zu einer verschlechterten Sauerstoff- und Nährstoffversorgung des Gewebes führen. Außerdem besteht eine verminderte Zellteilungs- und Zellmigrationsrate von Keratinozyten, Fibroblasten und dermalen Endothelzellen. Zusätzlich ist die Aktivität verschiedener Abwehrzellen (Granulozyten) mit reduzierter Phagozytose (Aufnahme und Zerstörung von Bakterien) eingeschränkt. Auch die Funktion der Antikörper (Immunglobuline) gegen Bakterien ist bei hohen Blutzuckerwerten eingeschränkt. Dementsprechend müssen Wunden und Infektionen bei Diabetes-Patienten besonders versorgt werden.

Der Edg-1-Rezeptor gehört zur Familie der Edg-Rezeptoren (Edg = Endothelial Differentiation Gene) von gegenwärtig acht identifizierten GPCRs (G-Proteingekoppelten Rezeptoren) der Klasse A. Diese Familie kann in Unterfamilien von durch Sphingosin-1-Phosphat (S1P) aktivierten Rezeptoren (fünf Mitglieder) und durch Lysophosphatidsäure (LPA) aktivierte Rezeptoren (drei Mitglieder) unterteilt werden. Der endogene Ligand S1P ist ein pluripotentes Lysophospholipid, das durch Aktivierung von GPCRs aus der Edg-Rezeptorfamilie, nämlich Edg-1 (= S1P1), Edg-3 (= S1P3), Edg-5 (= S1P2), Edg-6 (= S1P4) und Edg-8 (S1P5), auf verschiedene Zelltypen wirkt. Wenngleich S1P auch als intrazellulärer Botenstoff beschrieben wird, werden zahlreiche zelluläre Antworten von S1 P über die Aktivierung von Edg-Rezeptoren - vermittelt werden. S1P wird durch die Enzymfamilie der Sphingosinkinasen (SPHK) erzeugt und durch verschiedene Phosphatasen oder Lyasen abgebaut.

Gegenstand der vorliegenden Erfindung ist eine Oxazolopyrimidinverbindung der Formel I in einer beliebigen ihrer stereoisomeren Formen oder einer Mischung von stereoisomeren Formen in beliebigem Verhältnis oder ein physiologisch akzeptables Salz davon oder ein physiologisch akzeptables Solvat einer derartigen Verbindung oder eines derartigen Salzes, 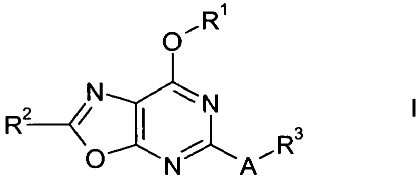 worin
A ausgewählt ist aus NH, O und S;
R¹ ausgewählt ist aus (C₁-C₆)-Alkyl, (C₂-C₆)-Alkenyl, (C₂-C₆)-Alkinyl, (C₃-C₇)-Cycloalkyl-CₜH₂ₜ- und Het-CₜH₂ₜ-, worin t aus 0, 1, 2 und 3 ausgewählt ist;
R² ausgewählt ist aus Phenyl und einem Rest eines aromatischen, 5-gliedrigen bis 6-gliedrigen monocyclischen Heterocyclus, der 1, 2 oder 3 gleiche oder verschiedene Ringheteroatome enthält, die aus N, O und S ausgewählt sind, wobei eines der Ringstickstoffatome ein Wasserstoffatom oder einen Substituenten R²¹ tragen kann und wobei das Phenyl und der Rest eines aromatischen Heterocyclus gegebenenfalls an einem oder mehreren Ringkohlenstoffatomen durch gleiche oder verschiedene Substituenten R²² substituiert sind;
R³ ausgewählt ist aus (C₁-C₆)-Alkyl, (C₂-C₆)-Alkenyl, (C₂-C₆)-Alkinyl, (C₃-C₇)-Cyclo-alkyl-CᵤH₂ᵤ- und Het-CᵥH₂ᵥ-, worin u und v aus 1 und 2 ausgewählt sind, oder R³ für einen Rest eines gesättigten oder ungesättigten, 3-gliedrigen bis 10-gliedrigen, monocyclischen oder bicyclischen Rings, der 0, 1, 2, 3 oder 4 gleiche oder verschiedene Ringheteroatome enthält, die aus N, O und S ausgewählt sind, steht, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist, mit der Maßgabe, daß R³ nicht für (C₁-C₆)-Alkyl stehen kann, wenn A für S steht;
R²¹ ausgewählt ist aus (C₁-C₄)-Alkyl, (C₃-C₇)-Cycloalkyl-C_{w}H_{2w}- und Oxy, wobei w aus 0, 1 und 2 ausgewählt ist;
R²² ausgewählt ist aus Halogen, Hydroxy, (C₁-C₄)-Alkyl-, (C₁-C₄)-Alkyloxy, (C₁-C₄)-Alkyl-S(O)ₘ-, Amino, Nitro, Cyano, Hydroxycarbonyl, (C₁-C₄)-Alkyloxycarbonyl, Aminocarbonyl, Aminosulfonyl, R²³ und R²³-O-;
R²³ für einen Rest eines gesättigten, 3-gliedrigen bis 7-gliedrigen, monocyclischen oder bicyclischen Rings, der 0, 1, 2, 3 oder 4 gleiche oder verschiedene Ringheteroatome enthält, die aus N, O und S ausgewählt sind, steht, wobei ein oder zwei der Ringstickstoffatome ein Wasserstoffatom oder einen (C₁-C₄)-Alkylsubstituenten tragen können und eines der Ringschwefelatome eine oder zwei Oxogruppen tragen kann und wobei der Rest eines Rings gegebenenfalls an einem oder mehreren Ringkohlenstoffatomen durch gleiche oder verschiedene Substituenten R²⁴ substituiert ist;
R²⁴ ausgewählt ist aus Halogen, (C₁-C₄)-Alkyl, Hydroxy und Oxo;
R³¹ ausgewählt ist aus Halogen, (C₁-C₄)-Alkyl, (C₃-C₇)-Cycloalkyl, Hydroxy, (C₁-C₄)-Alkyloxy, Oxo, (C₁-C₄)-Alkyl-S(O)ₘ-, Amino, (C₁-C₄)-Alkylamino, Di((C₁-C₄)-alkyl)amino, (C₁-C₄)-Alkylcarbonylamino, (C₁-C₄)-Alkylsulfonylamino, Nitro, Cyano, (C₁-C₄)-Alkylcarbonyl, Aminosulfonyl, (C₁-C₄)-Alkylaminosulfonyl und Di((C₁-C₄)-alkyl)aminosulfonyl;
Het für einen Rest eines gesättigten, 4-gliedrigen bis 7-gliedrigen, monocyclischen Heterocyclus, der 1 oder 2 gleiche oder verschiedene Ringheteroatome enthält, die unter N, O und S ausgewählt sind, und der über ein Ringkohlenstoffatom gebunden ist, steht, wobei der Rest eines Heterocyclus gegebenenfalls durch einen oder mehrere gleiche oder verschiedene Substituenten substituiert ist, die unter Fluor und (C₁-C₄)-Alkyl ausgewählt sind;
m aus 0, 1 und 2 ausgewählt ist, wobei alle Zahlen m voneinander unabhängig sind;
wobei alle Cycloalkylgruppen unabhängig voneinander und unabhängig von anderen Substituenten gegebenenfalls durch einen oder mehrere gleiche oder verschiedene Substituenten substituiert sind, die unter Fluor und (C₁-C₄)-Alkyl ausgewählt sind;
wobei alle Alkyl-, CₜH₂ₜ-, CᵤH₂ᵤ-, CᵥH₂ᵥ-, C_{w}H_{2w}-, Alkenyl- und Alkinylgruppen unabhängig voneinander und unabhängig von anderen Substituenten gegebenenfalls durch einen oder mehrere Fluorsubstituenten substituiert sind.

Strukturelemente wie Gruppen, Substituenten, Heteroringglieder, Zahlen oder andere Merkmale, beispielsweise Alkylgruppen, Gruppen wie R²² oder R³¹, Zahlen wie m, u und v, die in den Verbindungen der Formel I mehrmals vorkommen können, können alle unabhängig voneinander eine beliebige der angegebenen Bedeutungen besitzen und in jedem Fall gleich oder voneinander verschieden sein. Beispielsweise können die Alkylgruppen in einer Dialkylaminogruppe gleich oder verschieden sein.

Alkyl-, Alkenyl- und Alkinylgruppen können linear, d.h. geradkettig, oder verzweigt sein. Dies gilt auch, wenn sie Teil anderer Gruppen, beispielsweise Alkyloxygruppen (= Alkoxygruppen, Alkyl-O-Gruppen), Alkyloxycarbonylgruppen oder alkylsubstituierter Aminogruppen, sind oder wenn sie substituiert sind. Je nach der jeweiligen Definition kann die Zahl der Kohlenstoffatome in einer Alkylgruppe 1, 2, 3, 4, 5 oder 6 oder 1, 2, 3 oder 4 oder 1, 2 oder 3 betragen. Beispiele für Alkyl sind Methyl, Ethyl, Propyl einschließlich n-Propyl und Isopropyl, Butyl, einschließlich n-Butyl, sek.-Butyl, Isobutyl und tert.-Butyl, Pentyl einschließlich n-Pentyl, 1-Methylbutyl, Isopentyl, Neopentyl und tert.-Pentyl und Hexyl einschließlich n-Hexyl, 3,3-Dimethylbutyl und Isohexyl. Doppelbindungen und Dreifachbindungen in Alkenylgruppen und Alkinylgruppen können in beliebigen Positionen vorliegen. In einer Ausführungsform der Erfindung enthalten Alkenylgruppen eine Doppelbindung und Alkinylgruppen eine Dreifachbindung. In einer Ausführungsform der Erfindung enthält eine Alkenylgruppe oder Alkinylgruppe mindestens drei Kohlenstoffatome und ist über ein Kohlenstoffatom, das nicht Teil einer Doppelbindung oder Dreifachbindung ist, an den Rest des Moleküls gebunden. Beispiele für Alkenyl und Alkinyl sind Ethenyl, Prop-1-enyl, Prop-2-enyl (= Allyl), But-2-enyl, 2-Methylprop-2-enyl, 3-Methylbut-2-enyl, Hex-3-enyl, Hex-4-enyl, Prop-2-inyl (= Propargyl), But-2-inyl, But-3-inyl, Hex-4-inyl oder Hex-5-inyl. Substituierte Alkylgruppen, Alkenylgruppen und Alkinylgruppen können in beliebigen Positionen substituiert sein, mit der Maßgabe, daß die jeweilige Verbindung ausreichend stabil und für den gewünschten Zweck wie die Verwendung als Arzneistoff geeignet ist. Die Voraussetzung, daß eine spezifische Gruppe und eine Verbindung der Formel I ausreichend stabil und für den gewünschten Zweck wie die Verwendung als Arzneistoff geeignet sind, gilt allgemein in bezug auf die Definitionen aller Gruppen in den Verbindungen der Formel I.

Soweit anwendbar, gelten die vorstehenden Erklärungen bezüglich Alkylgruppen entsprechend für zweiwertige Alkylgruppen wie die Gruppen CₜH₂ₜ, CᵤH₂ᵤ, CᵥH₂ᵥ und C_{w}H_{2w}, die somit ebenfalls linear und verzweigt sein können. Beispiele für zweiwertige Alkylgruppen sind -CH₂- (= Methylen), -CH₂-CH₂-, -CH₂-CH₂-CH₂-, -CH(CH₃)-, -C(CH₃)₂-, -CH(CH₃)-CH₂-, -CH₂-CH(CH₃)-. Wenn eine Zahl in einer zweiwertigen Gruppe, wie beispielsweise die Zahl t in der Gruppe CₜH₂ₜ, für 0 (= null) steht, sind die beiden Gruppen, die an die in Rede stehende Gruppe, wie CₜH₂ₜ, gebunden sind, über eine Einfachbindung direkt miteinander verbunden.

Die Zahl der Ringkohlenstoffatome in einer Cycloalkylgruppe kann 3, 4, 5, 6 oder 7 betragen. In einer Ausführungsform der Erfindung beträgt die Zahl der Ringkohlenstoffatome in einer Cycloalkylgruppe unabhängig von der Zahl der Ringkohlenstoffatome in einer anderen Cycloalkylgruppe 3, 4, 5 oder 6, in einer anderen Ausführungsform 3, 4 oder 5, in einer anderen Ausführungsform 3 oder 4, in einer anderen Ausführungsform 3, in einer anderen Ausführungsform 5, 6 oder 7, in einer anderen Ausführungsform 5 oder 6, in einer anderen Ausführungsform 6 oder 7, in einer anderen Ausführungsform 6. Beispiele für Cycloalkylgruppen sind Cyclopropyl, Cyclobutyl, Cyclopentyl, Cyclohexyl und Cycloheptyl. Unabhängig voneinander und unabhängig von anderen Substituenten sind Cycloalkylgruppen gegebenenfalls durch einen oder mehrere gleiche oder verschiedene (C₁-C₄)-Alkylsubstituenten substituiert, die in beliebigen Positionen stehen können, d.h. Cycloalkylgruppen können durch Alkylsubstituenten unsubstituiert oder durch Alkylsubstituenten, beispielsweise 1, 2, 3 oder 4 oder 1 oder 2 (C₁-C₄)-Alkylsubstituenten, beispielsweise Methylgruppen, substituiert sein. Beispiele für alkylsubstituierte Cycloalkylgruppen sind 4-Methylcyclohexyl, 4-tert.-Butylcyclohexyl oder 2,3-Dimethylcyclopentyl. Beispiele für Cycloalkylalkylgruppen, die beispielsweise Gruppen wie (C₃-C₇)-Cycloalkyl-CₜH₂ₜ-repräsentieren können, sind Cyclopropylmethyl, Cyclobutylmethyl, Cyclopentylmethyl, Cyclohexylmethyl, Cycloheptylmethyl, 1-Cyclopropylethyl, 2-Cyclopropylethyl, 1-Cyclobutylethyl, 2-Cyclobutylethyl, 2-Cyclopentylethyl, 2-Cyclohexylethyl, 2-Cycloheptylethyl.

Unabhängig voneinander und unabhängig von anderen Substituenten sind Alkylgruppen, zweiwertige Alkylgruppen, Alkenylgruppen, Alkinylgruppen und Cycloalkylgruppen gegebenenfalls durch einen oder mehrere Fluorsubstituenten substituiert, die in beliebigen Positionen stehen können, d.h. diese Gruppen können durch Fluorsubstituenten unsubstituiert oder durch Fluorsubstituenten, beispielsweise 1, 2, 3, 4, 5, 6, 7, 8, 9, 10, 11, 12 oder 13 oder 1, 2, 3, 4, 5, 6, 7, 8 oder 9 oder 1, 2, 3, 4, 5, 6 oder 7 oder 1, 2, 3, 4 oder 5 oder 1, 2 oder 3 oder 1 oder 2 Fluorsubstituenten, substituiert sein. Beispiele für fluorsubstituierte derartige Gruppen sind Trifluormethyl, 2-Fluorethyl, 2,2,2-Trifluorethyl, Pentafluorethyl, 3,3,3-Trifluorpropyl, 2,2,3,3,3-Pentafluorpropyl, 4,4,4-Trifluorbutyl, Heptafluorisopropyl, -CHF-, -CF₂-, -CF₂-CH₂-, -CH₂-CF₂-, -CF₂-CF₂-, -CF(CH₃)-, -C(CF₃)₂-, 1-Fluorcyclopropyl, 2,2-Difluorcyclopropyl, 3,3-Difluorcyclobutyl, 1-Fluorcyclohexyl, 4,4-Difluorcyclohexyl, 3,3,4,4,5,5-Hexafluorcyclohexyl. Beispiele für Alkyloxygruppen, in denen die Alkylgruppierung fluorsubstituiert ist, sind Trifluormethoxy, 2,2,2-Trifluorethoxy, Pentafluorethoxy und 3,3,3-Trifluorpropoxy. In einer Ausführungsform der Erfindung beträgt die Gesamtzahl der Fluorsubstituenten und (C₁-C₄)-Alkylsubstituenten, die unabhängig von anderen Substituenten gegebenenfalls an Cycloalkylgruppen in den Verbindungen der Formel I vorliegen, 1, 2, 3, 4, 5, 6, 7, 8, 9, 10 oder 11, in einer anderen Ausführungsform 1, 2, 3, 4, 5, 6, 7, 8 oder 9, in einer anderen Ausführungsform 1, 2, 3, 4 oder 5, in einer anderen Ausführungsform 1, 2, 3 oder 4.

Gruppen wie Phenyl, Naphthyl (= Naphthalinyl) und Reste von aromatischen Heterocyclen, die gegebenenfalls durch einen oder mehrere Substituenten substituiert sind, können unsubstituiert oder substituiert sein, beispielsweise durch 1, 2, 3, 4 oder 5 oder 1, 2, 3 oder 4 oder 1, 2 oder 3 oder 1 oder 2 oder 1 gleiche oder verschiedene Substituenten, die in beliebigen Positionen stehen können. In einer Ausführungsform der Erfindung ist die Gesamtzahl der Nitrosubstituenten in einer Verbindung der Formel I nicht größer als zwei. Aromatische Stickstoffheterocyclen, die in dem zugrundeliegenden Ringsystem ein Wasserstoffatom an einem Ringstickstoffatom in einem 5-gliedrigen Ring, wie beispielsweise einem Pyrrol-, Imidazol-, Indol- oder Benzoimidazolring, tragen, können an den Kohlenstoffatomen und/oder an derartigen Ringstickstoffatomen substituiert sein. In einer Ausführungsform der Erfindung sind Substituenten an derartigen Ringstickstoffatomen aus (C₁-C₄)-Alkylgruppen ausgewählt, d.h. derartige Ringstickstoffatome in aromatischen Heterocyclen tragen ein Wasserstoffatom oder einen (C₁-C₄)-Alkylsubstituenten. Wenn bezüglich Ringstickstoffatomen in aromatischen Heterocyclen und anderen Heterocyclen angegeben ist, daß sie ein Wasserstoffatom oder einen Substituenten tragen können, so tragen derartige Ringstickstoffatome entweder ein Wasserstoffatom oder einen Substituenten oder nicht. Ringstickstoffatome, die ein Wasserstoffatom oder einen Substituenten tragen, kommen in einem stickstoffhaltigen aromatischen 5-gliedrigen Ring, wie er beispielsweise in Pyrrol, Imidazol, Indol oder Benzoimidazol vorliegt, und in einem nichtaromatischen Ring einschließlich eines gesättigten Rings vor. Ringstickstoffatome, die kein Wasserstoffatom oder keinen Substituenten tragen, sofern sie nicht in positiv geladener Form vorliegen, einschließlich weiterer Ringstickstoffatome neben den Ringstickstoffatomen, die ein Wasserstoffatom oder einen Substituenten tragen, kommen in einem aromatischen Ring, wie er beispielsweise in Thiazol, Imidazol, Pyridin oder Benzoimidazol vorliegt, und in einem nichtaromatischen Ring, in dem sie Brückenkopfatome oder Teil einer Doppelbindung sind, und als Ringstickstoffatome, über die ein Ring gebunden ist, vor. Geeignete Ringstickstoffatome in aromatischen Heterocyclen in den Verbindungen der Formel I, wie das Ringstickstoffatom in einem Pyridinring, spezifisch ein Ringstickstoffatom in einem aromatischen Heterocyclus, der R² repräsentiert, können auch einen Oxysubstituenten -O⁻ tragen und als N-Oxid vorliegen, und derartige Ringstickstoffatome können auch als quartäres Salz, beispielsweise als N-(C₁-C₄)-Alkylsalz wie N-Methylsalz, vorliegen, wobei in einer Ausführungsform der Erfindung das Gegenanion in einem derartigen quartären Salz ein physiologisch akzeptables Anion ist, das sich von einer Säure, die ein physiologisch akzeptables Salz bildet, ableitet. In monosubstituierten Phenylgruppen kann der Substituent in der 2-Position, der 3-Position oder der 4-Position stehen. In disubstituierten Phenylgruppen können die Substituenten in 2,3-Position, 2,4-Position, 2,5-Position, 2,6-Position, 3,4-Position oder 3,5-Position stehen. In trisubstituierten Phenylgruppen können die Substituenten in 2,3,4-Position, 2,3,5-Position, 2,3,6-Position, 2,4,5-Position, 2,4,6-Position oder 3,4,5-Position stehen. Naphthyl kann 1-Naphthyl (= Naphthalin-1-yl) oder 2-Naphthyl (= Naphthalin-2-yl) sein. In monosubstituierten 1-Naphthylgruppen kann der Substituent in der 2-, 3-, 4-, 5-, 6-, 7- oder 8-Position stehen. In monosubstituierten 2-Naphthylgruppen kann der Substituent in der 1-, 3-, 4-, 5-, 6-, 7- oder 8-Position stehen. In disubstituierten Naphthylgruppen können die Substituenten ebenfalls in beliebigen Positionen sowohl in dem Ring, über den die Naphthylgruppe gebunden ist, und/oder in dem anderen Ring stehen.

In R² oder R³ repräsentierenden Resten von aromatischen Heterocyclen, die als Heteroarylgruppen bezeichnet sein können, sowie in allen anderen heterocyclischen Ringen in den Verbindungen der Formel I einschließlich der Gruppe Het und der nichtaromatischen heterocyclischen Gruppen, die R³ repräsentieren, sind die Ringheteroatome im allgemeinen aus N, O und S ausgewählt, wobei N Ringstickstoffatome, die ein Wasserstoffatom oder einen Substituenten tragen, sowie Ringstickstoffatome, die kein Wasserstoffatom und keinen Substituenten tragen, einschließt. Ringheteroatome können in beliebigen Positionen stehen, vorausgesetzt, daß das heterocyclische System in der Technik bekannt und stabil und als Untergruppe für den gewünschten Zweck der Verbindung der Formel I wie die Verwendung als Arzneistoff geeignet ist. In einer Ausführungsform der Erfindung können zwei Ringsauerstoffatome nicht in benachbarten Ringpositionen eines Heterocyclus stehen, in einer anderen Ausführungsform können zwei Ringheteroatome, die aus Sauerstoff und Schwefel ausgewählt sind, nicht in benachbarten Ringpositionen eines beliebigen Heterocyclus stehen. Gesättigte Ringe enthalten keine Doppelbindung im Ring. Ungesättigte Ringsysteme können aromatisch oder teilweise ungesättigt einschließlich teilweise aromatisch sein, wobei in letzterem Fall ein Ring in einem bicyclischen Ringsystem aromatisch ist und das Ringsystem über ein Atom im nichtaromatischen Ring gebunden ist. Je nach der jeweiligen Gruppe können ungesättigte Ringe eine, zwei, drei, vier oder fünf Doppelbindungen im Ring enthalten. Aromatische Gruppen enthalten ein cyclisches System von sechs oder zehn delokalisierten pi-Elektronen im Ring. Je nach der jeweiligen Gruppe können gesättigte und nichtaromatisch ungesättigte heterocyclische Ringe einschließlich Het und nichtaromatischen Gruppen, die R³ repräsentieren, 3-gliedrig, 4-gliedrig, 5-gliedrig, 6-gliedrig, 7-gliedrig, 8-gliedrig, 9-gliedrig oder 10-gliedrig sein. In einer Ausführungsform der Erfindung sind aromatische heterocyclische Ringe 5-gliedrige oder 6-gliedrige monocyclische Ringe oder 8-gliedrige, 9-gliedrige oder 10-gliedrige bicyclische Ringe, in einer anderen Ausführungsform 5-gliedrige oder 6-gliedrige monocyclische Ringe oder 9-gliedrige oder 10-gliedrige bicyclische Ringe, in einer anderen Ausführungsform 5-gliedrige oder 6-gliedrige monocyclische Ringe, wobei die 8-gliedrigen, 9-gliedrigen oder 10-gliedrigen bicyclischen Ringe aus zwei anellierten 5-gliedrigen Ringen, einem 5-gliedrigen Ring und einem 6-gliedrigen Ring, die miteinander anelliert sind, bzw. zwei anellierten 6-gliedrigen Ringen zusammengesetzt sind. In bicyclischen aromatischen heterocyclischen Gruppen können ein oder beide Ringe Heteroringglieder enthalten, und ein oder beide Ringe können aromatisch sein. Im allgemeinen werden bicyclische Ringsysteme mit einem aromatischen Ring und einem nichtaromatischen Ring als aromatisch erachtet, wenn sie über ein Kohlenstoffatom im aromatischen Ring gebunden sind, und als nichtaromatisch, wenn sie über ein Kohlenstoffatom im nichtaromatischen Ring gebunden sind. Sofern nicht anders angegeben, können heterocyclische Gruppen einschließlich aromatischer heterocyclischer Gruppen über ein beliebiges geeignetes Ringkohlenstoffatom und im Fall von Stickstoffheterocyclen über ein beliebiges geeignetes Ringstickstoffatom gebunden sein. In einer Ausführungsform der Erfindung ist eine aromatische heterocyclische Gruppe in einer Verbindung der Formel I unabhängig von jeder anderen aromatischen heterocyclischen Gruppe über ein Ringkohlenstoffatom gebunden, in einer anderen Ausführungsformen über ein Ringstickstoffatom. Je nach der Definition der jeweiligen heterocyclischen Gruppe beträgt in einer Ausführungsform der Erfindung die Zahl der Ringheteroatome, die in einer heterocyclischen Gruppe unabhängig von der Zahl von Ringheteroatomen in einer anderen heterocyclischen Gruppe vorliegen kann, 1, 2, 3 oder 4, in einer anderen Ausführungsform 1,2 oder 3, in einer anderen Ausführungsform 1 oder 2, in einer anderen Ausführungsform 1, wobei die Ringheteroatome gleich oder verschieden sein können. Heterocyclische Gruppen, die gegebenenfalls substituiert sind, können unabhängig von jeder anderen heterocyclischen Gruppe unsubstituiert oder durch einen oder mehrere gleiche oder verschiedene Substituenten, beispielsweise 1, 2, 3, 4 oder 5 oder 1, 2, 3 oder 4 oder 1, 2 oder 3 oder 1 oder 2 oder 1 Substituenten, die bei der Definition der jeweiligen Gruppe angegeben sind, substituiert sein. Substituenten an heterocyclischen Gruppen können in beliebigen Positionen stehen. So können Substituenten in einer Pyridin-2-ylgruppe beispielsweise in der 3-Position und/oder 4-Position und/oder 5-Position und/oder 6-Position stehen, in einer Pyridin-3-ylgruppe in der 2-Position und/oder 4-Position und/oder 5-Postion und/oder 6-Position stehen und in einer Pyridin-4-ylgruppe in der 2-Position und/oder 3-Position und/oder 5-Position und/oder 6-Position stehen. Beispiele für Grundkörper von Heterocyclen, von denen sich heterocyclische Gruppen einschließlich aromatischer heterocyclischer Gruppen, gesättigter heterocyclischer Gruppen und nichtaromatischer ungesättigter heterocyclischer Gruppen ableiten können, sind Azet, Oxet, Pyrrol, Furan, Thiophen, Imidazol, Pyrazol, [1,3]Dioxol, Oxazol (= [1,3]Oxazol), Isoxazol (= [1,2]Oxazol), Thiazol (= [1,3]Thiazol), Isothiazol (= [1,2]Thiazol), [1,2,3]Triazol, [1,2,4]Triazol, [1,2,4]Oxadiazol, [1,3,4]Oxadiazol, [1,2,4]Thiadiazol, [1,3,4]Thiadiazol, Tetrazol, Pyridin, Pyran, Thiopyran, Pyridazin, Pyrimidin, Pyrazin, [1,3]Oxazin, [1,4]Oxazin, [1,3]Thiazin, [1,4]Thiazin, [1,2,3]Triazin, [1,3]Dithiin, [1,4]Dithiin, [1,2,4]Triazin, [1,3,5]Triazin, [1,2,4,5]Tetrazin, Azepin, [1,3]Diazepin, [1,4]Diazepin, [1,3]Oxazepin, [1,4]Oxazepin, [1,3]Thiazepin, [1,4]Thiazepin, Azocin, Azecin, Cyclopenta[b]pyrrol, 2-Azabicyclo[3.1.0]hexan, 3-Azabicyclo[3.1.0]hexan, 2-Oxa-5-azabicyclo[2.2.1]heptan, Indol, Isoindol, Benzothiophen, Benzofuran, [1,3]Benzodioxol (= 1,2-Methylendioxybenzol), [1,3]Benzoxazol, [1,3]Benzothiazol, Benzoimidazol, Thieno[3,2-c]pyridin, Chromen, Isochromen, [1,4]Benzodioxin, [1,4]Benzoxazin, [1,4]Benzothiazin, Chinolin, Isochinolin, Cinnolin, Chinazolin, Chinoxalin, Phthalazin, Thienothiophen, [1,8]Naphthyridin und andere Naphtyridine, Pteridin und die jeweiligen gesättigten und teilweise ungesättigten Heterocyclen, in denen eine oder mehrere, beispielsweise eine, zwei, drei, vier oder alle Doppelbindungen im Ringsystem einschließlich der Doppelbindungen im aromatischen Ring durch Einfachbindungen ersetzt sind, wie beispielsweise Azetidin, Oxetan, Pyrrolidin, Tetrahydrofuran, Tetrahydrothiophen, Imidazolidin, Oxazolidin, Thiazolidin, Dihydropyridin, Piperidin, Tetrahydropyran, Piperazin, Morpholin, Thiomorpholin, Azepan, Chroman, Isochroman, [1,4]Benzodioxan (= 1,2-Ethylendioxybenzol), 2,3-Dihydrobenzofuran, 1,2,3,4-Tetrahydrochinolin, 1,2,3,4-Tetrahydroisochinolin.

Beispiele für Reste von aromatischen Heterocyclen, die in den Verbindungen der Formel I vorkommen können, sind Thiophenyl (= Thienyl) einschließlich Thiophen-2-yl und Thiophen-3-yl, Pyridinyl (= Pyridyl) einschließlich Pyridin-2-yl (= 2-Pyridyl), Pyridin-3-yl (= 3-Pyridyl) und Pyridin-4-yl (= 4-Pyridyl), Imidazolyl einschließlich beispielsweise 1H-Imidazol-1-yl, 1H-Imidazol-2-yl, 1H-Imidazol-4-yl und 1H-Imidazol-5-yl, [1,2,4]Triazolyl einschließlich 1H-[1,2,4]-Triazol-1-yl und 4H-[1,2,4]-Triazol-3-yl, Tetrazolyl einschließlich 1 H-Tetrazol-1-yl und 1H-Tetrazol-5-yl, Chinolinyl (= Chinolyl) einschließlich Chinolin-2-yl, Chinolin-3-yl, Chinolin-4-yl, Chinolin-5-yl, Chinolin-6-yl, Chinolin-7-yl und Chinolin-8-yl, die alle gegebenenfalls wie in der Definition der jeweiligen Gruppe angegeben substituiert sind. Beispiele für Reste von gesättigten und teilweise ungesättigten Heterocyclen, die in den Verbindungen der Formel I vorkommen können, sind Azetidinyl, Pyrrolidinyl einschließlich Pyrrolidin-1-yl, Pyrrolidin-2-yl und Pyrrolidin-3-yl, 2,5-Dihydro-1H-pyrrolyl, Piperidinyl einschließlich Piperidin-1-yl, Piperidin-2-yl, Piperidin-3-yl und Piperidin-4-yl, 1,2,3,4-Tetrahydropyridinyl, 1,2,5,6-Tetrahydropyridinyl, 1,2-Dihydropyridinyl, Azepanyl, Azocanyl, Azecanyl, Octahydrocyclopenta[b]pyrrolyl, 2,3-Dihydrobenzofuranyl einschließlich 2,3-Dihydrobenzofuran-7-yl, 2,3-Dihydro-1H-indolyl, Octahydro-1 H-indolyl, 2,3-Dihydro-1 H-isoindolyl, Octahydro-1 H-isoindolyl, 1,2-Dihydrochinolinyl, 1,2,3,4-Tetrahydrochinolinyl, Decahydrochinolinyl, 1,2-Dihydroisochinolinyl, 1,2,3,4-Tetrahydroisochinolinyl, 1,2,3,4-Tetrahydroisochinolinyl, Decahydroisochinolinyl, Decahydroisochinolinyl, 4,5,6,7-Tetrahydrothieno[3,2-c]pyridinyl, Pyrazolidinyl, Imidazolidinyl, Hexahydropyrimidinyl, 1,2-Dihydropyrimidinyl, Piperazinyl, [1,3]Diazepanyl, [1,4]Diazepanyl, Oxazolidinyl, [1,3]Oxazinanyl, [1,3]Oxazepanyl, Morpholinyl einschließlich Morpholin-2-yl, Morpholin-3-yl und Morpholin-4-yl, [1,4]Oxazepanyl, Thiazolidinyl, [1,3]Thiazinanyl, Thiomorpholinyl einschließlich Thiomorpholin-2-yl, Thiomorpholin-3-yl und Thiomorpholin-4-yl, 3,4-Dihydro-2H-[1,4]thiazinyl, [1,3]Thiazepanyl, [1,4]Thiazepanyl, [1,4]Thiazepanyl, Oxetanyl, Tetrahydrofuranyl, Tetrahydrothienyl, Isoxazolidinyl, Isothiazolidinyl, Oxazolidinyl, [1,2,4]-Oxadiazolidinyl, [1,2,4]-Thiadiazolidinyl, [1,2,4]Triazolidinyl, [1,3,4]Oxadiazolidinyl, [1,3,4]Thiadiazolidinyl, [1,3,4]Triazolidinyl, 2,3-Dihydrofuranyl, 2,5-Dihydrofuranyl, 2,3-Dihydrothienyl, 2,5-Dihydrothienyl, 2,3-Dihydropyrrolyl, 2,3-Dihydroisoxazolyl, 4,5-Dihydroisoxazolyl, 2,5-Dihydroisoxazolyl, 2,3-Dihydroisothiazolyl, 4,5-Dihydroisothiazolyl, 2,5-Dihydroisothiazolyl, 2,3-Dihydropyrazolyl, 4,5-Dihydropyrazolyl, 2,5-Dihydropyrazolyl, 2,3-Dihydrooxazolyl, 4,5-Dihydrooxazolyl, 2,5-Dihydrooxazolyl, 2,3-Dihydrothiazolyl, 4,5-Dihydrothiazolyl, 2,5-Dihydrothiazolyl, 2,3-Dihydroimidazolyl, 4,5-Dihydroimidazolyl, 2,5-Dihydroimidazolyl, Tetrahydropyridazinyl, Tetrahydropyrimidinyl, Tetrahydropyrazinyl, Tetrahydro[1,3,5]triazinyl, [1,3]Dithianyl, Tetrahydropyranyl, Tetrahydrothiopyranyl, [1,3]Dioxolanyl, 3,4,5,6-Tetrahydropyridinyl, 4H-[1,3]Thiazinyl, 1,1-Dioxo-2,3,4,5-tetrahydrothienyl, 2-Azabicyclo[3.1.0]hexyl einschließlich 2-Azabicyclo[3.1.0]hex-2-yl, 3-Azabicyclo[3.1.0]hexyl einschließlich 3-Azabicyclo[3.1.0]hex-3-yl, 2-Oxa-5-azabicyclo[2.2.1]heptyl einschließlich 2-Oxa-5-azabicyclo[2.2.1]hept-5-yl, die alle über ein geeignetes Ringkohlenstoffatom oder Ringstickstoffatom gebunden sind und gegebenenfalls wie in der Definition der jeweiligen Gruppe angegebenen substituiert sind.
Halogen steht für Fluor, Chlor, Brom oder Iod. In einer Ausführungsform der Erfindung ist jedes Halogen in einer Verbindung der Formel I unabhängig von jedem anderen Halogen aus Fluor, Chlor und Brom ausgewählt, in einer anderen Ausführungsform aus Fluor und Chlor.

Wenn eine Oxogruppe an ein Kohlenstoffatom gebunden ist, ersetzt sie zwei Wasserstoffatome an einem Kohlenstoffatom des zugrundeliegenden Systems. Somit wird eine CH₂-Gruppe in einer Kette oder einem Ring dann, wenn sie durch Oxo, d.h. durch ein doppelt gebundenes Sauerstoffatom, substituiert ist, zu einer C(O)-Gruppe (= C(=O)-Gruppe). Offensichtlich kann eine Oxogruppe nicht als Substituent an einem Kohlenstoffatom in einem aromatischen Ring wie beispielsweise in einer Phenylgruppe vorkommen. Wenn ein Ringschwefelatom in einer heterocyclischen Gruppe eine oder zwei Oxogruppen tragen kann, handelt es sich in dem Fall, daß es keine Oxogruppe trägt, um ein nichtoxidiertes Schwefelatom S, oder in dem Fall, daß es eine Oxogruppe trägt, um eine S(O)-Gruppe (= Sulfoxidgruppe, S-Oxid-Gruppe) oder in dem Fall, daß es zwei Oxogruppen trägt, um eine S(O)₂-Gruppe (= Sulfongruppe, S,S-Dioxid-Gruppe).

Die vorliegende Erfindung schließt alle stereoisomeren Formen der Verbindungen der Formel I und ihre Salze und Solvate ein. Bezüglich jedes chiralen Zentrums können die Verbindungen der Formel I unabhängig von jedem anderen chiralen Zentrum in S-Konfiguration oder weitgehend S-Konfiguration oder in R-Konfiguration oder weitgehend R-Konfiguration oder als Mischung des S-Isomers und des R-Isomers in beliebigen Verhältnissen vorliegen. Die Erfindung schließt alle möglichen Enantiomere und Diastereomere und Mischungen von zwei oder mehr Stereoisomeren, zum Beispiel Gemische von Enantiomeren und/oder Diastereomeren, in allen Verhältnissen ein. Somit können erfindungsgemäße Verbindungen, die als Enantiomere existieren können, in enantiomerenreiner Form sowohl als linksdrehende als auch rechtsdrehende Antipoden und in Form von Mischungen der beiden Enantiomere in allen Verhältnissen einschließlich Racematen vorliegen. Im Fall einer E/Z-Isomerie bzw. cis/trans-Isomerie, beispielsweise an Doppelbindungen oder Ringen wie Cycloalkylringen, schließt die Erfindung sowohl die E-Form als auch die Z-Form bzw. die cis-Form und die trans-Form sowie Mischungen dieser Formen in allen Verhältnissen ein. In einer Ausführungsform der Erfindung handelt es sich bei einer Verbindung, die in zwei oder mehr stereoisomeren Formen vorkommen kann, um ein reines oder weitgehend reines einzelnes Stereoisomer. Die Herstellung von einzelnen Stereoisomeren kann beispielsweise durch Trennung einer Mischung von Isomeren nach üblichen Methoden, beispielsweise durch Chromatographie oder Kristallisation, durch Verwendung von stereochemisch einheitlichen Ausgangsstoffen bei der Synthese oder durch stereoselektive Synthese erfolgen. Gegebenenfalls kann vor einer Trennung von Stereoisomeren eine Derivatisierung durchgeführt werden. Die Trennung einer Mischung von Stereoisomeren kann auf der Stufe der Verbindung der Formel I oder auf der Stufe eines Ausgangsstoffs oder eines Zwischenprodukts im Verlauf der Synthese durchgeführt werden. Die vorliegende Erfindung schließt auch alle tautomeren Formen der Verbindungen der Formel I und ihre Salze und Solvate ein.

Wenn die Verbindungen der Formel I eine oder mehrere saure und/oder basische Gruppen, d.h. salzbildende Gruppen, enthalten, schließt die Erfindung auch ihre entsprechenden physiologisch oder toxikologisch akzeptablen Salze, d.h. nichttoxischen Salze, insbesondere ihre pharmazeutisch akzeptablen Salze, ein. Somit können die Verbindungen der Formel I, die eine saure Gruppe, wie eine Hydroxycarbonylgruppe (= Carboxygruppe = C(O)-OH-Gruppe), enthalten, an derartigen Gruppen vorliegen und erfindungsgemäß verwendet werden, beispielsweise als Alkalimetallsalze, Erdalkalimetallsalze oder als Ammoniumsalze. Genauere Beispiele für derartige Salze sind Natriumsalze, Kaliumsalze, Calciumsalze, Magnesiumsalze, quartäre Ammoniumsalze wie Tetraalkylammoniumsalze oder Säureadditionssalze mit Ammoniak oder organischen Aminen wie beispielsweise Ethylamin, Ethanolamin, Triethanolamin oder Aminosäuren. Verbindungen der Formel I, die eine basische Gruppe, d.h. eine protonierbare Gruppe wie eine Aminogruppe oder einen Stickstoffheterocyclus, enthalten, können in Form ihrer Additionssalze mit anorganischen und organischen Säuren an derartigen Gruppen vorliegen und erfindungsgemäß verwendet werden. Beispiele für geeignete Säuren sind Chlorwasserstoff, Bromwasserstoff, Phosphorsäure, Schwefelsäure, Methansulfonsäure, Oxalsäure, Essigsäure, Trifluoressigsäure, Weinsäure, Milchsäure, Benzoesäure, Malonsäure, Fumarsäure, Maleinsäure, Citronensäure und andere dem Fachmann bekannten Säuren. Wenn eine Verbindung der Formel I gleichzeitig eine saure Gruppe und eine basische Gruppe im Molekül enthält, so schließt die Verbindung zusätzlich zu den erwähnten Salzformen auch innere Salze (= Betaine, Zwitterionen) ein. Die Salze der Verbindungen der Formel I sind nach dem Fachmann bekannten üblichen Methoden erhältlich, beispielsweise durch Inberührungbringen der Verbindung der Formel I in einem Lösungsmittel oder Verdünnungsmittel mit einer organischen oder anorganischen Säure oder Base oder durch Anionenaustausch oder Kationenaustausch aus einem anderen Salz. Die Erfindung schließt auch alle Salze der Verbindungen der Formel I ein, die aufgrund geringer physiologischer Verträglichkeit der salzbildenden Säure oder Base nicht direkt für eine Verwendung in Pharmazeutika geeignet sind, aber zum Beispiel als Zwischenprodukte für chemische Reaktionen oder zur Herstellung von physiologisch akzeptablen Salzen verwendet werden können.

Die vorliegende Erfindung schließt alle Solvate von Verbindungen der Formel I, beispielsweise Hydrate oder Addukte mit Alkoholen wie (C₁-C₄)-Alkanolen, aktive Metaboliten der Verbindungen der Formel I und auch Prodrugs und Derivate der Verbindungen der Formel I, die in vitro nicht unbedingt pharmakologische Wirkung zeigen, aber in vivo in pharmakologisch wirksame Verbindungen umgewandelt werden, beispielsweise Ester oder Amide von Carbonsäuregruppen, ein.

In einer Ausführungsform der Erfindung ist A aus NH und O ausgewählt, in einer anderen Ausführungsform ist A unter NH und S ausgewählt, in einer anderen Ausführungsform ist A unter O und S ausgewählt, in einer anderen Ausführungsform steht A für NH, in einer anderen Ausführungsform steht A für O, in einer anderen Ausführungsform steht A für S.

In einer anderen Ausführungsform der Erfindung ist die Zahl t aus 0, 1 oder 2 ausgewählt, in einer anderen Ausführungsform aus 0 oder 1, in einer anderen Ausführungsform aus 1, 2 oder 3, in einer anderen Ausführungsform aus 1 oder 2, in einer anderen Ausführungsform steht t für 0, in einer anderen Ausführungsform steht t für 1. In einer Ausführungsform ist R¹ aus (C₁-C₆)-Alkyl, (C₃-C₇)-Cycloalkyl-CₜH₂ₜ- und Het-CₜH₂ₜ- ausgewählt, in einer anderen Ausführungsform aus (C₁-C₆)-Alkyl und (C₃-C₇)-Cycloalkyl-CₜH₂ₜ-, in einer anderen Ausführungsform steht R¹ für (C₁-C₆)-Alkyl, in einer anderen Ausführungsform steht R¹ für (C₃-C₇)-Cycloalkyl-CₜH₂ₜ-, und in einer anderen Ausführungsform steht R¹ für Het-CₜH₂ₜ-. In einer Ausführungsform steht R¹ für (C₃-C₇)-Cycloalkyl-CₜH₂ₜ-, worin t aus 0, 1 und 2 ausgewählt ist, in einer anderen Ausführungsform steht R¹ für (C₃-C₇)-Cycloalkyl-CₜH₂ₜ-, worin t aus 0 und 1 ausgewählt ist, in einer anderen Ausführungsform steht R¹ für (C₃-C₇)-Cycloalkyl-CH₂-, in einer anderen Ausführungsform steht R¹ für (C₃-C₇)-Cycloalkyl, in einer anderen Ausführungsform steht R¹ für Het-CₜH₂ₜ-, worin t unter 0, 1 und 2 ausgewählt ist, in einer anderen Ausführungsform steht R¹ für Het-CₜH₂ₜ-, worin t unter 0 und 1 ausgewählt ist, in einer anderen Ausführungsform steht R¹ für Het-CH₂-, in einer anderen Ausführungsform steht R¹ für Het. In einer Ausführungsform ist eine (C₁-C₆)-Alkylgruppe, die R¹ repräsentiert, (C₂-C₆)-Alkyl, in einer anderen Ausführungsform (C₂-C₅)-Alkyl, in einer anderen Ausführungsform (C₃-C₅)-Alkyl. In einer Ausführungsform sind eine (C₂-C₆)-Alkenylgruppe und eine (C₂-C₆)-Alkinylgruppe, die R¹ repräsentieren, (C₃-C₆)-Alkenyl bzw. (C₃-C₆)-Alkinyl, in einer anderen Ausführungsform (C₃-C₄)-Alkenyl bzw. (C₃-C₄)-Alkinyl. In einer Ausführungsform ist eine (C₃-C₇)-Cycloalkylgruppe, die in R¹ vorliegt, (C₃-C₆)-Cycloalkyl, in einer anderen Ausführungsform (C₃-C₅)-Cycloalkyl, in einer anderen Ausführungsform (C₃-C₄)-Cycloalkyl, in einer anderen Ausführungsform Cyclopropyl. In einer Ausführungsform ist eine Gruppe Het, die R¹ repräsentiert, ein 4-gliedriger bis 6-gliedriger, in einer anderen Ausführungsform ein 4-gliedriger bis 5-gliedriger, in einer anderen Ausführungsform ein 4-gliedriger, gesättigter monocyclischer Heterocyclus, der über ein Ringkohlenstoffatom gebunden ist, der 1 oder 2 gleiche oder verschiedene Ringheteroatome, in einer anderen Ausführungsform 1 Ringheteroatom, enthält, die aus N, O und S, in einer anderen Ausführungsform aus O und S, ausgewählt sind und in einer anderen Ausführungsform für O-Atome stehen. In einer Ausführungsform ist eine Gruppe Het, die R¹ repräsentiert, eine Oxetanylgruppe, beispielsweise eine Oxetan-3-ylgruppe. In einer Ausführungsform ist die Zahl der Substituenten, die gegebenenfalls an einer R¹ repräsentierenden Gruppe Het vorliegen, eins, zwei oder drei, in einer anderen Ausführungsform eins oder zwei, in einer anderen Ausführungsform eins, und in einer anderen Ausführungsform ist eine derartige Gruppe Het unsubstituiert. In einer Ausführungsform ist ein (C₁-C₄)-Alkylsubstituent, der an einer R¹ repräsentierenden Gruppe Het vorkommt, eine Methylgruppe.

In einer Ausführungsform der Erfindung ist die Zahl der Ringheteroatome in einem aromatischen Heterocyclus, der R² repräsentiert, 1 oder 2, in einer anderen Ausführungsform 1. In einer Ausführungsform der Erfindung ist R² aus Phenyl und einem Rest eines aromatischen, 6-gliedrigen monocyclischen Heterocyclus, der 1, 2 oder 3 Ringstickstoffatome, in einer anderen Ausführungsform 1 oder 2 Ringstickstoffatome, in einer anderen Ausführungsform 1 Ringstickstoffatom enthält, ausgewählt, wobei eines der Ringstickstoffatome einen Substituenten R²¹ tragen kann, der Oxy ist, d.h. wobei eines der Ringstickstoffatome zum N-Oxid oxidiert sein kann, und wobei das Phenyl und der Rest eines aromatischen Heterocyclus gegebenenfalls an einem oder mehreren Ringkohlenstoffatomen durch gleiche oder verschiedene Substituenten R²² substituiert sind. In einer anderen Ausführungsform steht R² für Phenyl, wobei das Phenyl gegebenenfalls an einem oder mehreren Ringatomen durch gleiche oder verschiedene Substituenten R²² substituiert ist, und in einer anderen Ausführungsform steht R² für Pyridinyl, wobei das Ringstickstoffatom einen Substituenten R²¹, der Oxy ist, tragen kann, d.h. wobei das Ringstickstoffatom zum N-Oxid oxidiert sein kann, und wobei das Pyridinyl gegebenenfalls an einem oder mehreren Ringkohlenstoffatomen durch gleiche oder verschiedene Substituenten R²² substituiert ist. In einer anderen Ausführungsform steht R² für einen Rest eines aromatischen 5-gliedrigen Heterocyclus, der 1, 2 oder 3 gleiche oder verschiedene Ringheteroatome, die aus N, O und S ausgewählt sind, enthält, wobei eines der Ringstickstoffatome ein Wasserstoffatom oder einen Substituenten R²¹ tragen kann und wobei der Rest eines aromatischen Heterocyclus gegebenenfalls an einem oder mehreren Ringkohlenstoffatomen durch gleiche oder verschiedene Substituenten R²² substituiert ist. In einer Ausführungsform ist ein Rest einer aromatischen heterocyclischen Gruppe, die R² repräsentiert, aus Furanyl, Thiophenyl, Oxazolyl, Thiazolyl, Pyridinyl, Pyridazinyl, Pyrimidinyl und Pyrazinyl ausgewählt, in einer anderen Ausführungsform aus Furanyl, Thiophenyl, Thiazolyl, Pyridinyl, Pyridazinyl, Pyrimidinyl und Pyrazinyl, in einer anderen Ausführungsform aus Furanyl, Thiophenyl, Pyridinyl, Pyridazinyl, Pyrimidinyl und Pyrazinyl, in einer anderen Ausführungsform aus Furanyl, Thiophenyl, Pyridinyl und Pyrimidinyl, in einer anderen Ausführungsform aus Furanyl, Thiophenyl und Pyridinyl, die alle gegebenenfalls wie in bezug auf R² angegeben substituiert sind. In einer anderen Ausführungsform ist R² aus einer oder mehreren der Gruppen Furan-2-yl, Thiophen-2-yl, Pyridin-3-yl, Pyridin-4-yl und Pyrimidin-5-yl ausgewählt, in einer anderen Ausführungsform aus Phenyl, Furan-2-yl, Thiophen-2-yl, Pyridin-3-yl, Pyridin-4-yl und Pyrimidin-5-yl, in einer anderen Ausführungsform aus Pyridin-3-yl und Pyridin-4-yl, in einer anderen Ausführungsform aus Phenyl, Pyridin-3-yl und Pyridin-4-yl, die alle gegebenenfalls wie in bezug auf R² angegeben substituiert sind. In einer Ausführungsform ist die Zahl der Substituenten R²², die gegebenenfalls an Ringkohlenstoffatomen in R² vorliegen können, 1, 2, 3, 4 oder 5, in einer anderen Ausführungsform 1, 2, 3 oder 4, in einer anderen Ausführungsform 1, 2 oder 3, in einer anderen Ausführungsform 1 oder 2, in einer anderen Ausführungsform 1. Ringkohlenstoffatome in R², die keinen Substituenten R²² tragen, tragen ein Wasserstoffatom.

In einer Ausführungsform der Erfindung ist R³ aus (C₁-C₆)-Alkyl, (C₂-C₆)-Alkenyl und (C₂-C₆)-Alkinyl ausgewählt, in einer anderen Ausführungsform steht R³ für (C₁-C₆)-Alkyl, in einer anderen Ausführungsform steht R³ für (C₂-C₅)-Alkyl, und in einer anderen Ausführungsform steht R³ für (C₁-C₄)-Alkyl, mit der Maßgabe, daß R³ nicht für eine Alkylgruppe stehen kann, wenn A für S steht. In einer anderen Ausführungsform ist R³ aus (C₃-C₇)-Cycloalkyl-CᵤH₂ᵤ- und Het-CᵥH₂ᵥ- ausgewählt, in einer anderen Ausführungsform steht R³ für (C₃-C₇)-Cycloalkyl-CᵤH₂ᵤ-, und in einer anderen Ausführungsform steht R³ für Het-CᵥH₂ᵥ-, wobei in dieser Ausführungsform u und v unabhängig voneinander aus 1 und 2 ausgewählt sind. In einer Ausführungsform steht u für 1, in einer anderen Ausführungsform steht u für 2. In einer Ausführungsform steht v für 1, in einer anderen Ausführungsform steht v für 2. In einer Ausführungsform ist die Gruppe (C₃-C₇)-Cycloalkyl-CᵤH₂ᵤ-, die R³ repräsentiert, aus Cyclopropyl-CᵤH₂ᵤ-, Cyclobutyl-CᵤH₂ᵤ- und Cyclopentyl-CᵤH₂ᵤ- ausgewählt.

In einer Ausführungsform ist R³ aus (C₃-C₇)-Cycloalkyl-CᵤH₂ᵤ- und Het-CᵥH₂ᵥ-ausgewählt, oder R³ steht für einen Rest eines gesättigten oder ungesättigten, 3-gliedrigen bis 10-gliedrigen, monocyclischen oder bicyclischen Rings, der 0, 1, 2, 3 oder 4 gleiche oder verschiedene Ringheteroatome, die unter N, O und S ausgewählt sind, enthält, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist, und in einer anderen Ausführungsform steht R³ für einen Rest eines gesättigten oder ungesättigten, 3-gliedrigen bis 10-gliedrigen, monocyclischen oder bicyclischen Rings, der 0, 1, 2, 3 oder 4 gleiche oder verschiedene Ringheteroatome, die aus N, O und S ausgewählt sind, enthält, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist. In einer Ausführungsform ist die Zahl der Ringheteroatome im Ring, der R³ repräsentiert, 0, 1, 2 oder 3, in einer anderen Ausführungsform 0, 1 oder 2, in einer anderen Ausführungsform 0 oder 1, in einer anderen Ausführungsform 0, in einer anderen Ausführungsform ist sie 1, 2, 3 oder 4, in einer anderen Ausführungsform 1, 2 oder 3, in einer anderen Ausführungsform 1 oder 2, in einer anderen Ausführungsform 1. Der Rest des Rings, der R³ repräsentiert, kann somit carbocyclisch oder heterocyclisch sein. In einer Ausführungsform sind die Ringheteroatome in R³ aus N und O ausgewählt, in einer anderen Ausführungsform aus N und S, in einer anderen Ausführungsform aus O und S, in einer anderen Ausführungsform stehen sie für N, wobei Ringstickstoffatome ein Wasserstoffatom oder einen (C₁-C₄)-Alkylsubstituenten tragen können, wie es beispielsweise in gesättigten oder teilweise ungesättigten Heterocyclen oder in 5-gliedrigen aromatischen Ringen in Heterocyclen wie Pyrrol oder Benzoimidazol vorkommt, oder kein Wasserstoffatom und keinen (C₁-C₄)-Alkylsubstituenten tragen, wie es in aromatischen Heterocyclen wie beispielsweise Imidazol oder Pyridin vorkommt. In einem Rest eines R³ repräsentierenden Heterocyclus, der ein oder mehrere Ringschwefelatome enthält, ist in einer Ausführungsform eines der Ringschwefelatome nicht oxidiert oder trägt eine oder zwei Oxogruppen, und alle anderen Ringschwefelatome sind nicht oxidiert. Der Rest eines monocyclischen oder bicyclischen Rings, der R³ repräsentiert, kann über ein beliebiges geeignetes Ringkohlenstoffatom oder Ringstickstoffatom an die Gruppe A gebunden sein. In einer Ausführungsform ist er über ein Ringkohlenstoffatom gebunden, in einer anderen Ausführungsform ist er über ein Ringkohlenstoffatom oder dann, wenn A für NH steht, über ein Ringstickstoffatom gebunden, und in einer anderen Ausführungsform ist er über ein Ringstickstoffatom gebunden. Der Rest eines monocyclischen oder bicyclischen Rings, der R³ repräsentiert, kann ungesättigt sein und in diesem Fall 1, 2, 3, 4 oder 5 oder 1, 2, 3 oder 4 oder 1, 2 oder 3 oder 1 oder 2 oder 1 Doppelbindungen im Ring enthalten und kann in jedem der beiden Ringe aromatisch oder nichtaromatisch sein, oder er kann gesättigt sein und in diesem Fall keine Doppelbindungen im Ring enthalten. In einer Ausführungsform ist der Rest des Rings, der R³ repräsentiert, gesättigt oder aromatisch, in einer anderen Ausführungsform ist er gesättigt, und in einer anderen Ausführungsform ist er aromatisch. In einer Ausführungsform ist der Rest des 3-gliedrigen oder 4-gliedrigen Rings, der R³ repräsentiert, gesättigt. Wenn R³ Ringstickstoffatome enthält, die ein Wasserstoffatom oder einen (C₁-C₄)-Alkylsubstituenten tragen können, kann ein derartiges Ringstickstoffatom bzw. können zwei derartige Ringstickstoffatome vorliegen. In einer Ausführungsform ist die Zahl der fakultativen Substituenten R³¹ an Ringkohlenstoffatomen in dem R³ repräsentierenden Ring 1, 2, 3, 4, 5 oder 6, in einer anderen Ausführungsform 1, 2, 3, 4 oder 5, in einer anderen Ausführungsform 1, 2, 3 oder 4, in einer anderen Ausführungsform 1, 2 oder 3, in einer anderen Ausführungsform 1 oder 2, in einer anderen Ausführungsform 1.

Der Ring, der R³ repräsentieren kann, kann 3-gliedrig, 4-gliedrig, 5-gliedrig, 6-gliedrig, 7-gliedrig, 8-gliedrig, 9-gliedrig oder 10-gliedrig sein. In einer Ausführungsform ist R³ 4-gliedrig bis 10-gliedrig, in einer anderen Ausführungsform 4-gliedrig bis 9-gliedrig, in einer anderen Ausführungsform 4-gliedrig bis 8-gliedrig, in einer anderen Ausführungsform 4-gliedrig bis 7-gliedrig, in einer anderen Ausführungsform 5-gliedrig bis 7-gliedrig, in einer anderen Ausführungsform 5-gliedrig oder 6-gliedrig, in einer anderen Ausführungsform 6-gliedrig, in einer anderen Ausführungsform 8-gliedrig bis 10-gliedrig, in einer anderen Ausführungsform 9-gliedrig bis 10-gliedrig. In einer Ausführungsform enthält ein 3-gliedriger Ring, der R³ repräsentiert, keine Ringheteroatome. In einer Ausführungsform ist R³ monocyclisch, in einer anderen Ausführungsform bicyclisch. In einer Ausführungsform ist eine bicyclische Gruppe, die R³ repräsentiert, mindestens 7-gliedrig. Unter anderem kann der Rest eines Rings, der R³ repräsentiert, eine Cycloalkylgruppe, eine Phenylgruppe, eine Naphthylgruppe, ein Rest einer ungesättigten, aromatischen oder nichtaromatischen heterocyclischen Gruppe oder ein Rest einer gesättigten heterocyclischen Gruppe sein, die alle gegebenenfalls an Ringkohlenstoffatomen und Ringstickstoffatomen wie in bezug auf R³ angegeben substituiert sind. Soweit anwendbar, gelten alle oben angegebenen Erklärungen in bezug auf derartige Gruppen entsprechend für R³. Ein anderes Beispiel für Gruppen, die R³ repräsentieren können, sind Cycloalkenylgruppen wie (C₅-C₇)-Cycloalkenylgruppen, die über ein beliebiges Ringkohlenstoffatom gebunden sein können und gegebenenfalls wie in bezug auf R³ angegeben substituiert sind. In einer Ausführungsform sind fakultative Substituenten R³¹ an einer R³ repräsentierenden Cycloalkenylgruppe aus Fluor und (C₁-C₄)-Alkyl ausgewählt. In einer Ausführungsform enthalten Cycloalkenylgruppen eine Doppelbindung im Ring, die in einer beliebigen Position vorliegen kann. Beispiele für Cycloalkenyl sind Cyclopentenyl einschließlich Cyclopent-1-enyl, Cyclopent-2-enyl und Cyclopent-3-enyl, Cyclohexenyl einschließlich Cyclohex-1-enyl, Cyclohex-2-enyl und Cyclohex-3-enyl und Cycloheptenyl einschließlich Cyclohept-1-enyl, Cyclohept-2-enyl, Cyclopent-3-enyl und Cyclohept-4-enyl. Beispiele für Reste von Ringen, aus denen R³ in einer Ausführungsform der Erfindung ausgewählt ist, sind Cyclobutyl, Cyclopentyl, Cyclohexyl, Phenyl, Oxetanyl einschließlich Oxetan-3-yl, Tetrahydrofuranyl einschließlich Tetrahydrofuran-3-yl, Tetrahydrothiophenyl einschließlich Tetrahydrothiophen-3-yl, Tetrahydropyranyl einschließlich Tetrahydropyran-4-yl, Azetidinyl einschließlich Azetidin-1-yl, Pyrrolidinyl, Piperidinyl, Imidazolidinyl, Piperazinyl, Morpholinyl einschließlich Morpholin-1-yl, Thiomorpholinyl, Furanyl einschließlich Furan-3-yl, Thiophenyl einschließlich Thiophen-3-yl, Pyrazolyl einschließlich Pyrazol-3-yl, Imidazolyl, Thiazolyl einschließlich Thiazol-2-yl, Pyridinyl einschließlich Pyridin-2-yl, Pyridin-3-yl und Pyridin-4-yl, Pyridazinyl einschließlich Pyridazin-3-yl, wobei in allen davon, sofern anwendbar, ein oder zwei der Ringstickstoffatome ein Wasserstoffatom oder (C₁-C₄)-Alkyl tragen können und wobei alle davon gegebenenfalls an einem oder mehreren Ringkohlenstoffatomen durch gleiche oder verschiedene Substituenten R³¹ substituiert sind und wobei in allen davon, sofern anwendbar, ein Ringschwefelatom nichtoxidiert sein kann, d.h. als Schwefelatom vorliegen kann, oder eine oder zwei Oxogruppen tragen kann, d.h. in Form eines Sulfoxids oder Sulfons vorliegen kann.

In einer Ausführungsform ist R³ aus Phenyl und einem Rest eines gesättigten oder ungesättigten 3-gliedrigen bis 7-gliedrigen, monocyclischen Rings ausgewählt, in einer anderen Ausführungsform aus Phenyl und einem Rest eines gesättigten oder ungesättigten 5-gliedrigen bis 7-gliedrigen, monocyclischen Rings, in einer anderen Ausführungsform aus Phenyl, Pyridinyl und einem Rest eines gesättigten 3-gliedrigen bis 7-gliedrigen, monocyclischen Rings, in einer anderen Ausführungsform aus Phenyl, Pyridinyl und einem Rest eines gesättigten 5-gliedrigen bis 7-gliedrigen, monocyclischen Rings, in einer anderen Ausführungsform aus Phenyl und einem Rest eines gesättigten 3-gliedrigen bis 7-gliedrigen, monocyclischen Rings, in einer anderen Ausführungsform aus Phenyl und einem Rest eines gesättigten 5-gliedrigen bis 7-gliedrigen, monocyclischen Rings, wobei in allen diesen Ausführungsformen der monocyclischen Ring 1 oder 2 gleiche oder verschiedene Ringheteroatome, die aus N, O und S ausgewählt sind, enthält, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei das Phenyl, das Pyridinyl und der Rest eines Rings gegebenenfalls an einem oder mehreren Ringkohlenstoffatomen durch gleiche oder verschiedene Substituenten R³¹ substituiert sind und wobei Pyridinyl Pyridin-2-yl, Pyridin-3-yl und Pyridin-4-yl einschließt. In einer anderen Ausführungsform ist R³ aus Phenyl und Pyridinyl ausgewählt, in einer anderen Ausführungsform steht R³ für Pyridinyl, und in einer anderen Ausführungsform steht R³ für Phenyl, wobei in diesen Ausführungsformen Pyridinyl die Gruppen Pyridin-2-yl, Pyridin-3-yl und Pyridin-4-yl einschließt und in einer Ausführungsform aus einer oder mehreren dieser Gruppen ausgewählt ist und wobei in allen diesen Ausführungsformen das Phenyl und das Pyridinyl gegebenenfalls an einem oder mehreren Ringkohlenstoffatomen durch gleiche oder verschiedene Substituenten R³¹ substituiert sind.

In einer Ausführungsform der Erfindung ist die Zahl w aus 0 und 1 ausgewählt, in einer anderen Ausführungsform steht sie für 0, in einer anderen Ausführungsform steht sie für 1. In einer Ausführungsform ist eine (C₃-C₇)-Cycloalkylgruppe, die in R²¹ vorliegt, (C₃-C₆)-Cycloalkyl, in einer anderen Ausführungsform (C₃-C₅)-Cycloalkyl, in einer anderen Ausführungsform Cyclopropyl. In einer Ausführungsform ist R²¹ aus (C₁-C₄)-Alkyl und Oxy ausgewählt, in einer anderen Ausführungsform steht R²¹ für (C₁-C₄)-Alkyl, in einer anderen Ausführungsform für (C₁-C₃)-Alkyl, in einer anderen Ausführungsform für Methyl und in einer anderen Ausführungsform für Oxy.

In einer Ausführungsform der Erfindung sind die Substituenten R²², die gegebenenfalls an der Gruppe R² vorliegen, aus Halogen, Hydroxy, (C₁-C₄)-Alkyl-, (C₁-C₄)-Alkyloxy-, (C₁-C₄)-Alkyl-S(O)ₘ-, Amino, Nitro, Cyano, R²³ und R²³-O- ausgewählt, in einer anderen Ausführungsform aus Halogen, Hydroxy, (C₁-C₄)-Alkyl-, (C₁-C₄)-Alkyloxy-, Amino, Cyano, R²³ und R²³-O-, in einer anderen Ausführungsform aus Halogen, Hydroxy, (C₁-C₄)-Alkyl-, (C₁-C₄)-Alkyloxy-, R²³ und R²³-O-, in einer anderen Ausführungsform aus Halogen, Hydroxy, (C₁-C₄)-Alkyl- und (C₁-C₄)-Alkyloxy-, wobei in allen diesen Ausführungsformen R²³ wie definiert ist.

In einer Ausführungsform sind 1, 2 oder 3 der Substituenten R²², in einer anderen Ausführungsform 1 oder 2 der Substituenten R²² und in einer anderen Ausführungsform 1 der Substituenten R²², die gegebenenfalls an der Gruppe R² vorliegen, wie in der allgemeinen Definition von R²² definiert und somit aus Halogen, Hydroxy, (C₁-C₄)-Alkyl-, (C₁-C₄)-Alkyloxy-, (C₁-C₄)-Alkyl-S(O)ₘ-, Amino, Nitro, Cyano, Hydroxycarbonyl, (C₁-C₄)-Alkyloxycarbonyl, Aminocarbonyl, Aminosulfonyl, R²³ und R²³-O- ausgewählt, wobei R²³ wie definiert ist, und jegliche weiteren Substituenten R²², die gegebenenfalls an der Gruppe R² vorliegen, beispielsweise 1, 2 oder 3 weitere Substituenten R²², oder 1 oder 2 weitere Substituenten R²² oder 1 weiterer Substituent R²², sind aus Halogen, Hydroxy, (C₁-C₄)-Alkyl-, (C₁-C₄)-Alkyloxy-, (C₁-C₄)-Alkyl-S(O)ₘ-, Amino, Nitro, Cyano, R²³ und R²³-O- ausgewählt, wobei alle Alkylgrupppen unabhängig voneinander gegebenenfalls durch einen oder mehrere Fluorsubstituenten substituiert sind, wie es allgemein für Alkylgruppen gilt. In einer Ausführungsform sind die Substituenten R²², die gegebenenfalls an der Gruppe R² vorliegen und in der oben aufgeführten Ausführungsform wie in der allgemeinen Definition von R²² definiert sind, beispielsweise 1 oder 2 derartige Substituenten R²² oder 1 derartiger Substituent R²², aus Halogen, Hydroxy, (C₁-C₄)-Alkyl-, (C₁-C₄)-Alkyloxy-, (C₁-C₄)-Alkyl-S(O)ₘ-, Amino, Nitro, Cyano, R²³ und R²³-O- ausgewählt, in einer anderen Ausführungsform aus Halogen, Hydroxy, (C₁-C₄)-Alkyl-, (C₁-C₄)-Alkyloxy-, (C₁-C₄)-Alkyl-S(O)ₘ-, Amino, R²³ und R²³-O-, in einer anderen Ausführungsform aus Halogen, Hydroxy, (C₁-C₄)-Alkyl-, (C₁-C₄)-Alkyloxy-, Amino, R²³ und R²³-O-, in einer anderen Ausführungsform aus Halogen, Hydroxy, (C₁-C₄)-Alkyl-, (C₁-C₄)-Alkyloxy-, (C₁-C₄)-Alkyl-S(O)ₘ-, Amino und Cyano, in einer anderen Ausführungsform aus Halogen, Hydroxy, (C₁-C₄)-Alkyl-, (C₁-C₄)-Alkyloxy- und Cyano, wobei R²³ wie definiert ist. In einer Ausführungsform befinden sich die Substituenten R²², die gegebenenfalls an der Gruppe R² vorliegen und in der oben aufgeführten Ausführungsform wie in der allgemeinen Definition von R²² definiert sind, beispielsweise 1 oder 2 derartige Substituenten R²² oder 1 derartiger Substituent R²², nicht an Ringkohlenstoffatomen in der Gruppe R², die dem Atom, über das die Gruppe R² an den in Formel I dargestellten Oxazolopyrimidinring gebunden ist, benachbart ist. In einer anderen Ausführungsform liegen im Fall einer R² repräsentierenden Phenylgruppe 1 oder 2 derartige Substituenten R²² oder 1 derartiger Substituent R²² gegebenenfalls in einer der Positionen 3, 4 und 5 der Phenylgruppe vor, und in einer anderen Ausführungsform liegt 1 derartiger Substituent R²² in Position 4 der Phenylgruppe vor. In einer Ausführungsform sind die weiteren Substituenten R²², die gegebenenfalls an der Gruppe R² vorliegen, beispielsweise 1, 2 oder 3 weitere Substituenten R²² oder 1 oder 2 weitere Substituenten R²² oder 1 weiterer Substituent R²², aus Halogen, Hydroxy, (C₁-C₄)-Alkyl-, (C₁-C₄)-Alkyloxy-, (C₁-C₄)-Alkyl-S(O)ₘ-, Amino und Cyano ausgewählt, in einer anderen Ausführungsform aus Halogen, Hydroxy, (C₁-C₄)-Alkyl-, (C₁-C₄)-Alkyloxy-, Amino und Cyano, in einer anderen Ausführungsform aus Halogen, Hydroxy, (C₁-C₄)-Alkyl-, (C₁-C₄)-Alkyloxy- und Cyano, in einer anderen Ausführungsform aus Halogen, (C₁-C₄)-Alkyl- und (C₁-C₄)-Alkyloxy-, in einer anderen Ausführungsform aus Halogen und (C₁-C₄)-Alkyl-, wobei in allen diesen Ausführungsformen alle Alkylgruppen unabhängig voneinander gegebenenfalls durch einen oder mehrere Fluorsubstituenten substituiert sind.

In einer Ausführungsform der Erfindung steht R²³ für einen Rest eines monocyclischen Rings, in einer anderen Ausführungsform einen Rest eines bicyclischen Rings. Der Rest eines Rings, der R²³ repräsentiert, kann carbocyclisch oder heterocyclisch sein. In einer Ausführungsform ist der Rest eines monocyclischen Rings, der R²³ repräsentiert, carbocyclisch, in einer anderen Ausführungsform heterocyclisch. In einer Ausführungsform ist der Rest eines bicyclischen Rings, der R²³ repräsentiert, carbocyclisch, in einer anderen Ausführungsform heterocyclisch. In einer Ausführungsform der Erfindung ist die Zahl der Ringheteroatome in R²³ 0, 1, 2 oder 3, in einer anderen Ausführungsform 0, 1 oder 2, in einer anderen Ausführungsform 0 oder 1, in einer anderen Ausführungsform 1, 2, 3 oder 4, in einer anderen Ausführungsform 1, 2 oder 3, in einer anderen Ausführungsform 1 oder 2, in einer anderen Ausführungsform 1 und in einer anderen Ausführungsform 0, und in dieser letztgenannten Ausführungsform ist R²³ somit eine (C₃-C₇)-Cycloalkylgruppe. In einer Ausführungsform ist der Rest eines monocyclischen Rings, der R²³ repräsentiert, eine Oxetanylgruppe, beispielsweise Oxetan-3-yl.

In einer Ausführungsform sind die Ringheteroatome in R²³ aus N und O ausgewählt, in einer anderen Ausführungsform aus O und S, in einer anderen Ausführungsform stehen sie für N, und in einer anderen Ausführungsform stehen sie für O, wobei Ringstickstoffatome ein Wasserstoffatom oder einen (C₁-C₄)-Alkylsubstituenten tragen können. R²³ kann über ein beliebiges geeignetes Ringkohlenstoffatom und Ringstickstoffatom gebunden sein. Wenn R²³ an ein Sauerstoffatom gebunden ist, ist R²³ in einer Ausführungsform über ein Ringkohlenstoffatom gebunden. In einer anderen Ausführungsform ist R²³ unabhängig von dem Atom, an das R²³ gebunden ist, über ein Ringkohlenstoffatom gebunden. In einer anderen Ausführungsform ist R²³ über ein Ringstickstoffatom gebunden. In einer anderen Ausführungsform ist die Zahl fakultativer Substituenten R²⁴ an Ringkohlenstoffatome in R²³ 1, 2, 3 oder 4 oder 5, in einer anderen Ausführungsform 1, 2, 3 oder 4, in einer anderen Ausführungsform 1, 2 oder 3, in einer anderen Ausführungsform 1 oder 2, in einer anderen Ausführungsform 1. R²³ kann 3-gliedrig, 4-gliedrig, 5-gliedrig, 6-gliedrig oder 7-gliedrig sein. In einer Ausführungsform ist R²³ 4-gliedrig bis 7-gliedrig, in einer anderen Ausführungsform 4-gliedrig bis 6-gliedrig, in einer anderen Ausführungsform 5-gliedrig bis 6-gliedrig, in einer anderen Ausführungsform 4-gliedrig bis 5-gliedrig. In einer Ausführungsform enthält ein 3-gliedriger Ring, der R²³ repräsentiert, keine Ringheteroatome. Beispiele für Reste von Ringen, aus denen R²³ in einer Ausführungsform der Erfindung ausgewählt ist, sind Oxetan-3-yl, Azetidin-1-yl, Pyrrolidin-1-yl, Piperidin-1-yl, Piperidin-4-yl, Morpholin-4-yl und Piperazin-1-yl, die alle gegebenenfalls wie angegeben substituiert sind. In einer Ausführungsform ist R²³ unter einem oder mehreren der Reste Oxetan-3-yl, Azetidin-1-yl, Pyrrolidin-1-yl, Piperidin-1-yl, Morpholin-4-yl und Piperazin-1-yl ausgewählt, in einer anderen Ausführungsform aus einem oder mehreren der Reste Oxetan-3-yl, Azetidin-1-yl, Piperidin-1-yl, Morpholin-4-yl und Piperazin-1-yl, in einer anderen Ausführungsform aus einem oder mehreren der Reste Oxetan-3-yl, Azetidin-1-yl, Pyrrolidin-1-yl und Piperidin-1-yl, und in einer anderen Ausführungsform steht R²³ für Oxetan-3-yl, die alle gegebenenfalls wie angegeben substituiert sind.

In einer Ausführungsform der Erfindung ist R²⁴ aus Halogen, (C₁-C₄)-Alkyl und Hydroxy ausgewählt, in einer anderen Ausführungsform aus Fluor, (C₁-C₄)-Alkyl und Hydroxy, in einer anderen Ausführungsform aus Fluor, Methyl und Hydroxy, in einer anderen Ausführungsform aus Fluor und Methyl, in einer anderen Ausführungsform aus Methyl und Hydroxy, in einer anderen Ausführungsform aus Fluor, (C₁-C₄)-Alkyl, Hydroxy und Oxo.

In einer Ausführungsform der Erfindung ist R³¹ aus Halogen, (C₁-C₄)-Alkyl, (C₃-C₇)-Cycloalkyl, Hydroxy, (C₁-C₄)-Alkyloxy, Oxo, (C₁-C₄)-Alkyl-S(O)ₘ-, Amino, (C₁-C₄)-Alkylamino, Di((C₁-C₄)-alkyl)amino, (C₁-C₄)-Alkylcarbonylamino, (C₁-C₄)-Alkyl-sulfonylamino, Cyano, (C₁-C₄)-Alkylcarbonyl, Aminosulfonyl, (C₁-C₄)-Alkyl-aminosulfonyl und Di((C₁-C₄)-alkyl)aminosulfonyl ausgewählt, in einer anderen Ausführungsform aus Halogen, (C₁-C₄)-Alkyl, (C₃-C₇)-Cycloalkyl, Hydroxy, (C₁-C₄)-Alkyloxy, Oxo, (C₁-C₄)-Alkyl-S(O)ₘ-, Amino, (C₁-C₄)-Alkylamino, Di((C₁-C₄)-alkyl)amino, Cyano, Aminosulfonyl, (C₁-C₄)-Alkylaminosulfonyl und Di((C₁-C₄)-alkyl)aminosulfonyl, in einer anderen Ausführungsform aus Halogen, (C₁-C₄)-Alkyl, (C₃-C₇)-Cycloalkyl, Hydroxy, (C₁-C₄)-Alkyloxy, Oxo, (C₁-C₄)-Alkyl-S(O)ₘ-, Amino, (C₁-C₄)-Alkylamino, Di((C₁-C₄)-alkyl)amino, Cyano und Aminosulfonyl, in einer anderen Ausführungsform aus Halogen, (C₁-C₄)-Alkyl, (C₃-C₇)-Cycloalkyl, Hydroxy, (C₁-C₄)-Alkyloxy, Oxo, Amino, (C₁-C₄)-Alkylamino, Di((C₁-C₄)-alkyl)amino, Cyano und Aminosulfonyl, in einer anderen Ausführungsform aus Halogen, (C₁-C₄)-Alkyl, (C₃-C₇)-Cycloalkyl, Hydroxy, (C₁-C₄)-Alkyloxy, Oxo, Amino, (C₁-C₄)-Alkylamino und Di((C₁-C₄)-alkyl)amino, in einer anderen Ausführungsform aus Halogen, (C₁-C₄)-Alkyl, (C₃-C₇)-Cycloalkyl, (C₁-C₄)-Alkyloxy und Di((C₁-C₄)-alkyl)amino, in einer anderen Ausführungsform aus Halogen, (C₁-C₄)-Alkyl, (C₃-C₇)-Cycloalkyl, Hydroxy und (C₁-C₄)-Alkyloxy, in einer anderen Ausführungsform aus Halogen, (C₁-C₄)-Alkyl und (C₁-C₄)-Alkyloxy, in einer anderen Ausführungsform aus Fluor, Chlor, (C₁-C₄)-Alkyl, (C₃-C₇)-Cycloalkyl, Hydroxy und (C₁-C₄)-Alkyloxy, wobei in allen diesen Ausführungsformen alle Alkylgruppen unabhängig voneinander gegebenenfalls durch einen oder mehrere Fluorsubstituenten substituiert sind.

In einer Ausführungsform sind die fakultativen Substituenten R³¹ an dem Rest eines aromatischen Rings, der R³ repräsentiert, beispielsweise an einer Phenylgruppe oder Pyridinylgruppe, die R³ repräsentiert, unter Halogen, (C₁-C₄)-Alkyl, (C₃-C₇)-Cycloalkyl, Hydroxy, (C₁-C₄)-Alkyloxy, (C₁-C₄)-Alkyl-S(O)ₘ-, Amino, (C₁-C₄)-Alkylamino, Di((C₁-C₄)-alkyl)amino, (C₁-C₄)-Alkylcarbonylamino, (C₁-C₄)-Alkylsulfonylamino, Cyano, (C₁-C₄)-Alkylcarbonyl, Aminosulfonyl, (C₁-C₄)-Alkylaminosulfonyl und Di((C₁-C₄)-alkyl)aminosulfonyl ausgewählt, in einer anderen Ausführungsform aus Halogen, (C₁-C₄)-Alkyl, (C₃-C₇)-Cycloalkyl, Hydroxy, (C₁-C₄)-Alkyloxy, (C₁-C₄)-Alkyl-S(O)ₘ-, Amino, (C₁-C₄)-Alkylamino, Di((C₁-C₄)-alkyl)amino, Cyano, Aminosulfonyl, (C₁-C₄)-Alkylaminosulfonyl und Di((C₁-C₄)-alkyl)aminosulfonyl, in einer anderen Ausführungsform aus Halogen, (C₁-C₄)-Alkyl, (C₃-C₇)-Cycloalkyl, Hydroxy, (C₁-C₄)-Alkyloxy, (C₁-C₄)-Alkyl-S(O)ₘ-, Amino, (C₁-C₄)-Alkylamino, Di((C₁-C₄)-alkyl)amino, Cyano und Aminosulfonyl, in einer anderen Ausführungsform aus Halogen, (C₁-C₄)-Alkyl, (C₃-C₇)-Cycloalkyl, Hydroxy, (C₁-C₄)-Alkyloxy, Amino, (C₁-C₄)-Alkylamino, Di((C₁-C₄)-alkyl)amino, Cyano und Aminosulfonyl, in einer anderen Ausführungsform aus Halogen, (C₁-C₄)-Alkyl, (C₃-C₇)-Cycloalkyl, Hydroxy, (C₁-C₄)-Alkyloxy, Amino, (C₁-C₄)-Alkylamino und Di((C₁-C₄)-alkyl)amino, in einer anderen Ausführungsform aus Halogen, (C₁-C₄)-Alkyl, (C₃-C₇)-Cycloalkyl, (C₁-C₄)-Alkyloxy und Di((C₁-C₄)-alkyl)amino, in einer anderen Ausführungsform aus Halogen, (C₁-C₄)-Alkyl, (C₃-C₇)-Cycloalkyl, Hydroxy und (C₁-C₄)-Alkyloxy, in einer anderen Ausführungsform aus Halogen, (C₁-C₄)-Alkyl und (C₁-C₄)-Alkyloxy, in einer anderen Ausführungsform aus Fluor, Chlor, (C₁-C₄)-Alkyl, (C₃-C₇)-Cycloalkyl, Hydroxy und (C₁-C₄)-Alkyloxy, wobei in allen diesen Ausführungsformen alle Alkylgruppen unabhängig voneinander gegebenenfalls durch einen oder mehrere Fluorsubstituenten substituiert sind.

In einer Ausführungsform sind die fakultativen Substituenten R³¹ an dem Rest eines gesättigten oder nichtaromatischen ungesättigten Rings, der R³ repräsentiert, aus Halogen, (C₁-C₄)-Alkyl, (C₃-C₇)-Cycloalkyl, Hydroxy, (C₁-C₄)-Alkyloxy, Oxo, (C₁-C₄)-Alkyl-S(O)ₘ-, Amino, (C₁-C₄)-Alkylamino, Di((C₁-C₄)-alkyl)amino, (C₁-C₄)-Alkyl-carbonylamino, (C₁-C₄)-Alkylsulfonylamino und Cyano ausgewählt, in einer anderen Ausführungsform aus Halogen, (C₁-C₄)-Alkyl, (C₃-C₇)-Cycloalkyl, Hydroxy, (C₁-C₄)-Alkyloxy, Oxo, Amino, (C₁-C₄)-Alkylamino, Di((C₁-C₄)-alkyl)amino und Cyano, in einer anderen Ausführungsform aus Halogen, (C₁-C₄)-Alkyl, (C₃-C₇)-Cycloalkyl, Hydroxy, (C₁-C₄)-Alkyloxy und Oxo, in einer anderen Ausführungsform aus Halogen, (C₁-C₄)-Alkyl, Hydroxy, (C₁-C₄)-Alkyloxy und Oxo, in einer anderen Ausführungsform aus Fluor, Chlor, (C₁-C₄)-Alkyl, Hydroxy, (C₁-C₄)-Alkyloxy und Oxo, in einer anderen Ausführungsform aus (C₁-C₄)-Alkyl, Hydroxy und Oxo, in einer anderen Ausführungsform aus Alkyl und Hydroxy, und in einer anderen Ausführungsform stehen sie für (C₁-C₄)-Alkyl, wobei in allen diesen Ausführungsformen alle Alkylgruppen unabhängig voneinander gegebenenfalls durch einen oder mehrere Fluorsubstituenten substituiert sind. Wenn der Rest eines Rings, der R³ repräsentiert, Oxogruppen als Substituenten R³¹ enthält, liegen in einer Ausführungsform nicht mehr als zwei derartige Oxosubstituenten vor, und in einer anderen Ausführungsform liegt nicht mehr als ein derartiger Oxosubstituent vor.

In einer Ausführungsform der Erfindung sind die Ringheteroatome in Het aus N und O ausgewählt, in einer anderen Ausführungsform aus O und S, in einer anderen Ausführungsform stehen sie für O-Atome. In einer anderen Ausführungsform ist die Zahl der Ringheteroatome in Het 1. In einer Ausführungsform liegen zwei Ringsauerstoffatome in Het nicht in benachbarten Ringpositionen vor, in einer anderen Ausführungsform liegen zwei aus O und S ausgewählte Ringheteroatome nicht in benachbarten Ringpositionen vor, in einer anderen Ausführungsform liegen zwei Ringheteroatome nicht in benachbarten Ringpositionen vor. Ringstickstoffatome in Het tragen ein Wasserstoffatom oder einen Substituenten wie angegeben. In einer Ausführungsform sind fakultative Substituenten an Ringstickstoffatomen in Het (C₁-C₄)-Alkylsubstituenten. In einer Ausführungsform sind fakultative Substituenten an Ringstickstoffatomen und Ringkohlenstoffatomen in Het (C₁-C₄)-Alkylsubstituenten. In einer Ausführungsform ist die Zahl fakultativer Substituenten an Het 1, 2, 3, 4 oder 5, in einer anderen Ausführungsform 1, 2, 3 oder 4, in einer anderen Ausführungsform 1, 2 oder 3, in einer anderen Ausführungsform 1 oder 2, in einer anderen Ausführungsform 1. Het kann über ein beliebiges geeignetes Ringkohlenstoffatom gebunden sein. In einer Ausführungsform ist Het über ein Ringkohlenstoffatom gebunden, das nicht einem Ringheteroatom benachbart ist. Het kann 4-gliedrig, 5-gliedrig, 6-gliedrig oder 7-gliedrig sein. In einer Ausführungsform ist Het 4-gliedrig oder 5-gliedrig, in einer anderen Ausführungsform 5-gliedrig bis 7-gliedrig, in einer anderen Ausführungsform 5-gliedrig oder 6-gliedrig, in einer anderen Ausführungsform 4-gliedrig. Beispiele für Het, aus denen Het in einer Ausführungsform ausgewählt ist, sind Oxetanyl einschließlich Oxetan-2-yl und Oxetan-3-yl, Tetrahydrofuranyl einschließlich Tetrahydrofuran-2-yl und Tetrahydrofuran-3-yl, Tetrahydropyranyl einschließlich Tetrahydropyran-2-yl, Tetrahydropyran-3-yl und Tetrahydropyran-4-yl, Oxepanyl einschließlich Oxepan-2-yl, Oxepan-3-yl und Oxepan-4-yl, [1,3]Dioxolanyl einschließlich [1,3]Dioxolan-2-yl und [1,3]Dioxolan-4-yl, [1,4]Dioxanyl einschließlich [1,4]Dioxan-2-yl, Thietanyl einschließlich Thietan-2-yl und Thietan-3-yl, Tetrahydrothiophenyl einschließlich Tetrahydrothiophen-2-yl und Tetrahydrothiophen-3-yl, Tetrahydrothiopyranyl einschließlich Tetrahydrothiopyran-2-yl, Tetrahydrothiopyran-3-yl und Tetrahydrothiopyran-4-yl, [1,4]Dithianyl einschließlich [1,4]Dithian-2-yl, Azetidinyl einschließlich Azetidin-2-yl und Azetidin-3-yl, Pyrrolidinyl einschließlich Pyrrolidinyl-2-yl und Pyrrolidinyl-3-yl, Piperidinyl einschließlich Piperidinyl-2-yl, Piperidinyl-3-yl und Piperidinyl-4-yl, Azepanyl einschließlich Azepan-2-yl, Azepan-3-yl und Azepan-4-yl, Oxazolidinyl einschließlich Oxazolidin-2-yl, Oxazolidin-4-yl und Oxazolidin-5-yl, Thiazolidinyl einschließlich Thiazolidin-2-yl, Thiazolidin-4-yl und Thiazolidin-5-yl, Morpholinyl einschließlich Morpholin-2-yl und Morpholin-3-yl, Thiomorpholinyl einschließlich Thiomorpholin-2-yl und Thiomorpholin-3-yl, die alle gegebenenfalls wie in bezug auf Het angegeben substituiert sind.

Gegenstand der Erfindung sind alle Verbindungen der Formel I, worin ein oder mehrere Strukturelemente wie Gruppen, Substituenten und Zahlen wie in einer der angegebenen Ausführungsformen oder Definitionen der Elemente definiert sind oder eine oder mehrere der spezifischen Bedeutungen, die hier als Beispiele für Elemente angegeben sind, besitzen, wobei alle Kombinationen einer oder mehrerer angegebener Ausführungsformen und/oder Definitionen und/oder spezifischer Bedeutungen der Elemente Gegenstand der vorliegenden Erfindung sind. Auch in bezug auf alle derartigen Verbindungen der Formel I sind alle ihre stereoisomeren Formen und Mischungen von stereoisomeren Formen in beliebigem Verhältnis und ihre physiologisch annehmbaren Salze und die physiologisch annehmbaren Solvate davon Gegenstand der vorliegenden Erfindung.

Ein Beispiel für erfindungsgemäße Verbindungen, die in bezug auf beliebige Strukturelemente wie in den angegebenen Ausführungsformen der Erfindung oder Definitionen derartiger Elemente definiert sind und die Gegenstand der Erfindung sind, sind Verbindungen der Formel I, worin
R¹ ausgewählt ist aus (C₁-C₆)-Alkyl , (C₃-C₇)-Cycloalkyl-CₜH₂ₜ- und Het-CtH₂ₜ-, worin t aus 0, 1 und 2 ausgewählt ist;
die Gruppe Het, die in R¹ vorkommt, für einen Rest eines gesättigten, 4-gliedrigen bis 7-gliedrigen, monocyclischen Heterocyclus, der 1 Ringheteroatom, das aus O und S ausgewählt ist, enthält und über ein Ringkohlenstoffatom gebunden ist, steht, wobei der Rest eines Heterocyclus gegebenenfalls durch einen oder mehrere gleiche oder verschiedene Substituenten substituiert ist, die aus Fluor und (C₁-C₄)-Alkyl ausgewählt sind;
und alle andern Gruppen und Zahlen wie in der allgemeinen Definition der Verbindungen der Formel I oder in einer der angegebenen Ausführungsformen der Erfindung oder Definitionen von Strukturelementen definiert sind, in einer beliebigen ihrer stereoisomeren Formen oder einer Mischung von stereoisomeren Formen in beliebigem Verhältnis und ihre physiologisch akzeptablen Salze und die physiologisch akzeptablen Solvate einer derartigen Verbindung oder eines derartigen Salzes.

Ein anderes derartiges Beispiel sind die Verbindungen der Formel I, worin
R¹ ausgewählt ist aus (C₁-C₆)-Alkyl, (C₃-C₇)-Cycloalkyl-CₜH₂ₜ- und Het-CₜH₂ₜ-, worin t ausgewählt ist aus 0, 1 und 2;
R² ausgewählt ist aus Phenyl und Pyridinyl, worin das Ringstickstoffatom des Pyridinyls einen Oxysubstituenten tragen kann und wobei das Phenyl und das Pyridinyl gegebenenfalls an einem oder mehreren Ringkohlenstoffatomen durch gleiche oder verschiedene Substituenten R²² substituiert sind;
R³ ausgewählt ist aus (C₃-C₇)-Cycloalkyl-CᵤH₂ᵤ- und Het-CᵥH₂ᵥ-, worin u und v aus 1 und 2 ausgewählt sind, oder R³ für einen Rest eines gesättigten oder ungesättigten, 3-gliedrigen bis 1 0-gliedrigen, monocyclischen oder bicyclischen Rings, der 0, 1, 2, 3 oder 4 gleiche oder verschiedene Ringheteroatome, die aus N, O und S ausgewählt sind, enthält, steht, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist;
und alle anderen Gruppen und Zahlen wie in der allgemeinen Definition der Verbindungen der Formel I oder in einer der angegebenen Ausführungsformen der Erfindung oder Definitionen von Strukturelementen definiert sind, in einer beliebigen ihrer stereoisomeren Formen oder einer Mischung von stereoisomeren Formen in beliebigem Verhältnis und ihre physiologisch akzeptablen Salze und die physiologisch akzeptablen Solvate derartiger Verbindungen oder derartiger Salze.

Ein weiteres derartiges Beispiel sind Verbindungen der Formel I, worin
A ausgewählt ist aus NH, O und S;
R¹ ausgewählt ist aus (C₁-C₆)-Alkyl, (C₃-C₇)-Cycloalkyl-CₜH₂ₜ- und Het-CₜH₂ₜ-, worin t aus 0, 1 und 2 ausgewählt ist;
R² ausgewählt ist aus Phenyl und Pyridinyl, wobei das Ringstickstoffatom des Pyridinyls einen Oxysubstituenten tragen kann und wobei das Phenyl und das Pyridinyl gegebenenfalls an einem oder mehreren Ringkohlenstoffatomen durch gleiche oder verschiedene Substituenten R₂₂ substituiert sind;
R³ ausgewählt ist aus (C₃-C₇)-Cycloalkyl-CᵤH₂ᵤ- und Het-CᵥH₂ᵥ-, worin u und v unter 1 und 2 ausgewählt sind, oder R³ für einen Rest eines gesättigten oder ungesättigten, 3-gliedrigen bis 7-gliedrigen, monocyclischen Rings, der 0, 1 oder 2 gleiche oder verschiedene Ringheteroatome enthält, die unter N, O und S ausgewählt sind, steht, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist;
R²² ausgewählt ist aus Halogen, Hydroxy, (C₁-C₄)-Alkyl-, (C₁-C₄)-Alkyloxy, (C₁-C₄)-Alkyl-S(O)ₘ-, Amino, Cyano, Aminocarbonyl, Aminosulfonyl, R²³ und R²³-O-;
R²³ für einen Rest eines gesättigten 3-gliedrigen bis 6-gliedrigen, monocyclischen Rings, der 0, 1 oder 2 gleiche oder verschiedene Ringheteroatome enthält, die aus N, O und S ausgewählt sind, steht, wobei ein oder zwei der Ringstickstoffatome ein Wasserstoffatom oder einen (C₁-C₄)-Alkylsubstituenten tragen können und eines der Ringschwefelatome eine oder zwei Oxogruppen tragen kann und wobei der Rest eines Rings gegebenenfalls an einem oder mehreren Ringkohlenstoffatomen durch gleiche oder verschiedene Substituenten R²⁴ substituiert ist;
R²⁴ ausgewählt ist aus Fluor, (C₁-C₄)-Alkyl und Hydroxy;
R³¹ ausgewählt ist aus Halogen, (C₁-C₄)-Alkyl, (C₃-C₇)-Cycloalkyl, Hydroxy, (C₁-C₄)-Alkyloxy, (C₁-C₄)-Alkyl-S(O)ₘ-, Amino, (C₁-C₄)-Alkylamino, Di((C₁-C₄)-alkyl)amino, (C₁-C₄)-Alkylcarbonylamino, (C₁-C₄)-Alkylsulfonylamino, Cyano, Aminosulfonyl, (C₁-C₄)-Alkylaminosulfonyl und Di((C₁-C₄)-alkyl)aminosulfonyl;
Het für einen Rest eines gesättigten, 4-gliedrigen bis 7-gliedrigen, monocyclischen Heterocyclus, der 1 oder 2 gleiche oder verschiedene Ringheteroatome enthält, die aus N, O und S ausgewählt sind, und der über ein Ringkohlenstoffatom gebunden ist, steht, wobei der Rest eines Heterocyclus gegebenenfalls durch einen oder mehrere gleiche oder verschiedene Substituenten substituiert ist, die aus Fluor und (C₁-C₄)-Alkyl ausgewählt sind;
m ausgewählt ist aus 0, 1 und 2, wobei alle Zahlen m voneinander unabhängig sind;
wobei alle Cycloalkylgruppen unabhängig voneinander und unabhängig von anderen Substituenten gegebenenfalls durch einen oder mehrere gleiche oder verschiedene Substituenten substituiert sind, die aus Fluor und (C₁-C₄)-Alkyl ausgewählt sind;
wobei alle Alkyl-, CₜH₂ₜ-, CᵤH₂ᵤ- und CᵥH₂ᵥ-Gruppen unabhängig voneinander und unabhängig von anderen Substituenten gegebenenfalls durch einen oder mehrere Fluorsubstituenten substituiert sind,
in einer beliebigen ihrer stereoisomeren Formen oder einer Mischung von stereoisomeren Formen in beliebigem Verhältnis und ihre physiologisch akzeptablen Salze und die physiologisch akzeptablen Solvate derartiger Verbindungen oder derartiger Salze.

Ebenso gilt auch in bezug auf alle hier offenbarten spezifischen Verbindungen, wie die Beispielverbindungen, die Ausführungsformen der Erfindung repräsentieren, in denen die verschiedenen Gruppen und Zahlen in der allgemeinen Definition der Verbindungen der Formel I die in der jeweiligen spezifischen Verbindung vorliegenden spezifischen Bedeutungen besitzen, daß sie in einer beliebigen ihrer stereoisomeren Formen und/oder einer Mischung von stereoisomeren Formen in beliebigem Verhältnis und in Form ihrer physiologisch akzeptablen Salze und in Form der physiologisch akzeptablen Solvate derartiger Verbindungen oder derartiger Salze Gegenstand der vorliegenden Erfindung sind. Unabhängig davon, ob eine spezifische Verbindung hier als freie Verbindung und/oder als spezifisches Salz offenbart wird, ist sie sowohl in Form der freien Verbindung als auch in Form aller ihrer physiologisch akzeptablen Salze und bei Offenbarung eines spezifischen Salzes zusätzlich in Form dieses spezifischen Salzes und in Form der physiologisch akzeptablen Solvate einer derartigen Verbindung oder derartiger Salze Gegenstand der Erfindung. Gegenstand der Erfindung ist somit auch eine Verbindung der Formel I, die aus einer oder mehreren der hier offenbarten spezifischen Verbindungen der Formel I einschließlich der nachstehend angeführten Beispielverbindungen ausgewählt ist, und die physiologisch akzeptablen Salze davon und die physiologisch akzeptablen Solvate einer derartigen Verbindung oder derartiger Salze, wobei die Verbindung der Formel II in einer beliebigen ihrer stereoisomeren Formen oder als Mischung von stereoisomeren Formen in beliebigem Verhältnis, sofern anwendbar, Gegenstand der Erfindung ist.

Ein weiterer Gegenstand der vorliegenden Erfindung sind Verfahren zur Herstellung der Verbindungen der Formel I und ihrer Salze und Solvate, nach denen die Verbindungen erhältlich sind und die im folgenden umrissen werden. Bei einem Verfahren setzt man eine Verbindung der Formel II mit einer Verbindung der Formel III zu einer Verbindung der Formel I um, 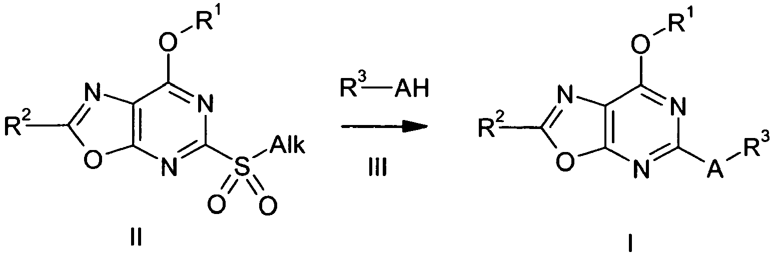 worin die Gruppen A, R¹, R² und R³ in den Verbindungen der Formeln II und III wie in den Verbindungen der Formel I definiert sind und zusätzlich funktionelle Gruppen in geschützter Form oder in Form einer Vorläufergruppe, die später in die endgültige Gruppe umgewandelt wird, vorliegen können. Bei der Gruppe Alk in den Verbindungen der Formel Ila handelt es sich um eine (C₁-C₄)-Alkylgruppe, beispielsweise Methyl oder Ethyl.

Die Reaktion der Verbindungen der Formeln II und III ist eine nukleophile aromatische Substitutionsreaktion an dem Kohlenstoffatom in der 5-Position des Oxazolo[5,4-d]pyrimidinrings, d.h. in der Pyrimidingruppierung, und kann unter Standardbedingungen für derartige Reaktionen, die dem Fachmann gut bekannt sind, durchgeführt werden. Im allgemeinen wird die Reaktion in einem inerten Lösungsmittel, beispielsweise einem Kohlenwasserstoff oder chlorierten Kohlenwasserstoff wie Benzol, Toluol, Xylol, Chlorbenzol, Dichlormethan, Chloroform oder Dichlorethan, einem Ether wie Tetrahydrofuran (THF), Dioxan, Dibutylether, Diisopropylether oder 1,2-Dimethoxyethan (DME), einem Keton wie Aceton oder Butan-2-on, einem Ester wie Essigsäureethylester oder Essigsäurebutylester, einem Nitril wie Acetonitril, einem Amin wie N,N-Dimethylformamid (DMF) oder N-Methylpyrrolidin-2-on (NMP) oder einer Lösungsmittelmischung bei Temperaturen von etwa 20°C bis etwa 160°C, beispielsweise bei Temperaturen von etwa 40°C bis etwa 100°C, je nach den Besonderheiten des jeweiligen Falls, durchgeführt. Im allgemeinen ist es günstig, zur Erhöhung der Nukleophilie der Verbindung der Formel III eine Base zuzusetzen, beispielsweise ein tertiäres Amin, wie Triethylamin, Ethyldiisopropylamin oder N-Methylmorpholin, oder eine anorganische Base wie ein Erdalkalimetallhydrid, - hydroxid, -carbonat oder -hydrogencarbonat wie Natriumhydrid, Natriumhydroxid, Kaliumhydroxid, Natriumcarbonat, Kaliumcarbonat, Cäsiumcarbonat oder Natriumhydrogencarbonat oder ein Alkoxid oder Amid wie Natriummethoxid, Natriumethoxid, Kaliummethoxid, Kalium-tert.-butoxid, Natriumamid oder Lithiumdiisopropylamid. Eine Verbindung der Formel III kann auch vor der Reaktion mit der Verbindung der Formel II separat mit einer Base behandelt und in ein Salz umgewandelt werden.

Die Ausgangsverbindungen der Formeln II und III sind nach in der Literatur beschriebenen Verfahrensweisen oder in Anlehnung daran erhältlich und in vielen Fällen im Handel erhältlich. Die Verbindungen der Formel II sind durch Umsetzung eines Aminomalonsäureesters der Formel IV mit einem aktivierten Carbonsäurederivat der Formel V zu einer Verbindung der Formel VI, Umsetzung der letzteren Verbindung mit Thioharnstoff der Formel VII zu einer Verbindung der Formel VIII, Alkylierung des Thiols mit einem Alkylierungsreagens der Formel IX zum Thioether der Formel X, Cyclisierung der letzteren Verbindung unter Bildung des Oxazolo[5,4-d]pyrimidinringsystems zur Verbindung der Formel XI, Alkylierung der letzteren Verbindung am Sauerstoffatom der Ketogruppe bzw. der tautomeren Hydroxygruppe mit einem Alkylierungsreagens der Formel XII und Oxidation der Thioethergruppierung in der erhaltenen Verbindung der Formel XIII zu dem entsprechenden Sulfon der Formel II erhältlich.

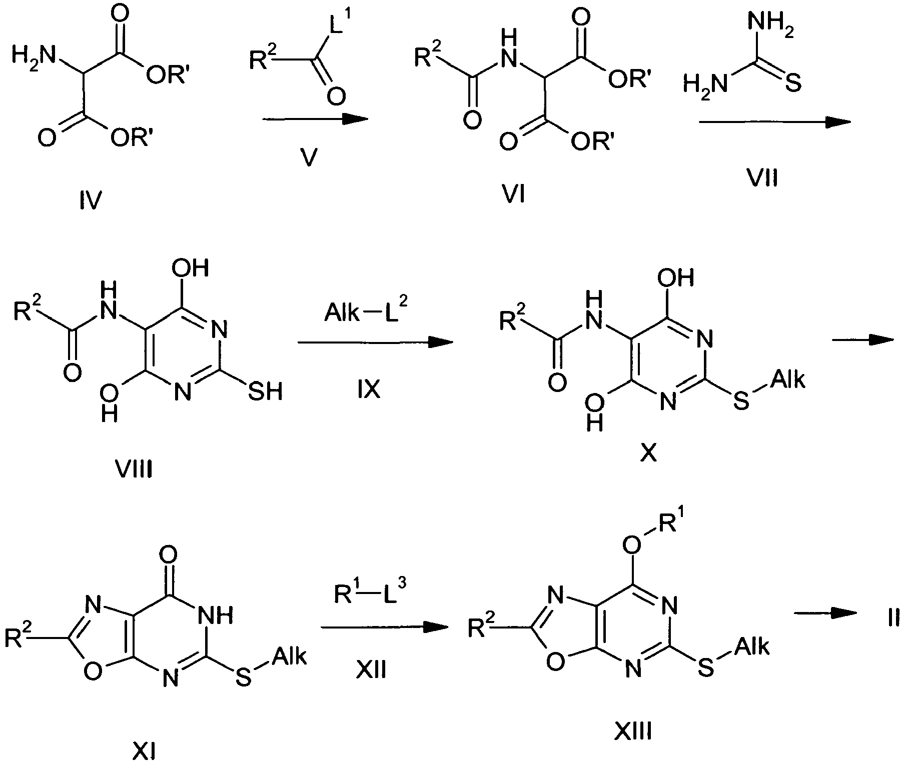

Die Gruppen R¹ und R² in den Verbindungen der Formeln V, VI, VIII, X, XI, XII und XIII sind wie in den Verbindungen der Formel I definiert, und zusätzlich können funktionelle Gruppen in geschützter Form oder in Form einer Vorläufergruppe, die später in die endgültige Gruppe umgewandelt wird, vorliegen. Die Gruppe Alk in den Verbindungen der Formeln IX, X, XI und XIII ist wie in den Verbindungen der Formel II definiert. Die Gruppe R' in den Verbindungen der Formeln IV und VI kann für Alkyl wie beispielsweise (C₁-C₃)-Alkyl, wie Methyl oder Ethyl, stehen. Die Gruppe L¹ in den Verbindungen der Formel V ist eine nukleophil substituierbare Abgangsgruppe und kann insbesondere ein Halogenatom, wie Chlor oder Brom, sein, und die Verbindung der Formel V kann somit ein Carbonsäurehalogenid sein. L¹ kann auch eine Gruppe der Formel R²-C(O)-O sein, und die Verbindung der Formel V kann somit beispielsweise ein Carbonsäureanhydrid sein. Die Gruppen L² und L³ sind Abgangsgruppen, die in einer nukleophilen Substitutionsreaktion ersetzt werden können, und können insbesondere ein Halogenatom wie Chlor, Brom oder lod oder eine Sulfonyloxygruppe wie Methansulfonyloxy, Trifluormethansulfonyloxy oder Toluolsulfonyloxy sein, d.h. die Verbindungen der Formeln IX und XII können beispielsweise organische Halogenide oder Sulfonate sein. Wie erwähnt, können die Verbindungen der Formel XI auch in einer anderen tautomeren Form vorliegen, beispielsweise in Form der jeweiligen 7-Hydroxyoxazolo[5,4-d]pyrimidinderivate, in denen das mobile Wasserstoffatom, das in Formel XI an das Ringstickstoffatom in der 6-Position des Oxazolopyrimidinringsystems gebunden ist, an das an das Ringkohlenstoffatom in der 7-Position gebundene Sauerstoffatom gebunden ist. Soweit anwendbar, gilt für alle Verbindungen, die bei der Herstellung der Verbindungen der Formel I vorkommen, daß sie in einer anderen tautomeren Form als der in ihren Formeln dargestellten vorliegen können. Bei den Reaktionen dieses Verfahrens zur Herstellung der Verbindungen der Formel II können wie bei allen anderen Reaktionen, die bei der Herstellung der Verbindungen der Formel I durchgeführt werden, Ausgangsverbindungen auch in Form eines Salzes eingesetzt und/oder Produkte in Form eines Salzes erhalten werden. So können beispielsweise Verbindungen der Formeln IV in Form eines Säureadditionssalzes wie des Hydrochlorids eingesetzt werden.

Die Umsetzung der Verbindungen der Formeln IV und V kann unter Standardbedingungen für die Acylierung eines Amins mit einem aktivierten Carbonsäurederivat wie einem Säurehalogenid oder-anhydrid durchgeführt werden. Im allgemeinen wird die Umsetzung in einem inerten Lösungsmittel, beispielsweise einem Kohlenwasserstoff oder chlorierten Kohlenwasserstoff wie Benzol, Toluol, Xylol, Chlorbenzol, Dichlormethan, Chloroform oder Dichlorethan, einem Ether wie THF, Dioxan, Dibutylether, Diisopropylether oder DME, einem Keton wie Aceton oder Butan-2-on, einem Ester wie Essigsäureethylester oder Essigsäurebutylester oder Wasser, oder einer Mischung von Lösungsmitteln, bei Temperaturen von etwa -10°C bis etwa 40°C, beispielsweise bei Temperaturen von etwa 0°C bis etwa 30°C durchgeführt. Im allgemeinen wird die Umsetzung unter Zugabe einer Base, beispielsweise eines tertiären Amins, wie Triethylamin, Ethyldiisopropylamin oder N-Methylmorpholin oder einer anorganischen Base wie eines Alkalimetallhydroxids, -carbonats oder - hydrogencarbonats wie Natriumhydroxid, Kaliumhydroxid, Natriumcarbonat, Kaliumcarbonat oder Natriumhydrogencarbonat durchgeführt. Die Umsetzung der Verbindungen der Formeln VI und VII wird im allgemeinen in einem inerten Lösungsmittel, beispielsweise einem Alkohol, wie Methanol, Ethanol oder Isopropanol, oder einem Ether wie THF, Dioxan oder DME oder einer Mischung von Lösungsmitteln bei Temperaturen von etwa 20°C bis etwa 80°C, beispielsweise Temperaturen von etwa 40°C bis etwa 80°C, in Gegenwart einer Base, beispielsweise eines Alkoxids wie Natriummethoxid, Natriumethoxid, Kaliummethoxid oder Kalium-tert.-butoxid, durchgeführt.

Die Umsetzung der Verbindungen der Formeln VIII und IX ist eine nukleophile Substitutionsreaktion am endständigen Kohlenstoffatom in der Gruppe Alkyl, die die Gruppe L² trägt, und kann unter Standardbedingungen für derartige Umsetzungen, die dem Fachmann gut bekannt sind, durchgeführt werden. Im allgemeinen wird die Umsetzung je nach den Besonderheiten des jeweiigen Falls in einem inerten Lösungsmittel, beispielsweise einem Kohlenwasserstoff oder chloriertem Kohlenwasserstoff wie Benzol, Toluol, Xylol, Chlorbenzol, Dichlormethan, Chloroform oder Dichlorethan, einem Ether wie THF, Dioxan, Dibutylether, Diisopropylether oder DME, einem Alkohol wie Methanol, Ethanol oder Isopropanol, einem Keton wie Aceton oder Butan-2-on, einem Ester wie Essigsäureethylester oder Essigsäurebutylester, einem Nitril wie Acetonitril, einem Amid wie DMF oder NMP, oder einer Mischung von Lösungsmitteln einschließlich zweiphasiger Mischungen mit wäßrigen Lösungen bei Temperaturen von etwa -20°C bis etwa 100°C, beispielsweise bei Temperaturen von etwa -10°C bis etwa 30°C durchgeführt. Im allgemeinen ist es günstig, zur Erhöhung der Nukleophilie der Verbindung der Formel VIII und/oder zur Bindung einer Säure, die bei der Umsetzung freigesetzt wird, eine Base, beispielsweise ein tertiäres Amin, wie Triethylamin, Ethyldiisopropylamin oder N-Methylmorpholin, oder eine anorganische Base wie ein Alkalimetallhydrid, -hydroxid, -carbonat oder -hydrogencarbonat wie Natriumhydrid, Natriumhydroxid, Kaliumhydroxid, Natriumcarbonat, Kaliumcarbonat, Cäsiumcarbonat oder Natriumhydrogencarbonat oder ein Alkoxid oder Amid wie Natriummethoxid, Natriumethoxid, Kaliummethoxid, Kalium-tert-butoxid, Natriumamid oder Lithiumdiisopropylamid zuzusetzen. Eine Verbindung der Formel VIII kann auch vor der Umsetzung mit der Verbindung der Formel IX separat mit einer Base behandelt und in ein Salz umgewandelt werden.

Die Cyclisierung der Verbindung der Formel X zu der Verbindung der Formel XI kann günstigerweise in Gegenwart eines Phosphorhalogenids, wie Phosphorpentachlorid oder Phosphoroxidchlorid oder einer Mischung davon, in einem inerten Lösungsmittel, beispielsweise einem Kohlenwasserstoff oder chloriertem Kohlenwasserstoff wie Benzol, Toluol, Xylol, Chlorbenzol, Dichlormethan, Chloroform oder Dichlorethan bei Temperaturen von etwa 20°C bis etwa 100°C, beispielsweise Temperaturen von etwa 50°C bis etwa 80°C, durchgeführt werden.

Die Umsetzung der Verbindungen der Formeln XI und XII ist eine weitere nukleophile Substitutionsreaktion an dem Kohlenstoffatom in der Gruppe R¹ das die Gruppe L³ trägt, und kann unter Standardbedingungen für derartige Umsetzungen, die dem Fachmann gut bekannt sind, durchgeführt werden. Im allgemeinen wird die Umsetzung je nach den Besonderheiten des jeweiligen Falls in einem inerten Lösungsmittel, beispielsweise einem Kohlenwasserstoff oder chloriertem Kohlenwasserstoff wie Benzol, Toluol, Xylol, Chlorbenzol, Dichlormethan, Chloroform oder Dichlorethan, einem Ether wie THF, Dioxan, Dibutylether, Diisopropylether oder DME, einem Alkohol wie Methanol, Ethanol oder Isopropanol, einem Keton wie Aceton oder Butan-2-on, einem Ester wie Essigsäureethylester oder Essigsäurebutylester, einem Nitril wie Acetonitril, einem Amid wie DMF oder NMP, oder einer Mischung von Lösungsmitteln bei Temperaturen von etwa 20°C bis etwa 100°C, beispielsweise bei Temperaturen von etwa 40°C bis etwa 80°C durchgeführt. Im allgemeinen ist es günstig, zur Erhöhung der Nukleophilie der Verbindung der Formel XI und/oder zur Bindung einer Säure, die bei der Umsetzung freigesetzt wird, eine Base, beispielsweise ein tertiäres Amin, wie Triethylamin, Ethyldiisopropylamin oder N-Methylmorpholin, oder eine anorganische Base wie ein Alkalimetallhydrid, -hydroxid, -carbonat oder - hydrogencarbonat wie Natriumhydrid, Natriumhydroxid, Kaliumhydroxid, Natriumcarbonat, Kaliumcarbonat, Cäsiumcarbonat oder Natriumhydrogencarbonat oder ein Alkoxid oder Amid wie Natriummethoxid, Natriumethoxid, Kaliummethoxid, Kalium-tert.-butoxid, Natriumamid oder Lithiumdiisopropylamid zuzusetzen. Eine Verbindung der Formel XI kann noch vor der Umsetzung mit der Verbindung der Formel XII separat mit einer Base behandelt werden und in ein Salz umgewandelt werden. Eine Verbindung der Formel XI kann nicht nur durch Umsetzung mit einer Verbindung der Formel XII hergestellt werden, sondern auch durch Umsetzung mit dem jeweiligen Alkohol der Formel R¹-OH, worin R¹ wie in den Verbindungen der Formel I definiert ist und zusätzlich funktionelle Gruppen in geschützter Form oder in Form einer Vorläufergruppe vorliegen können, unter den Bedingungen der Mitsunobu-Reaktion in Gegenwart eines Azodicarboxylats wie Diethylazodicarboxylat oder Düsopropylazodicarboxylat und eines Phosphins wie Triphenylphosphin oder Tributylphosphin in einem inerten aprotischen Lösungsmittel, beispielsweise einem Ether wie THF oder Dioxan, in eine Verbindung der Formel XIII umgewandelt werden (siehe O. Mitsunobu, Synthesis (1981), 1-28).

Die Oxidation der Alk-S-Gruppe in den Verbindungen der Formel XIII zur Sulfongruppe in den Verbindungen der Formel II kann mit Hilfe von Wasserstoffperoxid oder einer Persäure wie 3-Chlorperbenzoesäure oder Monoperoxyphthalsäure in einem inerten Lösungsmittel, beispielsweise einem chlorierten Kohlenwasserstoff wie Dichlormethan oder Chloroform oder einem Ester wie Essigsäureethylester oder Essigsäurebutylester, bei Temperaturen von etwa 0°C bis etwa 40°C, beispielsweise bei etwa 20°C, durchgeführt werden.

Man kann auch die Reihenfolge der Schritte bei der Herstellung der Verbindungen der Formel X ändern und zunächst einen Aminomalonsäureester der Formel IV wie den Diethylester in Gegenwart eines Alkalimetallalkoxids wie Natriumethoxid mit Thioharnstoff umsetzen, dann das Schwefelatom alkylieren, beispielsweise mit lodmethan methylieren, und das erhaltene Produkt mit einer Verbindung der Formel V acylieren (siehe M. H. Holschbach et al., Eur. J. Med. Chem. 41 (2006), 7-15).

Weitere Verbindungen der Formel I sind aus geeigneten, nach den obenbeschriebenen Verfahren hergestellten Verbindungen durch Funktionalisierung oder Modifizierung von enthaltenen funktionellen Gruppen nach Standardverfahrensweisen erhältlich, beispielsweise durch Veresterung, Amidierung, Hydrolyse, Veretherung, Alkylierung, Acylierung, Sulfonylierung, Reduktion, Oxidation, Umwandlung in Salze u.a. So kann beispielsweise eine Hydroxygruppe, die aus einer Ethergruppe durch Etherspaltung, beispielsweise mit Hilfe von Bortribromid, oder aus einer geschützten Hydroxygruppe durch Entschützung freigesetzt werden kann, zu einem Carbonsäureester oder einem Sulfonsäureester verestert oder verethert werden. Veretherungen von Hydroxygruppen können günstigerweise durch Alkylierung mit der jeweiligen Halogenverbindung, beispielsweise einem Bromid oder lodid, in Gegenwart einer Base, beispielsweise eines Alkalimetallcarbonats wie Kaliumcarbonat oder Cäsiumcarbonat, in einem inerten Lösungsmittel, beispielsweise einem Amid wie DMF oder NMP oder einem Keton wie Aceton oder Butan-2-on oder mit dem jeweiligen Alkohol unter den oben angesprochenen Bedingungen der Mitsunobu-Reaktion durchgeführt werden. Eine Hydroxygruppe kann durch Behandlung mit einem Halogenierungsmittel in ein Halogenid umgewandelt werden. Ein Halogenatom kann in einer Substitutionsreaktion, bei der es sich auch um eine übergangsmetallkatalysierte Reaktion handeln kann, durch verschiedene Gruppen ersetzt werden. Eine Nitrogruppe kann zu einer Aminogruppe reduziert werden, beispielsweise durch katalytische Hydrierung. Eine Aminogruppe kann unter Standardbedingungen für die Alkylierung, beispielsweise durch Umsetzung mit einer Halogenverbindung oder durch reduktive Aminierung einer Carbonylverbindung, oder für die Acylierung oder Sulfonylierung, beispielsweise durch Umsetzung mit einem reaktiven Carbonsäurederivat wie einem Säurechlorid oder Anhydrid oder einem Sulfonsäurechlorid oder mit einer aktivierten Carbonsäure, die aus der Carbonsäure beispielsweise durch Behandlung mit einem Kupplungsmittel wie N,N'-Carbonyldiimidazol (CDI), einem Carbodiimid wie 1,3-Dicyclohexylcarbodiimid (DCC) oder 1-(3-Dimethylaminopropyl)-3-ethylcarbodiimid-hydrochlorid (EDC), O-(7-Azabenzotriazol-1-yl)-N,N,N',N'-tetramethyluroniumhexafluorophosphat (HATU), O-(Cyano(ethoxycarbonyl)methylenamino)-N,N,N',N'-tetramethyluroniumtetrafluoroborat (TOTU) oder [(Benzotriazol-1-yloxy)dimethylaminomethylen]dimethylammoniumtetrafluoroborat (TBTU) erhältlich ist, modifiziert werden. Eine Carbonsäureestergruppe kann unter sauren oder basischen Bedingungen zu einer Carbonsäure hydrolysiert werden. Eine Carbonsäuregruppe kann wie oben erwähnt aktiviert oder in ein reaktives Derivat umgewandelt und mit einem Alkohol oder einem Amin oder Ammoniak zu einem Ester oder Amid umgesetzt werden. Ein primäres Amid kann zu einem Nitril dehydratisiert werden. Ein Schwefelatom, beispielsweise in einer Alkyl-S-Gruppe oder in einem heterocyclischen Ring, kann mit einem Peroxid wie Wasserstoffperoxid oder einer Persäure zu einer Sulfoxidgruppierung S(O) oder einer Sulfongruppierung S(O)₂ oxidiert werden. Eine Carbonsäuregruppe, eine Carbonsäureestergruppe und eine Ketongruppe können zu einem Alkohol reduziert werden, beispielsweise mit Hilfe eines komplexen Hydrids wie Lithiumaluminiumhydrid, Lithiumborhydrid oder Natriumborhydrid.

Alle bei den oben beschriebenen Synthesen der Verbindungen der Formel I verwendeten Reaktionen sind dem Fachmann an sich gut bekannt und können unter Standardbedingungen gemäß oder in Analogie zu in der Literatur, beispielsweise in Houben-Weyl, Methoden der Organischen Chemie (Methods of Organic Chemistry), Thieme-Verlag, Stuttgart, oder Organic Reactions, John Wiley & Sons, New York, beschriebenen Verfahrensweisen durchgeführt werden. Falls gewünscht, können die erhaltenen Verbindungen der Formel I sowie etwaige Zwischenverbindungen nach herkömmlichen Reinigungsverfahrensweisen gereinigt werden, beispielsweise durch Umkristallisieren oder Chromatographie. Wie bereits erwähnt, können alle bei den oben beschriebenen Synthesen eingesetzten Ausgangsverbindungen und Zwischenprodukte, die eine saure oder basische Gruppe enthalten, auch in Form von Salzen eingesetzt werden und alle Zwischenprodukte und entgültigen Zielverbindungen können auch in Form von Salzen erhalten werden. Wie ebenfalls oben erwähnt, kann es je nach den Umständen des jeweiligen Falls zur Vermeidung eines unerwünschten Verlaufs einer Reaktion oder von Nebenreaktionen im Lauf der Synthese einer Verbindung im allgemeinen erforderlich oder vorteilhaft sein, funktionelle Gruppen durch die Einführung von Schutzgruppen zeitweilig zu blockieren und sie in einer späteren Stufe der Synthese wieder zu entschützen oder funktionelle Gruppen in Form von Vorläufergruppen, die später in die gewünschten funktionellen Gruppen umgewandelt werden, einzuführen. Als Beispiele für Schutzgruppen seien Aminoschutzgruppen genannt, bei denen es sich um Acylgruppen oder Alkyloxycarbonylgruppen, beispielsweise eine tert.-Butyloxycarbonylgruppe (=Boc), die durch Behandung mit Trifluoressigsäure (=TFA) abgespalten werden kann, eine Benzyloxycarbonylgruppe, die durch katalytische Hydrierung abgespalten werden kann, oder eine Fluoren-9-ylmethoxycarbonylgruppe,die durch Behandlung mit Piperidin abgespalten werden kann, handeln kann, und Schutzgruppen von Carbonsäuregruppen, die als Estergruppen, wie tert.-Butylester, die durch Behandlung mit Trifluoressigsäure entschützt werden können, oder Benzylester, die durch katalytische Hydrierung entschützt werden können, geschützt werden können. Als Beispiel für eine Vorläufergruppe sei die Nitrogruppe genannt, die durch Reduktion, beispielsweise durch katalytische Hydrierung, in eine Aminogruppe umgewandelt werden kann. Derartige Synthesestrategien und Schutzgruppen und Vorläufergruppen, die in einem bestimmten Fall geeignet sind, sind dem Fachmann bekannt.

Ein weiterer Gegenstand der vorliegenden Erfindung sind die neuen Ausgangsverbindungen und Zwischenprodukte, die bei der Synthese der Verbindungen der Formel I vorkommen, einschließlich der Verbindungen der Formeln II, III, IV, V, VI, VIII, IX, X, XI, XII und XIII, worin A, R¹ R², R³, R', L¹, L² und L³ wie oben definiert sind, in einer beliebigen ihrer stereoisomeren Formen oder einer Mischung von stereoisomeren Formen in beliebigem Verhältnis und ihre Salze und Solvate derartiger Verbindungen oder derartiger Salze und ihre Verwendung als Zwischenprodukte. Die Erfindung schließt auch alle tautomeren Formen der Zwischenprodukte und Ausgangsverbindungen ein. Alle oben bezüglich der Verbindungen der Formel I angegebenen Erklärungen und Ausführungsformen gelten entsprechend auch für die Zwischenprodukte und Ausgangsverbindungen. Gegenstand der Erfindung sind insbesondere die hier offenbarten neuen spezifischen Ausgangsverbindungen und Zwischenprodukte. Unabhängig davon, ob sie als freie Verbindung und/oder als spezifisches Salz offenbart sind, sind sie sowohl in Form der freien Verbindungen als auch in Form ihrer Salze und im Fall der Offenbarung eines spezifischen Salzes zusätzlich in Form dieses spezifischen Salzes und in Form von Solvaten derartiger Verbindungen oder derartiger Salze Gegenstand der Erfindung.

Die Verbindungen der Formel I, gegebenenfalls in Kombination mit anderen pharmakologisch wirksamen Verbindungen, können Tieren, vorzugsweise Säugetieren einschließlich Menschen, als Pharmazeutika für sich alleine, in Mischungen miteinander oder in Form pharmazeutischer Zusammensetzungen verabreicht werden. Die Verabreichung kann oral, beispielsweise in Form von Tabletten, Filmtabletten, Dragees, Granulaten, Hart- und Weichgelatinekapseln, Lösungen einschließlich wäßriger, alkoholischer und öliger Lösungen, Säften, Tropfen, Sirupen, Emulsionen oder Suspensionen, rektal, beispielsweise in Form von Suppositorien, oder parenteral, beispielsweise in Form von Lösungen zur subkutanen, intramuskulären oder intravenösen Injektion oder Infusion, insbesondere wäßrigen Lösungen, durchgeführt werden. Die Verbindungen der Formel I können des weiteren in Modi der lokalen Arzneistoffzufuhr verwendet werden, beispielsweise in beschichteten Stents zur Verhinderung oder Verringerung der In-Stent-Restenose oder durch lokale Anwendung mit Hilfe eines Katheters. Die geeignete Verabreichungsform hängt u.a. von der zu behandelnden Erkrankung und ihrer Schwere ab.

Die Menge einer Verbindung der Formel I und/oder ihrer physiologisch akzeptablen Salze und/oder Solvate in den pharmazeutischen Zusammensetzungen liegt normalerweise im Bereich von etwa 0,2 bis etwa 800 mg, beispielsweise von etwa 0,5 bis etwa 500 mg, beispielsweise von etwa 1 bis etwa 200 mg, pro Einheitsdosis, kann aber je nach Art der pharmazeutischen Zusammensetzung auch höher sein. Die pharmazeutischen Zusammensetzungen enthalten in der Regel etwa 0,5 bis etwa 90 Gew.-% der Verbindung der Formel I und/oder ihrer physiologisch akzeptablen Salze und/oder Solvate. Die pharmazeutischen Zusammensetzungen können auf an sich bekannte Art und Weise hergestellt werden. Hierzu bringt man eine oder mehrere Verbindungen der Formel I und/oder ihre physiologisch akzeptablen Salze und/oder Solvate zusammen mit einer oder mehreren festen oder flüssigen pharmazeutischen Trägersubstanzen oder Vehikeln und/oder Additiven oder Hilfssubstanzen und dann, wenn ein Kombinationsmedikament gewünscht ist, anderen pharmakologisch wirksamen Verbindungen mit therapeutischer oder prophylaktischer Wirkung in eine für die Verabreichung und Dosierung geeignete Form, die dann in der Human- oder Tiermedizin verwendet werden kann. Als Trägersubstanzen und Additive können geeignete organische und anorganische Substanzen verwendet werden, die mit den Verbindungen der Formel I oder ihren physiologisch akzeptablen Salzen oder Solvaten nicht in unerwünschter Weise reagieren. Als Beispiele für Additivtypen, die in den pharmazeutischen Zusammensetzungen und Medikamenten enthalten sein können, seien Gleitmittel, Konservierungsstoffe, Verdicker, Stabilisatoren, Sprengmittel, Netzmittel, Mittel zur Erzielung eines Depoteffekts, Emulgatoren, Salze, beispielsweise zur Beeinflussung des osmotischen Drucks, Puffersubstanzen, Farbmittel, Geschmacksstoffe und aromatische Substanzen genannt. Beispiele für Trägersubstanzen und Additive sind Wasser, physiologische Natriumchloridlösung, Pflanzenöle, Wachse, Alkohole wie Ethanol, Isopropanol, 1,2-Propandiol, Benzylalkohole oder Glycerin, Polyole, Mannit, Polyethylenglykole, Polypropylenglykole, Glycerintriacetat, Polyvinylpyrrolidon, Gelatine, Cellulose, Kohlenhydrate wie Lactose, Glucose, Saccharose oder Stärke wie Maisstärke, Stearinsäure und Stearinsäuresalze wie Magnesiumstearat, Talk, Lanolin, Vaseline oder Mischungen davon, beispielsweise Mischungen von Wasser mit einem oder mehreren organischen Lösungsmitteln wie Mischungen von Wasser mit Alkoholen. Man kann die Verbindungen der Formel I und ihre physiologisch akzeptablen Salze und Solvate auch lyophilisieren und die erhaltenen Lyophilisate beispielsweise zur Herstellung von Injektionszusammensetzungen verwenden.

Die Dosierung einer zu verabreichenden Verbindung der Formel I und/oder eines physiologisch akzeptablen Salzes und/oder Solvats davon hängt vom Einzelfall ab und ist wie üblich zur Erzielung einer optimalen Wirkung vom Arzt nach den üblichen Regeln und Verfahrensweisen den individuellen Gegebenheiten anzupassen. So hängt sie beispielsweise ab von der Art und Schwere der zu behandelnden Störung, von Geschlecht, Alter, Gewicht und individueller Ansprechbarkeit des zu behandelnden Menschen oder Tiers, von der Effizienz und Wirkdauer der verwendeten Verbindung, davon, ob die Behandlung für die Therapie einer akuten oder chronischen Erkrankung oder prophylaktisch ist, oder davon, ob neben einer Verbindung der Formel I weitere Wirkstoffe verabreicht werden. Im allgemeinen ist eine Tagesdosis von beispielsweise etwa 0,01 mg/kg bis etwa 100 mg/kg oder von etwa 0,1 mg/kg bis etwa 10 mg/kg oder von etwa 0,3 mg/kg bis etwa 5 mg/kg (jeweils mg pro kg Körpergewicht) zur Verabreichung an einen 75 kg schweren Erwachsenen zur Erzielung der gewünschten Ergebnisse angemessen. Die Tagesdosis kann dabei als Einzeldosis verabreicht oder, insbesondere bei Verabreichung größerer Mengen, in mehrere, beispielsweise zwei, drei oder vier, Einzeldosen aufgeteilt werden. Die Verabreichung kann auch kontinuierlich durchgeführt werden, beispielsweise durch kontinuierliche Infusion oder Injektion. Im Einzelfall kann es je nach dem individuellen Verhalten erforderlich sein, von den angegebenen Dosierungen nach oben oder nach unten abzuweichen.

Die folgenden Beispiele erläutern die Erfindung.

Wenn Beispielsverbindungen mit einer basischen Gruppe durch präparative Hochdruck-Flüssigkeitschromatographie (HPLC) an Umkehrphasen-Säulenmaterial (RP-Säulenmaterial) gereinigt wurden und es sich bei dem Elutionsmittel wie üblich um eine Gradientenmischung von Wasser und Acetonitril mit Trifluoressigsäure (TFA) handelte, wurden sie je nach den Einzelheiten der Aufarbeitung wie Verdampfungs-oder Lyophilisierungsbedingungen zum Teil in Form ihres Säureadditionssalzes mit Trifluoressigsäure erhalten. In den Namen der Beispielverbindungen und ihren Strukturformeln ist jegliche derartige enthaltene Trifluoressigsäure nicht angegeben. Die hergestellten Verbindungen wurden im allgemeinen durch spektroskopische Daten und chromatografische Daten, insbesondere Massenspektrum (MS) und HPLC-Retentionszeiten (Rt; in min), die durch kombinierte analytische HPLC/MS-Charakterisierung (LC/MS) erhalten wurden, und/oder NMR-Spektren (NMR=kernmagnetische Resonanz), charakterisiert. Bei der NMR-Charakterisierung sind die chemische Verschiebung δ (in ppm), die Zahl der Wasserstoffatome und die Multiplizität (s = Singulett, d = Dublett, dd = doppeltes Dublett, t = Triplett, dt = doppeltes Triplett, q = Quartett, m = Multiplett; br = breit) der Signale angegeben. Bei der MS-Charakterisierung ist im allgemeinen die Massenzahl (m/z) des Peaks des Molekülions M, z.B. M⁺, oder eines verwandten Ions wie des Ions M+1, z.B. [M+1]⁺, d.h. des protonierten Molekülions [M+H]⁺, das je nach der verwendeten lonisierungsmethode gebildet wurde, angegeben. Bei der lonisierungsmethode handelte es sich im allgemeinen um Elektrospray-lonisierung (ESI). Es wurden die folgenden LC/MS-Bedingungen verwendet.

### Methode LC1

Säule: Waters XBridge C18, 50 x 4,6 mm, 2,5 µm; Durchfluß: 1,3 ml/min; Elutionsmittel A: Acetonitril + 0,08 % Ameisensäure; Elutionsmittel B: Wasser + 0,1 % Ameisensäure; Gradient: von 3% A + 97% B bis 60% A + 40% B in 3,5 min, dann von 60% A + 40% B bis 98% A + 2% B in 0,5 min, dann 98% A + 2% B über einen Zeitraum von 1 min; MSlonisierungsmethode: ESI⁺

### Methode LC2

Säule: Waters XBridge C18, 50 x 4,6 mm, 2,5 µm; Durchfluß: 1,7 ml/min; Elutionsmittel A: Acetonitril + 0,05 % TFA; Elutionsmittel B: Wasser + 0,05% TFA; Gradient: 5% A + 95% B über einen Zeitraum von 0,3 min, dann von 5% A + 95% B bis 95% A + 5% B in 3,2 min, dann 95% A + 5% B über einen Zeitraum von 0,5 min; MSlonisationsmethode: ESI⁺

### Methode LC3

Säule: YMC-Pack J'sphere H80, 33 x 2,1 mm, 4 µm; Durchfluß: 1,3 ml/min; Elutionsmittel A: Acetonitril + 0,05% TFA; Elutionsmittel B: Wasser + 0,05% TFA; Gradient: von 5% A + 95% B bis 95% A + 5% B in 2,5 min; MS-lonisationsmethode: ESI⁺

### Methode LC4

Säule: YMC-Pack J'sphere H80, 33 x 2,1 mm, 4 µm; Durchfluß: 1,0 ml/min; Elutionsmittel A: Acetonitril + 0,05% TFA; Elutionsmittel B: Wasser + 0,05 % TFA; Gradient: von 5% A + 95% B bis 95% A + 5% B in 3,7 min; MS-lonisationsmethode: ESI⁺

### Methode LC5

Säule: YMC-Pack J'sphere H80, 33 x 2,1 mm, 4 µm; Durchfluß: 1,0 ml/min; Elutionsmittel A: Acetonitril + 0,05% TFA; Elutionsmittel B: Wasser + 0,05% TFA; Gradient: von 2% A + 98% B bis 95% A + 5% B in 5 min, dann 95% A + 5% B über einen Zeitraum von 1,25 min; MS-Ionisationsmethode: ESI⁺

### Methode LC6

Säule: YMC-Pack J'sphere H80, 33 x 2,1 mm, 4 µm; Durchfluß: 1,3 ml/min; Elutionsmittel A: Acetonitril + 0,08% Ameisensäure; Elutionsmittel B: Wasser + 0,1 % Ameisensäure; Gradient: von 5% A + 95% B bis 95% A + 5% B in 2,5 min; MSlonisationsmethode: ESI⁺

### Methode LC7

Säule: YMC-Pack J'sphere H80, 33 x 2,1 mm, 4 µm; Durchfluß: 1,0 ml/min; Elutionsmittel A: Acetonitril + 0,05% TFA; Elutionsmittel B: Wasser + 0,05% TFA; Gradient: von 5% A + 95% B bis 95% A + 5 % B in 3,4 min, dann 95% A + 5% B über einen Zeitraum von 1,0 min; MS-Ionisationsmethode: ESI⁺

### Methode LC8

Säule: YMC-Pack J'sphere H80, 33 x 2,1 mm, 4 µm; Durchfluß: 1,3 ml/min; Elutionsmittel A: Acetonitril + 0,05% TFA; Elutionsmittel B: Wasser + 0,05% TFA; Gradient: von 5% A + 95% B bis Gradient: von 5% A + 95% B in 0,5 min, dann von Gradient: von 5% A + 95% B bis 95% A + 5% B in 3 min, dann 95% A + 5% B über einen Zeitraum von 0,5 min; MS-Ionisationsmethode: ESI⁺

### Methode LC9

Säule: Waters XBridge C18, 50 x 4,6 mm, 2,5 µm, Durchfluß: 1,3 ml/min; Elutionsmittel A: Acetonitril + 0,1% Ameisensäure; Elutionsmittel B: Wasser + 0,1% Ameisensäure; Gradient: von 2% A + 98% B bis 60% A + 40% B in 3,5 min, dann von 60% A + 40% B bis 98% A + 2% B in 1 min; MS-Ionisationsmethode: ESI⁺

### Methode LC10

Säule: Waters XBridge C18, 50 x 4,6 mm, 2,5 µm; Durchfluß: 1,7 ml/min; Elutionsmittel A: Acetonitril + 0,05% TFA; Elutionsmittel B: Wasser + 0,05% TFA; Gradient: 5% A + 95% B über einen Zeitraum von 0,2 min, dann von 5% A + 95% B bis 95% A + 5% B in 2,2 min, dann 95% A + 5% B über einen Zeitraum von 1,1 min; MSlonisationsmethode: ESI⁺

### Beispiel 1

### 5-(3,4-Dichlorphenoxy)-7-propoxy-2-(3-methyl-phenyl)oxazolo[5,4-d]pyrimidin

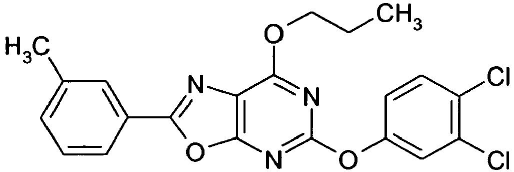

(a) 2-(3-Methylbenzoylamino)malonsäurediethylester
   41,1 g Aminomalonsäurediethylester-hydrochlorid wurden in 200 ml Dichlormethan gelöst und unter Kühlung in einem Eisbad mit 80,7 ml Triethylamin versetzt. Dann wurde eine Lösung von 30 g 3-Methylbenzoylchlorid in 200 ml Dichlormethan langsam zugetropft. Nach 2 h bei 0°C wurden 100 ml Wasser zugetropft. Nach Phasentrennung wurde die wäßrige Phase mit 100 ml Dichlormethan extrahiert. Die vereinigten organischen Phasen wurden mit Natriumsulfat getrocknet, filtriert und eingedampft, was 54 g der rohen Titelverbindung ergab.
(b) N-(4,6-Dihydroxy-2-mercaptopyrimidin-5-yl)-3-methylbenzamid
   7,79 g Thiohamstoff in 150 ml absolutem Ethanol wurden mit 1,5 Äquivalenten Natriummethoxid (30%ig in Methanol) versetzt. Nach Zutropfen einer Lösung von 30 g 2-(3-Methylbenzoylamino)malonsäurediethylester in 100 ml absolutem Ethanol wurde die Mischung 2 h bei 60°C gerührt. Dann wurde die Mischung 30 min auf 0°C abgekühlt, wonach der Niederschlag abgesaugt, gewaschen und getrocknet wurde. Es wurden 28,6 g der rohen Titelverbindung erhalten.
(c) N-(4,6-Dihydroxy-2-methylsulfanylpyrimidin-5-yl)-3-methylbenzamid

   28,6 g N-(4,6-Dihydroxy-2-mercaptopyrimidin-5-yl)-3-methylbenzamid in 280 ml Wasser wurden auf 0°C abgekühlt. Unter Kühlen wurden 10,3 g Natriumhydroxid zugegeben, wonach die Mischung 30 min bei 0°C gerührt wurde. Dann wurde eine Lösung von 6,4 ml lodmethan in 108 ml N-Methylpyrrolidin-2-on zugegeben. Nach Abschluß der Reaktion (6 h) wurde die Mischung mit 6 N Salzsäure angesäuert und der Niederschlag abfiltriert und getrocknet. Es wurden 21,3 g der Titelverbindung erhalten.
(d) 5-Methylsulfanyl-2-(3-methylphenyl)oxazolo[5,4-d]pyrimidin-7-ol 21 g N-(4,6-Dihydroxy-2-methylsulfanylpyrimidin-5-yl)-3-methylbenzamid in 100 ml Phosphoroxidchlorid wurden 3 h auf 70°C erhitzt. Nach dem Abkühlen wurde die Mischung in 500 ml Diethylether gegossen. Der Niederschlag wurde abfiltriert und mit Diethylether gewaschen. Es wurden 7,6 g der Titelverbindung erhalten.
   LC/MS (Methode LC3): Rt = 1,62 min; m/z = 274,10 [M+H]⁺
(e) 5-Methylsulfanyl-7-propoxy-2-(3-methylphenyl)oxazolo[5,4-d]pyrimidin
   7,1 g 5-Methylsulfanyl-2-(3-methylphenyl)oxazolo[5,4-d]pyrimidin-7-ol wurden in 50 ml Dimethylformamid gelöst und mit 14,4 g Kaliumcarbonat und dann mit 3,2 g 1-Brompropan versetzt. Die Suspension wurde 5 h bei 60°C gerührt und dann nach dem Abkühlen auf 150 ml Wasser gegossen. Der Niederschlag wurde abgesaugt und die erhaltene Mischung von Regioisomeren wurde durch Kieselgelchromatographie (Heptan/Essigsäureethylester-Gradient) getrennt. Neben 2,3 g 5-Methylsulfanyl-6-propyl-2-(3-methylphenyl)-6H-oxazolo[5,4-d]pyrimidin-7-on (LC/MS (Methode LC1): Rt = 2,16 min; m/z = 316,14 [M+H]⁺) wurden 3,4 g der Titelverbindung erhalten.
   LC/MS (Methode LC3): Rt = 2,54 min; m/z = 316,14 [M+H]⁺
(f) 5-Methansulfonyl-7-propoxy-2-(3-methylphenyl)oxazolo[5,4-d]pyrimidin
   3,9 g 5-Methylsulfanyl-7-propoxy-2-(3-methylphenyl)oxazolo[5,4-d]pyrimidin wurden in 100 ml Dichlormethan gelöst und mit 6,4 g 3-Chlorperbenzoesäure versetzt, wonach die Mischung 2 h bei Raumtemperatur gerührt wurde. Der Niederschlag wurde abfiltriert und mit Dichlormethan gewaschen. Die vereinigten Filtrate wurden zweimal mit jeweils 100 ml 0,1 N Natronlauge gewaschen, über Natriumsulfat getrocknet, filtriert und im Vakuum eingedampft. Es wurden 4,1 g der Titelverbindung erhalten.
   LC/MS (Methode LC3): Rt = 1,96 min; m/z = 348,07 [M+H]⁺
(g) 5-(3,4-Dichlorphenoxy)-7-propoxy-2-(3-methylphenyl)oxazolo[5,4-d]pyrimidin
   Eine Lösung von 49 mg 3,4-Dichlorphenol in 3 ml DMF wurde mit 103 mg Cäsiumcarbonat und 100 mg 5-Methansulfonyl-7-propoxy-2-(3-methylphenyl)oxazolo[5,4-d]pyrimidin versetzt. Die Mischung wurde 2 h bei Raumtemperatur gerührt. Nach Filtration wurde das Lösungsmittel im Vakuum abdestilliert und das Produkt mittels präparativer HPLC isoliert, was 54 mg der Titelverbindung ergab.
   LC/MS (Methode LC3): Rt = 2,76 min; m/z = 430,06 [M+H]⁺

### Beispiel 2

### 5-Cyclohexyloxy-7-propoxy-2-(3-methylphenyl)oxazolo[5,4-d]pyrimidin

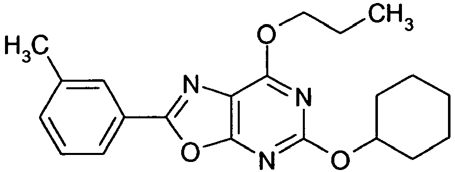

Eine Lösung von 29 mg Cyclohexanol in 3 ml DMF wurde mit 113 mg Cäsiumcarbonat und 100 mg 5-Methansulfonyl-7-propoxy-2-(3-methylphenyl)oxazolo[5,4-d]pyrimidin versetzt. Die Mischung wurde 18 h bei Raumtemperatur gerührt. Nach Filtration wurde das Lösungsmittel im Vakuum abdestilliert und das Produkt mittels präparativer HPLC isoliert, was 10 mg der Titelverbindung ergab.
LC/MS (Methode LC3): Rt = 2,75 min; m/z = 368,20 [M+H]⁺

### Beispiel 3

### (7-Propoxy-2-(3-methylphenyl)oxazolo[5,4-d]pyrimidin-5-yl)(tetrahydropyran-4-yl)amin

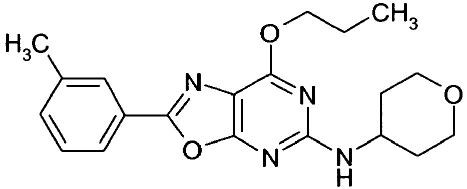

Eine Mischung von 100 mg 5-Methansulfonyl-7-propoxy-2-(3-methylphenyl)oxazolo[5,4-d]pyrimidin, 40 mg 4-Aminotetrahydropyran-hydrochlorid und 39 µl Triethylamin in 3 ml DMF wurde 8 h auf 50°C erhitzt. Nach Filtration wurde das Lösungsmittel im Vakuum abdestilliert und das Produkt mittels präparativer HPLC isoliert, was 22 mg der Titelverbindung ergab.
LC/MS (Methode LC4): Rt = 2,75 min; m/z = 369,27 [M+H]⁺

### Beispiel 4

### 7-Propoxy-5-(thiazol-2-ylsulfanyl)-2-(3-methylphenyl)oxazolo[5,4-d]pyrimidin

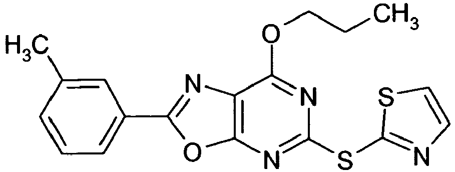

Eine Lösung von 34 mg 2-Mercaptothiazol in 3 ml DMF wurde mit 113 mg Cäsiumcarbonat und 100 mg 5-Methansulfonyl-7-propoxy-2-(3-methylphenyl)oxazolo[5,4-d]pyrimidin versetzt. Die Mischung wurde im Mikrowellenreaktor 15 min auf 100°C erhitzt. Nach Abkühlen und Filtration wurde das Lösungsmittel im Vakuum abdestilliert und das Produkt mittels präparativer HPLC isoliert, was 14 mg der Titelverbindung ergab.
LC/MS (Methode LC3): Rt = 2,44 min; m/z = 385,09 [M+H]⁺

### Beispiel 5

### 5-(2-Fluorphenoxy)-2-(4-methoxy-3,5-dimethylphenyl)-7-propoxyoxazolo[5,4-d]pyrimidin

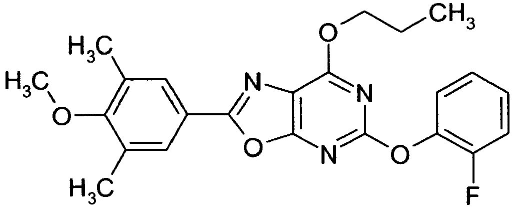

Die Titelverbindung wurde nach der in Beispiel 1 beschriebenen Verfahrensweise unter Verwendung von 4-Methoxy-3,5-dimethylbenzoylchlorid in Schritt (a) und 2-Fluorphenol in Schritt (g) hergestellt.
LC/MS (Methode LC5): Rt = 4,72 min; m/z = 424,25 [M+H]⁺

### Beispiel 6

### 5-(2-Fluorphenoxy)-2-(4-hydroxy-3,5-dimethylphenyl)-7-propoxyoxazolo[5,4-d]pyrimidin

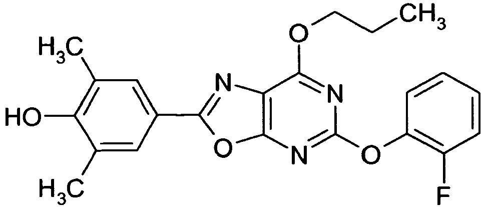

Eine eiskalte Lösung von 145 mg 5-(2-Fluorphenoxy)-2-(4-methoxy-3,5-dimethylphenyl)-7-propoxyoxazolo[5,4-d]pyrimidin in 8 ml Dichlormethan wurde langsam mit 1,0 ml einer 1 M Lösung von Bortribromid in Dichlormethan versetzt. Die Mischung wurde unter Eiskühlung 2 h und dann noch 1 h bei Raumtemperatur gerührt. Dann wurde vorsichtig eine gesättigte Lösung von Natriumhydrogencarbonat zugegeben. Durch Extraktion mit Dichlormethan, Trocknen über Natriumsulfat, Filtration und Entfernung des Lösungsmittels im Vakuum wurden 126 mg der Titelverbindung erhalten.
LC/MS (Methode LC2): Rt = 3,91 min; m/z = 410,02 [M+H]⁺

### Beispiel 7

### 7-Ethoxy-5-(2-fluorphenoxy)-2-(1-oxypyridin-3-yl)oxazolo[5,4-d]pyrimidin

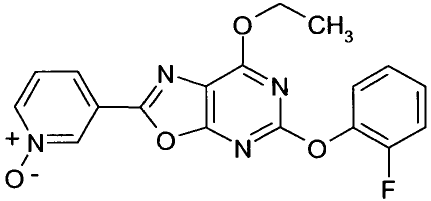

(a) 7-Ethoxy-5-methylsulfanyt-2-pyridin-3-yl-oxazolo[5,4-d]pyrimidin
   7-Ethoxy-5-methylsulfanyl-2-pyridin-3-yloxazolo[5,4-d]pyrimidin wurde nach der in Beispiel 1 beschriebenen Verfahrensweise unter Verwendung von Nicotinoylchlorid in Schritt (a) und Bromethan in Schritt (e) hergestellt.
(b) 7-Ethoxy-5-methansulfonyl-2-(1-oxypyridin-3-yl)oxazolo[5,4-d]pyrimidin
   Eine Lösung von 20 mg 7-Ethoxy-5-methylsulfanyl-2-pyridin-3-yloxazolo[5,4-d]pyrimidin in 1 ml Essigsäure wurde mit 21 µl 30%iger wäßriger Lösung von Wasserstoffperoxid versetzt. Die Lösung wurde 1 h auf 80°C erhitzt. Nach Zugabe von weiteren 21 µl 30%iger wäßriger Lösung von Wasserstoffperoxid und weiteren 2 h Erhitzen zum Rückfluß war die Reaktion abgeschlossen. Nach Abkühlen wurden 5 ml Toluol zugegeben und die Lösungsmittel im Vakuum abgezogen. Das Rohprodukt wurde ohne weitere Reinigung in Schritt (c) verwendet.
(c) 7-Ethoxy-5-(2-fluorphenoxy)-2-(1-oxypyridin-3-yl)oxazolo[5,4-d]pyrimidin
Eine Lösung von 9 mg 2-Fluorphenol in 2 ml DMF wurde mit 21 mg Kaliumcarbonat und 23 mg 7-Ethoxy-5-methansulfonyl-2-(1-oxypyridin-3-yl)oxazolo[5,4-d]pyrimidin versetzt. Die Mischung wurde 18 h bei Raumtemperatur gerührt. Nach Filtration wurde das Lösungsmittel im Vakuum abdestilliert und das Produkt mittels präparativer HPLC isoliert, was 6 mg der Titelverbindung ergab.
LC/MS (Methode LC10): Rt = 2,07 min; m/z = 369,09 [M+H]⁺

### Beispiel 8

### 5-(2-Fluorphenoxy)-2-(4-methoxy-3,5-dimethylphenyl)-7-(oxetan-3-yloxy)oxazolo[5,4-d]pyrimidin

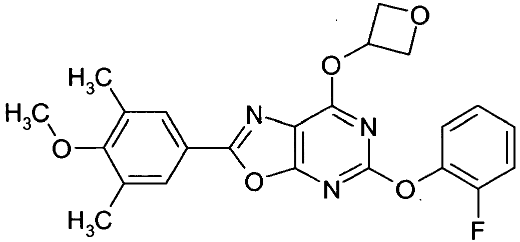

(a) 2-(4-Methoxy-3,5-dimethylphenyl)-5-methylsulfanyloxazolo[5,4-d]pyrimidin-7-ol
   2-(4-Methoxy-3,5-dimethylphenyl)-5-methylsulfanyloxazolo[5,4-d]pyrimidin-7-ol wurde nach der in Beispiel 1, Schritte (a) bis (d), beschriebenen Verfahrensweise unter Verwendung von 4-Methoxy-3,5-dimethylbenzoylchlorid in Schritt (a) hergestellt.
(b) 2-(4-Methoxy-3,5-dimethylphenyl)-5-methylsulfanyl-7-(oxetan-3-yloxy)oxazolo[5,4-d]pyrimidin
   Eine Lösung von 793 mg Triphenylphosphin und 527 mg Diethylazodicarboxylat in 50 ml Tetrahydrofuran wurde 15 min bei 0°C gerührt. Dann wurden 800 mg 2-(4-Methoxy-3,5-dimethylphenyl)-5-methylsulfanyloxazolo[5,4-d]pyrimidin-7-ol, 224 mg Oxetan-3-ol und 770 µl Triethylamin zugegeben. Die Mischung wurde 1,5 h bei Raumtemperatur gerührt. Dann wurden weitere 120 mg Oxetan-3-ol, 793 mg Triphenylphosphin und 527 mg Diethylazodicarboxylat zugegeben, wonach die Mischung 18 h gerührt wurde. Der Niederschlag wurde abfiltriert und mit Tetrahydrofuran gewaschen. Nach Abziehen des Lösungsmittels im Vakuum wurde die Titelverbindung durch Kieselgelchromatographie (Heptan/Essigsäureethylester-Gradient) in Form eines weißen Feststoffs isoliert. Ausbeute: 580 mg.
(c) 5-Methansulfonyl-2-(4-methoxy-3,5-dimethylphenyl)-7-(oxetan-3-yloxy)oxazolo[5,4-d]pyrimidin
   Eine Lösung von 580 mg 2-(4-Methoxy-3,5-dimethylphenyl)-5-methylsulfanyl-7-(oxetan-3-yloxy)oxazolo[5,4-d]pyrimidin in 30 ml Dichlormethan wurde mit 1,1 g 3-Chlorperbenzoesäure versetzt, wonach die Mischung 1,5 h bei Raumtemperatur gerührt wurde. Dann wurde die Lösung zweimal mit 30 ml 1 N Natronlauge extrahiert. Die vereinigten wäßrigen Schichten wurden zweimal mit 30 ml Dichlormethan extrahiert. Dann wurden die vereinigten organischen Schichten über Natriumsulfat getrocknet, filtriert und im Vakuum vom Lösungsmittel entfernt, was 547 mg der Titelverbindung in Form eines blaßgelben Öls ergab.
(d) 5-(2-Fluorphenoxy)-2-(4-methoxy-3,5-dimethylphenyl)-7-(oxetan-3-yloxy)oxazolo[5,4-d]pyrimidin
   Eine Lösung von 49 mg 2-Fluorphenol in 8 ml DMF wurde mit 410 mg Kaliumcarbonat und 547 mg 5-Methansulfonyl-2-(4-methoxy-3,5-dimethylphenyl)-7-(oxetan-3-yloxy)oxazolo[5,4-d]pyrimidin versetzt. Die Mischung wurde 2 h bei Raumtemperatur gerührt. Nach Filtration wurde das Lösungsmittel im Vakuum abdestilliert und das Produkt durch Kieselgelchromatographie (Heptan/Essigsäureethylester-Gradient) isoliert, was 275 mg der Titelverbindung ergab.
   LC/MS (Methode LC2): Rt = 3,87 min; m/z = 438,03 [M+H]⁺

### Beispiel 9

### 5-Pent-4-enyloxy-7-propoxy-2-(3-methylphenyl)oxazolo[5,4-d]pyrimidin

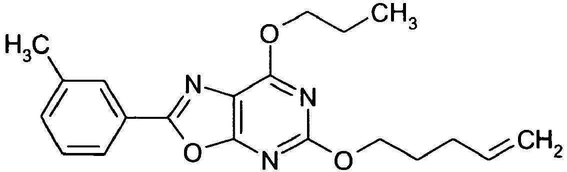

Eine Suspension von 500 mg Tetrahydrofurfurylbromid und 73 mg Magnesiumspänen in 10 ml THF wurde mit 50 mg 1,2-Dibromethan versetzt. Die Mischung wurde 5 h gerührt und dann in mehrere Portionen aufgeteilt. 1,5 ml der THF-Lösung wurden zu einer Lösung von 130 mg 5-Methansulfonyl-7-propoxy-2-(3-methylphenyl)oxazolo[5,4-d]pyrimidin und 16 mg Lithiumchlorid in 7 ml THF gegeben. Die Mischung wurde 16 h bei Raumtemperatur gerührt. Nach Abziehen des Lösungsmittels im Vakuum wurde das Produkt durch Kieselgelchromatographie (Heptan/Essigsäureethylester-Gradient) isoliert, was 78 mg der Titelverbindung ergab.
LC/MS (Methode LC1): Rt = 5,50 min; m/z = 354,25 [M+H]⁺

In Analogie zur Herstellung der oben beschriebenen Beispielverbindungen wurden die in Tabelle 1 aufgeführten Beispielverbindungen der Formel I hergestellt. Sie wurden zum Teil in Form ihres Trifluoressigsäuresalzes erhalten.

**Tabelle 1. Beispielverbindungen der Formel I**

| Beispiel | Name | LC/MS | m/z [M+H]⁺ | Rt [min] |
|---|---|---|---|---|
| 10 | 2-(2-Chlor-5-fluorphenyl)-5-(2,5-dimethyl-2H-pyrazol-3-yloxy)-7-ethoxyoxazolo[5,4-d]pyrimidin | LC1 | 445,36 (1) | 4,93 |
| 11 | 2-(2-Chlor-5-fluorphenyl)-7-cyclopropylmethoxy-5-(2,5-dimethyl-2H-pyrazol-3-yloxy)oxazolo[5,4-d]pyrimidin | LC1 | 430,35 | 5,04 |
| 12 | 5-(3,4-Difluorphenylsulfanyl)-7-ethoxy-2-(3-methylphenyl)oxazolo[5,4-d]pyrimidin | LC1 | 400,33 | 5,42 |
| 13 | 2-(2-Chlor-5-fluorphenyl)-7-cyclopropylmethoxy-5-(1,1-dioxotetrahydro-1lambda6-thiophen-3-ylsulfanyl)oxazolo[5,4-d]pyrimidin | LC1 | 470,12 | 5,01 |
| 14 | 5-(1,1-Dioxotetrahydro-1lambda6-thiophen-3-ylsulfanyl)-2-(3-fluorphenyl)-7-propoxyoxazolo[5,4-d]pyrimidin | LC1 | 424,17 | 4,99 |
| 15 | 2-(3,4-Dichlorphenyl)-5-(2-fluorphenoxy)-7-propoxyoxazolo[5,4-d]pyrimidin | LC1 | 434,11 | 5,53 |
| 16 | 7-Cyclopropylmethoxy-5-pent-4-enyloxy-2-(3-methylphenyl)oxazolo[5,4-d]pyrimidin | LC1 | 366,3 | 5,44 |
| 17 | 3-(7-Ethoxy-2-(3-methylphenyl)oxazolo[5,4-d]pyrimidin-5-yloxy)-N,N-dimethylbenzolsulfonamid | LC2 | 455,04 | 3,8 |
| 18 | 2-(3-Fluorphenyl)-5-(6-methylpyridazin-3-yloxy)-7-propoxyoxazolo[5,4-d]pyrimidin | LC2 | 382,1 | 2,97 |
| 19 | 5-(6-Methylpyridazin-3-yloxy)-7-propoxy-2-(3-methylphenyl)oxazolo[5,4-d]pyrimidin | LC2 | 378,11 | 3,09 |
| 20 | 3-(7-Propoxy-2-(3-methylphenyl)oxazolo[5,4-d]pyrimidin-5-yloxy)benzolsulfonamid | LC2 | 441,08 | 3,55 |
| 21 | 3-[2-(2-Chlor-5-fluorphenyl)-7-ethoxyoxazolo[5,4-d]pyrimidin-5-yloxy]benzolsulfonamid | LC2 | 465,04 | 3,34 |
| 22 | 3-(7-Ethoxy-2-(3-methylphenyl)oxazolo[5,4-d]pyrimidin-5-yloxy)benzolsulfonamid | LC2 | 427,06 | 3,4 |
| 23 | 3-[2-(3-Fluorphenyl)-7-propoxyoxazolo[5,4-d]pyrimidin-5-yloxy]benzolsulfonamid | LC2 | 445,1 | 3,42 |
| 24 | 5-(2,5-Dimethyl-2H-pyrazol-3-yloxy)-2-(3-fluorphenyl)-7-propoxyoxazolo[5,4-d]pyrimidin | LC2 | 384,19 | 3,62 |
| 25 | 5-(2,5-Dimethyl-2H-pyrazol-3-yloxy)-7-propoxy-2-(3-methylphenyl)oxazolo[5,4₋d]pyrimidin | LC2 | 380,13 | 3,74 |
| 26 | 5-(2,5-Dimethyl-2H-pyrazol-3-yloxy)-7-ethoxy-2-(3-methylphenyl)oxazolo[5,4-d]pyrimidin | LC2 | 366,09 | 3,69 |
| 27 | 5-(1,1-Dioxotetrahydro-1 lambda6-thiophen-3-ylsulfanyl)-7-propoxy-2-(3-methylphenyl)oxazolo[5,4-d]pyrimidin | LC2 | 420,17 | 3,78 |
| 28 | 7-Cyclopropylmethoxy-5-(1,1-dioxotetrahydro-1lambda6-thiophen-3-ylsulfanyl)-2-(3-methylphenyl)oxazolo[5,4-d]pyrimidin | LC2 | 432,16 | 3,75 |
| 29 | 7-Cyclopropylmethoxy-5-(2-fluorphenoxy)-2-(3-methylphenyl)oxazolo[5,4-d]pyrimidin | LC2 | 392,05 | 4,17 |
| 30 | 2-(2-Chlor-5-fluorphenyl)-7-ethoxy-5-(2-fluorphenoxy)oxazolo[5,4-d]pyrimidin | LC2 | 403,99 | 3,98 |
| 31 | 5-(2-Fluorphenoxy)-2-(3-fluorphenyl)-7-propoxyoxazolo[5,4-d]pyrimidin | LC2 | 384,04 | 4,08 |
| 32 | 7-Cyclopropylmethoxy-5-(2-fluorphenoxy)-2-(3-fluorphenyl)oxazolo[5,4-d]pyrimidin | LC2 | 396,05 | 4,05 |
| 33 | 2-(2-Fluor-5-methylphenyl)-5-phenoxy-7-propoxyoxazolo[5,4-d]pyrimidin | LC2 | 380,11 | 4,17 |
| 34 | 2-(2-Chlor-5-fluorphenyl)-7-cyclopropylmethoxy-5-(2,2-dimethyl[1,3]dioxolan-4-ylmethoxy)oxazolo[5,4-d]pyrimidin | LC2 | 450,11 | 3,96 |
| 35 | 7-Cyclopropylmethoxy-5-(2,2-dimethyl[1,3]dioxolan-4-ylmethoxy)-2-(3-fluorphenyl)oxazolo[5,4-d]pyrimidin | LC2 | 416,18 | 3,86 |
| 36 | 7-cyclopropylmethoxy-5-(2,2-dimethyl[1,3]dioxolan-4-ylmethoxy)-2-(3-methyl-phenyl)-oxazolo[5,4-d]pyrimidin | LC2 | 412,2 | 4 |
| 37 | 5-(2,2-Dimethyl[1,3]dioxolan-4-ylmethoxy)-7-ethoxy-2-(3-methylphenyl)oxazolo[5,4-d]pyrimidin | LC2 | 386,17 | 3,86 |
| 38 | 5-(Oxetan-3-yloxy)-7-propoxy-2-(3-methylphenyl)-oxazolo[5,4-d]pyrimidin | LC2 | 342,1 | 3,74 |
| 39 | 7-Cyclopropylmethoxy-5-(oxetan-3-yloxy)-2-(3-methylphenyl)oxazolo[5,4-d]pyrimidin | LC2 | 354,12 | 3,72 |
| 40 | 5-(2-Fluorphenoxy)-2-(3-methoxyphenyl)-7-propoxyoxazolo[5,4-d]pyrimidin | LC2 | 396,12 | 4,08 |
| 41 | 2-(3-Methoxyphenyl)-7-propoxy-5-(pyridin-3-yloxy)oxazolo[5,4-d)pyrimidin | LC2 | 379,12 | 3,14 |
| 42 | 3-[5-(2-Fluorphenoxy)-7-propoxyoxazolo[5,4-d]pyrimidin-2-yl]phenol | LC2 | 382,07 | 3,64 |
| 43 | 3-[7-Propoxy-5-(pyridin-3-yloxy)oxazolo[5,4-d]pyrimidin-2-yl]phenol | LC2 | 365,11 | 2,74 |
| 44 | 2-(3,4-Dichlorphenyl)-7-propoxy-5-(pyridin-3-yloxy)oxazolo[5,4-d]pyrimidin | LC2 | 417,05 | 3,7 |
| 45 | 2-(3,4-Dichlorphenyl)-5-(2,2-dimethyl[1,3]dioxolan-4-ylmethoxy)-7-propoxyoxazolo[5,4-d]pyrimidin | LC2 | 454,09 | 4,36 |
| 46 | 7-Ethoxy-5-(2-fluorphenoxy)-2-(4-methoxy-3,5-dimethylphenyl)oxazolo[5,4-d]pyrimidin | LC2 | 410,07 | 4,15 |
| 47 | 4-[7-Ethoxy-5-(2-fluorphenoxy)oxazolo[5,4-d]pyrimidin-2-yl]-2,6-dimethylphenol | LC2 | 396,05 | 3,73 |
| 48 | 5-((1S,2S)-2-Fluorcyclohexyloxy)-2-(3-fluorphenyl)-7-propoxyoxazolo[5,4-d]pyrimidin | LC2 | 390,09 | 4,15 |
| 49 | 2-[3,5-Dimethyl-4-(oxetan-3-yloxy)phenyl]-5-(2-fluorphenoxy)-7-(oxetan-3-yloxy)oxazolo[5,4-d]pyrimid in | LC2 | 480,08 | 3,63 |
| 50 | 5-(5-Fluor-2-methylphenoxy)-7-propoxy-2-(3-methylphenyl)oxazolo[5,4-d]pyrimidin | LC3 | 394,23 | 2,58 |
| 51 | 5-Phenoxy-7-propoxy-2-(3-methylphenyl)oxazolo[5,4-d]pyrimidin | LC3 | 362,2 | 2,47 |
| 52 | 5-(3-Chlor-4-trifluormethoxyphenoxy)-7-propoxy-2-(3-methylphenyl)oxazolo[5,4-d]pyrimidin | LC3 | 480,14 | 2,76 |
| 53 | 5-(2,4-Dimethylphenoxy)-7-propoxy-2-(3-methylphenyl)oxazolo[5,4-d]pyrimidin | LC3 | 390,22 | 2,67 |
| 54 | 5-(3-Fluor-4-methylphenoxy)-7-propoxy-2-(3-methylphenyl)oxazolo[5,4-d]pyrimidin | LC3 | 394,12 | 2,57 |
| 55 | 5-(4-Methylthiazol-2-ylsulfanyl)-7-propoxy-2-(3-methylphenyl)oxazolo[5,4-d]pyrimidin | LC3 | 399,09 | 2,52 |
| 56 | 2-Methyl-6-(7-propoxy-2-(3-methylphenyl)oxazolo[5,4-d]pyrimidin-5-yloxy)-2H-pyridazin-3-on | LC3 | 435,16 (1) | 1,91 |
| 57 | [2-(2-Chlor-5-fluorphenyl)-7-propoxyoxazolo[5,4-d]pyrimidin-5-yl](tetrahydropyran-4-yl)amin | LC3 | 407,17 | 2,14 |
| 58 | 2-(2-Chlor-5-fluorphenyl)-5-(4-methylthiazol-2-ylsulfanyl)-7-propoxyoxazolo[5,4-d]pyrimidin | LC3 | 436,98 | 2,44 |
| 59 | 2-(2-Chlor-5-fluorphenyl)-7-propoxy-5-(thiazol-2-ylsulfanyl)oxazolo[5,4-d]pyrimidin | LC3 | 423,07 | 2,35 |
| 60 | 5-(4-Chlor-2-methoxyphenoxy)-2-(3-fluorphenyl)-7-propoxyoxazolo[5,4-d]pyrimidin | LC3 | 430,13 | 2,44 |
| 61 | 2-(3-Fluorphenyl)-5-phenoxy-7-propoxyoxazolo[5,4-d]pyrimidin | LC3 | 366,17 | 2,36 |
| 62 | 5-Cyclohexyloxy-2-(3-fluorphenyl)-7-propoxyoxazolo[5,4-d]pyrimidin | LC3 | 372,15 | 2,65 |
| 63 | 2-(2-Chlor-5-fluorphenyl)-5-(4-chlor-2-methoxyphenoxy)-7-propoxyoxazolo[5,4-d]pyrimidin | LC3 | 464,21 | 2,54 |
| 64 | [2-(2-Chlor-5-fluorphenyl)-7-propoxyoxazolo[5,4-d]pyrimidin-5-yl](4,4-difluorcyclohexyl)amin | LC3 | 441,28 | 2,45 |
| 65 | Morphol in-4-yl-(7-propoxy-2-(3-methylphenyl)oxazolo[5,4-d]pyrimidin-5-yl)amin | LC3 | 370,18 | 1,83 |
| 66 | 2-(2-Chlor-5-fluorphenyl)-5-(5-chlor-2-methylphenoxy)-7-propoxyoxazolo[5,4-d]pyrimidin | LC3 | 448,06 | 2,67 |
| 67 | 5-(5-Chlor-2-methylphenoxy)-2-(3-fluorphenyl)-7-propoxyoxazolo[5,4-d]pyrimidin | LC3 | 414,1 | 2,64 |
| 68 | 2-(2-Chlor-5-fluorphenyl)-7-propoxy-5-(3-trifluormethylsulfanylphenoxy)oxazolo[5,4-d]-pyrimidin | LC3 | 500,07 | 2,71 |
| 69 | 2-(3-Fluorphenyl)-7-propoxy-5-(3-trifluormethylsulfanylphenoxy)oxazolo[5,4-d]-pyrimidin | LC3 | 466,09 | 2,68 |
| 70 | 5-(4-Methylcyclohexyloxy)-7-propoxy-2-(3-methylphenyl)oxazolo[5,4-d]pyrimidin | LC3 | 382,18 | 2,88 |
| 71 | 7-Propoxy-5-(tetrahydropyran-4-yloxy)-2-(3-methylphenyl)oxazolo[5,4-d]pyrimidin | LC3 | 370,18 | 2,22 |
| 72 | 2-(2-Chlor-5-fluorphenyl)-5-(5-fluorpyridin-3-yloxy)-7-propoxyoxazolo[5,4-d]pyrimidin | LC3 | 460,11 (1) | 2,17 |
| 73 | 7-Propoxy-2-(3-methylphenyl)-5-p-tolyloxyoxazolo[5,4-d]pyrimidin | LC4 | 376,27 | 3,32 |
| 74 | 5-(4-Chlor-3-fluorphenoxy)-7-propoxy-2-(3-methylphenyl )oxazolo[5,4-d]pyrimidin | LC4 | 414,23 | 3,42 |
| 75 | 5-(2-Fluor-4-methoxyphenoxy)-7-propoxy-2-(3-methylphenyl)oxazolo[5,4-d]pyrimidin | LC4 | 410,28 | 3,17 |
| 76 | 7-Propoxy-5-(pyridin-2-yloxy)-2-(3-methylphenyl)oxazolo[5,4-d]pyrimidin | LC4 | 363,23 | 2,68 |
| 77 | 7-Propoxy-5-(pyridin-3-yloxy)-2-(3-methylphenyl)oxazolo[5,4-d]pyrimidin | LC4 | 363,24 | 2,23 |
| 78 | 2-(2-Chlor-5-fluorphenyl)-5-phenoxy-7-propoxyoxazolo[5,4-d]pyrimidin | LC4 | 400,07 | 3,1 |
| 79 | 2-(2-Chlor-5-fluorphenyl)-5-(5-fluor-2-methyl-phenoxy)-7-propoxyoxazolo[5,4-d]pyrimidin | LC4 | 432,12 | 3,22 |
| 80 | 2-(2-Chlor-5-fluorphenyl)-5-cyclohexyloxy-7-propoxyoxazolo[5,4-d]pyrimidin | LC4 | 406,15 | 3,47 |
| 81 | 5-(3-Fluor-4-methylphenoxy)-2-(3-fluorphenyl)-7-propoxyoxazolo[5,4-d]pyrimidin | LC5 | 398,14 | 4,69 |
| 82 | 2-(3-Fluorphenyl)-7-propoxy-5-(4-trifluormethylsulfanylphenoxy)oxazolo[5,4-d]-pyrimidin | LC5 | 466,17 | 2,72 |
| 83 | 5-(2,2-Dimethyl-2,3-dihydrobenzofuran-7-yloxy)-7-propoxy-2-(3-methylphenyl)oxazolo[5,4-d]pyrimidin | LC5 | 432,26 | 4,84 |
| 84 | 2-(2-Chlor-5-fluorphenyl)-5-(2,2-dimethyl-2,3-dihydrobenzofuran-7-yloxy)-7-propoxyoxazolo[5,4-d]pyrimidin | LC5 | 470,21 | 4,8 |
| 85 | 2-(2-Chlor-5-fluorphenyl)-5-(4-methylcyclohexyloxy)-7-propoxyoxazolo[5,4-d]pyrimidin | LC5 | 420,24 | 5,09 |
| 86 | 5-(2,6-Dichlorphenoxy)-7-propoxy-2-(3-methylphenyl)oxazolo(5,4-d]pyrimidin | LC5 | 430,11 | 4,89 |
| 87 | 3-(7-Propoxy-2-(3-methylphenyl)oxazolo[5,4-d]pyrimidin-5-yloxy)benzonitril | LC5 | 387,16 | 4,45 |
| 88 | 5-(4-Chlor-2-methylphenoxy)-7-propoxy-2-(3-methylphenyl)oxazolo[5,4-d]pyrimidin | LC5 | 410,18 | 4,95 |
| 89 | 5-(3-Chlor-4-methyiphenoxy)-7-propoxy-2-(3-methylphenyl)oxazolo[5,4-d]pyrimidin | LC5 | 410,18 | 4,97 |
| 90 | 5-(2-Chlor-4-methylphenoxy)-7-propoxy-2-(3-methylphenyl)oxazolo[5,4-d]pyrimidin | LC5 | 410,18 | 4,82 |
| 91 | 5-(2-Chlor-6-methylphenoxy)-7-propoxy-2-(3-methylphenyl)oxazolo[5,4-d]pyrimidin | LC5 | 410,17 | 4,84 |
| 92 | 3-Methoxy-4-(7-propoxy-2-(3-methylphenyl)oxazolo[5,4-d]pyrimidin-5-yloxy)benzonitril | LC5 | 417,17 | 4,4 |
| 93 | 5-(3-Ethoxyphenoxy)-7-propoxy-2-(3-methylphenyl)oxazolo[5,4-d]pyrimidin | LC5 | 406,17 | 4,82 |
| 94 | 5-(2,5-Difluorphenoxy)-7-propoxy-2-(3-methylphenyl)oxazolo[5,4-d]pyrimidin | LC5 | 398,11 | 4,6 |
| 95 | 5-(2,4-Difluorphenoxy)-7-propoxy-2-(3-methylphenyl)oxazolo[5,4-d]pyrimidin | LC5 | 398,12 | 4,59 |
| 96 | 5-(2-tert.-Butylphenoxy)-7-propoxy-2-(3-methylphenyl)oxazolo[5,4-d]pyrimidin | LC5 | 418,22 | 5,14 |
| 97 | 5-(2,6-Difluorphenoxy)-7-propoxy-2-(3-methylphenyl)oxazolo[5,4-d]pyrimidin | LC5 | 398,15 | 4,65 |
| 98 | 7-Propoxy-2-(3-methylphenyl)-5-(3-methylphenyl)oxyoxazolo[5,4-d]pyrimidin | LC5 | 376,16 | 4,75 |
| 99 | 5-(4-Fluor-2-methoxyphenoxy)-7-propoxy-2-(3-methylphenyl)oxazolo(5,4-d]pyrimidin | LC5 | 410,14 | 4,6 |
| 100 | 5-(2-Chlor-3,5-difluorphenoxy)-7-propoxy-2-(3-methylphenyl)oxazolo[5,4-d]pyrimidin | LC5 | 432,13 | 4,85 |
| 101 | 5-(2-Chlor-5-fluorphenoxy)-7-propoxy-2-(3-methylphenyl)oxazolo[5,4-d]pyrimidin | LC5 | 414,15 | 4,79 |
| 102 | 7-Cyclopropylmethoxy-5-(2,5-dimethyl-2H-pyrazol-3-yloxy)-2-(3-fluorphenyl)oxazolo[5,4-d]pyrimidin | LC5 | 396,07 | 3,97 |
| 103 | 5-(2-Fluorphenoxy)-7-propoxy-2-(3-methylphenyl)oxazolo[5,4-d]pyrimidin | LC5 | 380,11 | 4,57 |
| 104 | 2-(2-Chlor-5-fluorphenyl)-7-cyclopropylmethoxy-5-(2-fluorphenoxy)oxazolo[5,4-d]pyrimidin | LC5 | 430,04 | 4,45 |
| 105 | 2-(3,4-Dichlorphenyl)-5-phenoxy-7-propoxyoxazolo[5,4-d]pyrimidin | LC5 | 416,11 | 4,94 |
| 106 | 7-Cyclopropylmethoxy-2-(3,4-dichlorphenyl)-5-(pyridin-3-yloxy)oxazolo[5,4-d]pyrimidin | LC5 | 429,1 | 3,97 |
| 107 | 5-(2-Fluorphenoxy)-7-propoxy-2-pyridin-3-yloxazolo[5,4-d]pyrimidin | LC5 | 367,12 | 3,7 |
| 108 | 7-Cyclopropylmethoxy-2-(3,4-dichlorphenyl)-5-(5-fluorpyridin-3-yloxy)oxazolo[5,4-d]pyrimidin | LC5 | 447,06 | 4,6 |
| 109 | 5-(2-Fluorphenoxy)-2-(1-oxypyridin-3-yl)-7-propoxyoxazolo[5,4-d]pyrimidin | LC5 | 383,15 | 3,2 |
| 110 | 7-Cyclopropylmethoxy-2-(3,4-dichlorphenyl)-5-phenoxyoxazolo[5,4-d]pyrimidin | LC5 | 428,13 | 4,85 |
| 111 | 5-(2-Fluor-4-methylphenoxy)-7-propoxy-2-(3-methylphenyl)oxazolo[5,4-d]pyrimidin | LC6 | 394,21 | 3,01 |
| 112 | 7-Propoxy-2-(3-methylphenyl)-5-(4-trifluormethylsulfanylphenoxy)oxazolo[5,4-d]-pyrimidin | LC6 | 462,19 | 3,18 |
| 113 | [2-(2-Chlor-5-fluorphenyl)-7-propoxyoxazolo[5,4-d]pyrimidin-5-yl]-(4-methylcyclohexyl)amin | LC6 | 419,36 | 3,24 |
| 114 | 6-[2-(2-Chlor-5-fluorphenyl)-7-propoxyoxazolo[5,4-d]pyrimidin-5-yloxy]-2-methyl-2H-pyridazin-3-on | LC7 | 432,1 (2) | 2,29 |
| 115 | 5-(5-Chlor-2-methylphenoxy)-7-propoxy-2-(3-methylphenyl)oxazolo[5,4-d]pyrimidin | LC8 | 410,08 | 3,64 |
| 116 | 7-Propoxy-2-(3-methylphenyl)-5-(3-trifluormethylsulfanylphenoxy)oxazolo[5,4-d]-pyrimidin | LC8 | 462,04 | 3,71 |
| 117 | 2-(2-Chlor-5-fluorphenyl)-7-propoxy-5-(tetrahydropyran-4-yloxy)oxazolo[5,4-d]pyrimidin | LC8 | 408,14 | 2,96 |
| 118 | 5-(3-Methoxyphenoxy)-7-propoxy-2-(3-methylphenyl)oxazolo[5,4-d]pyrimidin | LC8 | 392,12 | 3,33 |
| 119 | 5-(2,4-Dichlorphenoxy)-7-propoxy-2-(3-methylphenyl)oxazolo[5,4-d]pyrimidin | LC8 | 430,03 | 3,67 |
| 120 | 5-(2,5-Dichlorphenoxy)-7-propoxy-2-(3-methylphenyl)oxazolo[5,4-d]pyrimidin | LC8 | 430,03 | 3,65 |
| 121 | 5-(3-Chlorphenoxy)-7-propoxy-2-(3-rnethylphenyl)oxazolo[5,4-d]pyrimidin | LC8 | 396,08 | 3,55 |
| 122 | 5-(4-Ethylphenoxy)-7-propoxy-2-(3-methylphenyl)oxazolo[5,4-d]pyrimidin | LC8 | 390,13 | 3,62 |
| 123 | 7-Propoxy-2-(3-methylphenyl)-5-(2,3,5-trimethylphenoxy)oxazolo[5,4-d]pyrimidin | LC8 | 404,15 | 3,7 |
| 124 | 5-(2-Isopropylphenoxy)-7-propoxy-2-(3-methylphenyl)oxazolo[5,4-d]pyrimidin | LC8 | 404,15 | 3,68 |
| 125 | 5-(3-ter.-Butylphenoxy)-7-propoxy-2-(3-methylphenyl)oxazolo[5,4-d]pyrimidin | LC8 | 418,16 | 3,79 |
| 126 | 7-Propoxy-5-(2-propylphenoxy)-2-(3-methylphenyl)oxazolo[5,4-d]pyrimidin | LC8 | 404,15 | 3,7 |
| 127 | 5-(3,5-Difluorphenoxy)-7-propoxy-2-(3-methylphenyl)oxazolo[5,4-d]pyrimidin | LC8 | 398,09 | 3,46 |
| 128 | 2-(7-Propoxy-2-(3-methylphenyl)oxazolo[5,4-d]pyrimidin-5-yloxy)benzonitril | LC8 | 387,15 | 3,12 |
| 129 | 5-(4-Fluor-3-trifluormethylphenoxy)-7-propoxy-2-(3-methylphenyl)oxazolo[5,4-d]pyrimidin | LC8 | 448,12 | 3,52 |
| 130 | 5-(4-Fluor-3-methylphenoxy)-7-propoxy-2-(3-methylphenyl)oxazolo[5,4-d]pyrimidin | LC8 | 394,15 | 3,47 |
| 131 | Dimethyl(3-(7-propoxy-2-(3-methylphenyl)oxazolo[5,4-d]pyrimidin-5-yloxy)phenyl]amin | LC8 | 405,19 | 2,87 |
| 132 | 5-(2,6-Dimethylphenoxy)-7-propoxy-2-(3-methylphenyl)oxazolo[5,4-d]pyrimidin | LC8 | 390,18 | 3,54 |
| 133 | 5-(2,5-Dimethylphenoxy)-7-propoxy-2-(3-methylphenyl)oxazolo[5,4-d]pyrimidin | LC8 | 390,18 | 3,54 |
| 134 | 2-Fluor-4-(7-propoxy-2-(3-methylphenyl)oxazolo[5,4-d]pyrimidin-5-yloxy)benzonitril | LC8 | 405,14 | 3,24 |
| 135 | 5-(2-Methoxy-4-methylphenoxy)-7-propoxy-2-(3-methylphenyl)oxazolo[5,4-d]pyrimidin | LC8 | 406,18 | 3,36 |
| 136 | 5-(3,4-Dimethoxyphenoxy)-7-propoxy-2-(3-methylphenyl)oxazolo[5,4-d]pyrimidin | LC8 | 422,18 | 3,12 |
| 137 | 5-(2-Chlor-5-methylphenoxy)-7-propoxy-2-(3-methylphenyl)oxazolo[5,4-d]pyrimidin | LC8 | 410,09 | 3,57 |
| 138 | 5-(3-Chlor-5-fluorphenoxy)-7-propoxy-2-(3-methylphenyl)oxazolo[5,4-d]pyrimidin | LC8 | 414,06 | 3,62 |
| 139 | 5-(2-Methylsulfanylphenoxy)-7-propoxy-2-(3-methylphenyl)oxazolo[5,4-d]pyrimidin | LC8 | 408,09 | 3,38 |
| 140 | 5-(4-Chlor-3-methylphenoxy)-7-propoxy-2-(3-methylphenyl)oxazolo[5,4-d]pyrimidin | LC8 | 410,08 | 3,67 |
| 141 | 7-Propoxy-2-(3-methylphenyl)-5-(2-trifluormethylphenoxy)oxazolo[5,4-d]pyrimidin | LC8 | 430,08 | 3,44 |
| 142 | 7-Propoxy-2-(3-methylphenyl)-5-(2,4,6-trimethylphenoxy)oxazolo[5,4-d]pyrimidin | LC8 | 404,15 | 3,7 |
| 143 | 5-(2-Chlor-5-trifluormethylphenoxy)-7-propoxy-2-(3-methylphenyl)oxazolo[5,4-d]pyrimidin | LC8 | 464,04 | 3,64 |
| 144 | 5-(3-Fluor-5-trifluormethylphenoxy)-7-propoxy-2-(3-methylphenyl)oxazolo[5,4-d]pyrimidin | LC8 | 448,07 | 3,61 |
| 145 | 5-(2-Chlor-4-trifluormethylphenoxy)-7-propoxy-2-(3-methylphenyl)oxazolo[5,4-d]pyrimidin | LC8 | 464,04 | 3,67 |
| 146 | 7-Propoxy-5-(2-propoxyphenoxy)-2-(3-methylphenyl)oxazolo[5,4-d]pyrimidin | LC8 | 420,18 | 3,48 |
| 147 | [2-(2-Chlor-5-fluorphenyl)-7-propoxyoxazolo[5,4-d]pyrimidin-5-yl]-((1S,3S)-3-methylcyclohexyl)amin | LC8 | 419,1 | 3,7 |
| 148 | [2-(2-Chlor-5-fluorphenyl)-7-propoxyoxazolo[5,4-d]pyrimidin-5-yl](4-methylcyclohexyl)amin | LC8 | 419,1 | 3,71 |
| 149 | [2-(2-Chlor-5-fluorphenyl)-7-propoxyoxazolo[5,4-d]pyrimidin-5-yl]cyclohexylamin | LC8 | 405,08 | 3,53 |
| 150 | 2-(2-Chlor-5-fluorphenyl)-5-phenylsulfanyl-7-propoxyoxazolo[5,4-d]pyrimidin | LC8 | 416,07 | 3,39 |
| 151 | 2-(2-Chlor-5-fluorphenyl)-5-(2,5-dimethylfuran-3-ylsulfanyl)-7-propoxyoxazolo[5,4-d]pyrimidin | LC8 | 434,06 | 3,47 |
| 152 | 2-(2-Chlor-5-fluorphenyl)-5-(3-methyloxetan-3-ylmethoxy)-7-propoxyoxazolo[5,4-d]pyrimidin | LC8 | 407,99 | 2,82 |
| 153 | 2-(2-Chlor-5-fluorphenyl)-5-(5-fluor-2-methylphenylsulfanyl)-7-propoxyoxazolo[5,4-d]pyrimidin | LC8 | 448,07 | 3,5 |
| 154 | 2-(2-Chlor-5-fluorphenyl)-7-propoxy-5-(4-trifluormethylcyclohexyloxy)oxazolo[5,4-d]-pyrimidin | LC8 | 474,12 | 3,4 |
| 155 | 2-(2-Chlor-5-fluorphenyl)-5-(5-methylpyridin-2-ylsulfanyl)-7-propoxyoxazolo[5,4-d]pyrimidin | LC8 | 431,05 | 2,97 |
| 156 | 2-(3,4-Dichlorphenyl)-5-phenylsulfanyl-7-propoxyoxazolo[5,4-d]pyrimidin | LC9 | 432,08 | 5,74 |
| 157 | 2-(3,4-Dichlorphenyl)-5-(5-fluor-2-methylphenoxy)-7-propoxyoxazolo[5,4-d]pyrimidin | LC9 | 448,11 | 5,49 |
| 158 | 7-Cyclopropylmethoxy-2-(3,4-dichlorphenyl)-5-(2-fluorphenoxy)oxazolo[5,4-d]pyrimidin | LC9 | 446,07 | 5,28 |
| 159 | 7-Cyclopropylmethoxy-2-(3,4-dichlorphenyl)-5-(5-fluor-2-methyl-phenoxy)oxazolo[5,4-d]pyrimidin | LC9 | 460,12 | 5,38 |
| 160 | 2-(3,4-Dichlorphenyl)-5-(5-fluorpyridin-3-yloxy)-7-propoxyoxazolo[5,4-d]pyrimidin | LC9 | 435,18 | 5,13 |

| | | | | |
|---|---|---|---|---|
| (1) [M⁺CH₃CN+H]⁺ (2) [M-H]⁺ Bestimmung der pharmakologischen Wirkung | | | | |

### A) GTP-γ-S-Assay mit humanen Edg-1-Rezeptoren

Zur Bestimmung der Edg-1-Rezeptor-Aktivierung durch die erfindungsgemäßen Verbindungen wurde ein GTP-γ-S-Assay (GTP-γ-S = Guanosin-5'-[thio]triphosphat) auf die Bindung an G-Protein gekoppeltem Rezeptor auf Basis des Szintillationsproximitätsassay-Prinzips verwendet, wobei ein Zellmembranpräparat einer CHO-Flp-In-Zelllinie, die den humanen Edg-1-Rezeptor konstitutiv überexprimiert, eingesetzt wurde.

### (a) Erzeugung der Zelllinie

Das Flp-In™-Expressionssystem (Invitrogen, Kat.-Nr. K6010-01) erlaubt die Erzeugung von stabilen Säugetierzelllinien, in die das interessierende Gen durch homologe Rekombination an einem spezifischen Genomort, der als FRT-Ort (FRT = Flp Recombination Target) bezeichnet wird, mit Hilfe einer durch das pOG44-Expressionsplasmid codierten Flp-Rekombinase integriert worden ist. Die Integration des pcDNA5/FRT-Expressionskonstrukts in das Flp-In-Wirtszellliniengenom führt zur Transkription des interessierenden Gens. Die stabil transfizierten Zellen werden hygromycinresistent.

Einen Tag vor der Transfektion wurden 200 000 Flp-In-CHO-Zellen in Ham-F-12-Medium (Invitrogen, Kat.-Nr. 31765) mit 10% fötalem Kälberserum (FCS; Perbio Science, Kat.-Nr. SH30068.03) in einer Platte mit 6 Vertiefungen ausgesät und über Nacht bei 37°C/5 % CO₂ inkubiert. Unter Verwendung von FuGENE^{®}-6-Transfektionsreagens (Roche, Kat.-Nr. 11988387001) wurden Zellen mit dem Flp-Rekombinase-Expressionsplasmid pOG44 und einem modifizierten Plasmid, das zusätzlich das edg-1-Gen (Zugangsnummer NM_001400) enthält und als pcDNA5-FRT-TO_nFLAG_DEST-EDG-1 bezeichnet wird, mit einem Verhältnis von 9:1 kotransfiziert. Zum Erhalt des modifizierten pcDNA5-FRT-TO_nFLAG_DEST-Plasmids wurde das Invitrogen-Plasmid pcDNA5/FRT/TO (Invitrogen, Kat.-Nr. V6520-20) durch Insertierung einer Gateway-Kassette mit ein ccdB-Gen und ein Chloramphenicolresistenzgen flankierenden attR-Rekombinationsstellen (Gateway Conversion System, Invitrogen, Kat.-Nr. 11828-029) auf das Gateway^{®}-Kloniersystem (Invitrogen) angepasst. Außerdem wurde vor der 5'-att-Rekombinationsstelle ein FLAG-Tag-Epitop hinzugefügt, um eine rekombinante Expression von Proteinen mit N-terminalem FLAG-Tag zu ermöglichen.

Für die Transfektion einer Vertiefung wurden 1,08 µg pOG44 und 0,12 µg pcDNA5-FRT-TO_nFLAG_DEST-EDG-1 mit 100 µl serumfreiem Ham-F-12-Medium mit 6 µl FuGENE^{®}-6-Transfektionsreagens gemischt. Nach 20 min Inkubation wurde der Transfektionsreagens/DNA-Komplex tropfenweise auf den Zellen verteilt. Die Zellen wurden 24 h bei 37°C inkubiert. Dann wurden die Zellen aus drei Vertiefungen in eine T75-Flasche (Greiner Cellstar^{®}, Kat.-Nr. 658175) mit Ham-F-12-Medium mit 10% FCS, aber ohne Antibiotikum, überführt und noch 24 h inkubiert. 48 h nach der Transfektion wurde das Medium durch Selektionsmedium (Ham F-12 mit 10 % FCS und 300 µg/ml Hygromycin B (Invitrogen, Kat.-Nr. 10687-010)) ersetzt. Das Medium wurde alle 2 bis 3 Tage ausgetauscht, bis eine resistente Population von Zellen herangewachsen war. Zellen wurden mehrmals aufgeteilt und in eine neue Flasche ausgesät, so daß die Zellen nicht mehr als 25% Konfluenz erreichten. Nach 2 Wochen Selektion wurden die Zellen in T175-Flaschen (Greiner Cellstar^{®}, Kat.-Nr. 660175) überführt und für die Batchproduktion kultiviert. Die Zellen wurden durch kurze Behandlung (2 bis 5 min) mit Accutase (PAA, Kat.-Nr. L11-007) aus den Kulturflaschen geerntet, in Selektionsmedium (siehe oben) resuspendiert und 5 min bei 200 x g zentrifugiert. Die Zellen wurden in einer Mischung von 90% FCS und 10% Dimethylsulfoxid resuspendiert und in flüssigem Stickstoff gefroren gelagert.

### (b) Membranpräparat

Aus der obenbeschriebenen CHO-Flp-In-Zelllinie, die den humanen Edg-1-Rezeptor konstitutiv überexprimiert, wurde nach Standardmethoden ein Membranpräparat erhalten. Kurz gesagt, wurden die kryokonservierten Zellen in Kultur genommen und in T175-Zellkulturflaschen (Becton Dickinson, Kat.-Nr. 35 5001) bis zur Konfluenz angezogen. Die Zellkultur wurde durch Waschen mit calciumfreier phosphatgepufferter Kochsalzlösung (PBS; Gibco, Kat.-Nr. 14190) gestoppt, und die Zellen wurden mit einem Gummischaber in 4°C kaltem und calciumfreiem PBS mit einem Proteaseinhibitorcocktail (Complete Protease Inhibitor; Roche, Kat.-Nr. 1697498; 1 Tablette pro 50 ml) geerntet und danach bei 4°C 15 min bei 1100 x g zentrifugiert (Heraeus Minifuge T). Zur Zelllyse wurde das Pellett in 4°C kaltem hypotonischem Puffer aus 5 mM HEPES (Sigma-Aldrich, Kat.-Nr. H-0981), 1 mM EDTA (Dinatriumsalz; Merck, Kat.-Nr. 8418) mit Proteaseinhibitorcocktail (wie oben) resuspendiert, in dem die Zellen noch 15 min auf Eis gelagert wurden. Nach der Lyse wurden die Zellen bei 4°C 10 min bei 400 x g zentrifugiert (Heraeus Minifuge T). Das Pellet wurde in einem Dounce-Homogenisator auseinandergebrochen, mit dem Überstand der vorhergehenden Zentrifugation verdünnt und danach bei 4°C 10 min bei 500 x g zentrifugiert (Heraeus Minifuge T), um Nuklei und noch intakte Zellen von den hauptsächlich im Überstand vorliegenden Membranen abzutrennen. Der Überstand wurde dann in hypotonischem Puffer verdünnt und bei 4°C bei ungefähr 18600 x g 2 h zentrifugiert (Beckmann, Avanti J251). Danach wurde das Membranpellet in einem Lagerpuffer aus 20 mM HEPES; 150 mM NaCl (Merck, Kat.-Nr. 6400), 1 mM EDTA (wie oben) mit Proteaseinhibitorcocktail (wie oben) resuspendiert. Das Membranpräparat wurde aliquotiert und bei -80°C gelagert. Die Proteinkonzentration des Membranpräparats wurde in einer Probe mit Hilfe eines kommerziellen Proteinassays (Bio-Rad, DC Protein Assay, Kat.-Nr. 500-0113, 500-0114, 500-0115) bestimmt.

### (c) GTP-γ-S-Assay

Das in (b) erhaltene Edg-1-Membranpräparat wurde in einem kommerziell erhältlichen Szintillationsproximitätsassay-Kit (SPA-Kit) auf die Bindung am G-Protein-gekoppelten Rezeptor von Amersham Biosciences/GE Healthcare (Code RPNQ0210) eingesetzt, indem die ligandeninduzierte Bindung von ³⁵S-radiomarkiertem GTP-γ-S an die rezeptorhaltige Membran, die an Szintillationsperlen gebunden ist, die Emission von Licht induziert und die Quantifizierung der in-vitro-Wirkung der Edg-1-agonistischen Verbindung erlaubt. Der Assay wurde auf einer Platte mit 96 Vertiefungen weitgehend nach den Anweisungen des Herstellers durchgeführt. Vor dem Beginn der Experimente wurden Szintillationsperlen in einem Rekonstitutionspuffer aus Tris-HCl (pH 7,4) mit 0,1% (w/v) Natriumazid suspendiert und dann auf Eis mit Assaypuffer (aus 20 mM HEPES, 100 mM NaCl, 1 mM EDTA (wie oben), 1 mM Dithiothreitol (DTT), auf pH 7,4 eingestellt) auf eine Perlenendkonzentration von 30 mg/ml verdünnt.

Die Vertiefungen wurden mit 10 µl des angegebenen Assaypuffers, 10 µl 100 µM Guanosindiphosphat-Lösung (GDP-Lösung) und 10 µl einer Lösung der Testverbindung in Assaypuffer/Dimethylsulfoxid versetzt, was eine Endkonzentration der Testverbindung von 10 µM ergibt. Anstelle der Lösung der Testverbindung wurden für die hohen Kontrollen 10 µl einer Lösung von Sphingosin-1-phosphat (S1P; Sigma, Kat.-Nr. S-9666), was eine S1 P-Endkonzentration von 10 µM ergab, und für die niedrigen Kontrollen 10 µl Assaypuffer in die jeweiligen Vertiefungen gegeben. Alle Vertiefungen enthielten äquivalente Mengen von Dimethylsulfoxid. Dann wurden in jede Vertiefung 10 µl einer [³⁵S]GTP-γ-S-Lösung (4 nM) und das in (b) erhaltene Edg-1-Membranpräparat (15 µg Membranprotein in 100 µl Assaypuffer) gegeben. Nach Inkubation der Platten bei Raumtemperatur über einen Zeitraum von 5 min wurden 50 µl der angegebenen Szintillationsperlensuspension (30 mg/ml) zugegeben. Nach einem weiteren Inkubationszeitraum von 45 min bei Raumtemperatur wurden die Platten 10 min bei 500 x g zentrifugiert. Die Quantifizierung der [³⁵S]GTP-γ-S-Bindung und somit der Rezeptoraktivierung wurde mit Hilfe eines Beta-Zählers (MicroBeta, Wallac) über einen Zeitraum von 1 min gemessen. Die Werte wurden durch Subtraktion der jeweiligen niedrigen Kontrolle hintergrundkorrigiert. Alle Messungen wurden dreifach durchgeführt. Die Rezeptoraktivierung durch die Testverbindung wird in % der jeweiligen hohen Kontrolle (10 µM S1P; wird als 100% Aktivierung erachtet) ausgedrückt. Die mit Beispielverbindungen bei 10 µM beobachteten Aktivierungen sind in Tabelle 2 aufgeführt.

**Tabelle 2, Edg-1-Rezeptor-Aktivierung durch Beispielverbindungen bei 10 µM in Prozent der Aktivierung durch 10 µM S1 P**

| Beispiel | % Aktivierung | | Beispiel | % Aktivierung |
|---|---|---|---|---|
| 1 | 62 | | 81 | 89 |
| 2 | 90 | | 82 | 107 |
| 3 | 74 | | 83 | 63 |
| 4 | 93 | | 84 | 60 |
| 5 | 111 | | 85 | 67 |
| 6 | 105 | | 86 | 98 |
| 7 | 116 | | 87 | 93 |
| 8 | 124 | | 88 | 86 |
| 9 | 87 | | 89 | 96 |
| 10 | 107 | | 90 | 67 |
| 11 | 105 | | 91 | 105 |
| 12 | 84 | | 92 | 51 |
| 13 | 104 | | 93 | 60 |
| 14 | 72 | | 94 | 122 |
| 15 | 95 | | 95 | 107 |
| 16 | 83 | | 96 | 67 |
| 17 | 55 | | 97 | 124 |
| 18 | 42 | | 98 | 107 |
| 19 | 62 | | 99 | 91 |
| 20 | 83 | | 100 | 108 |
| 21 | 81 | | 101 | 104 |
| 22 | 100 | | 102 | 130 |
| 23 | 47 | | 103 | 109 |
| 24 | 99 | | 104 | 117 |
| 25 | 110 | | 105 | 88 |
| 26 | 101 | | 106 | 117 |
| 27 | 93 | | 107 | 129 |
| 28 | 69 | | 108 | 98 |
| 29 | 109 | | 109 | 108 |
| 30 | 134 | | 110 | 80 |
| 31 | 80 | | 111 | 88 |
| 32 | 61 | | 112 | 46 |
| 33 | 124 | | 113 | 52 |
| 34 | 99 | | 114 | 96 |
| 35 | 72 | | 115 | 96 |
| 36 | 78 | | 116 | 70 |
| 37 | 86 | | 117 | 63 |
| 38 | 70 | | 118 | 98 |
| 39 | 47 | | 119 | 116 |
| 40 | 120 | | 120 | 86 |
| 41 | 102 | | 121 | 113 |
| 42 | 130 | | 122 | 65 |
| 43 | 98 | | 123 | 65 |
| 44 | 94 | | 124 | 112 |
| 45 | 74 | | 125 | 61 |
| 46 | 102 | | 126 | 121 |
| 47 | 119 | | 127 | 110 |
| 48 | 105 | | 128 | 105 |
| 49 | 123 | | 129 | 85 |
| 50 | 107 | | 130 | 107 |
| 51 | 110 | | 131 | 51 |
| 52 | 51 | | 132 | 129 |
| 53 | 73 | | 133 | 92 |
| 54 | 105 | | 134 | 104 |
| 55 | 81 | | 135 | 1 |
| 56 | 97 | | 136 | 91 |
| 57 | 80 | | 137 | 89 |
| 58 | 102 | | 138 | 97 |
| 59 | 103 | | 139 | 120 |
| 60 | 80 | | 140 | 60 |
| 61 | 74 | | 141 | 103 |
| 62 | 111 | | 142 | 50 |
| 63 | 80 | | 143 | 52 |
| 64 | 109 | | 144 | 76 |
| 65 | 105 | | 145 | 82 |
| 66 | 106 | | 146 | 126 |
| 67 | 84 | | 147 | 63 |
| 68 | 41 | | 148 | 58 |
| 69 | 41 | | 149 | 70 |
| 70 | 77 | | 150 | 92 |
| 71 | 102 | | 151 | 66 |
| 72 | 95 | | 152 | 46 |
| 73 | 80 | | 153 | 108 |
| 74 | 69 | | 154 | 87 |
| 75 | 72 | | 155 | 62 |
| 76 | 81 | | 156 | 40 |
| 77 | 111 | | 157 | 92 |
| 78 | 70 | | 158 | 69 |
| 79 | 64 | | 159 | 55 |
| 80 | 46 | | 160 | 75 |

Aus den Messdaten ist ersichtlich, dass die Verbindungen gut zur Wundheilung und insbesondere zur Behandlung von Wundheilungsstörungen von Diabetes Patienten geeignet sind.

## Patentansprüche

1. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes, 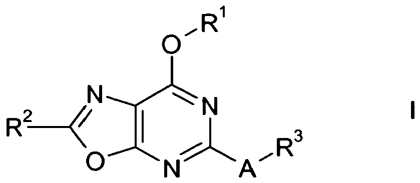 worin
A ausgewählt ist aus NH, O und S;
R¹ ausgewählt ist aus (C₁-C₆)-Alkyl, (C₂-C₆)-Alkenyl, (C₂-C₆)-Alkinyl, (C₃-C₇)-Cycloalkyl-CₜH₂ₜ- und Het-CₜH₂ₜ-, worin t aus 0, 1, 2 und 3 ausgewählt ist;
R² ausgewählt ist aus Phenyl und einem Rest eines aromatischen, 5-gliedrigen bis 6-gliedrigen monocyclischen Heterocyclus, der 1, 2 oder 3 gleiche oder verschiedene Ringheteroatome enthält, die aus N, O und S ausgewählt sind, wobei eines der Ringstickstoffatome ein Wasserstoffatom oder einen Substituenten R²¹ tragen kann und wobei das Phenyl und der Rest eines aromatischen Heterocyclus gegebenenfalls an einem oder mehreren Ringkohlenstoffatomen durch gleiche oder verschiedene Substituenten R²² substituiert sind;
R³ ausgewählt ist aus(C₁-C₆)-Alkyl, (C₂-C₆)-Alkenyl, (C₂-C₆)-Alkinyl, (C₃-C₇)-Cyclo-alkyl-CᵤH₂ᵤ- und Het-CᵥH₂ᵥ-, worin u und v aus 1 und 2 ausgewählt sind, oder R³ für einen Rest eines gesättigten oder ungesättigten, 3-gliedrigen bis 10-gliedrigen, monocyclischen oder bicyclischen Rings, der 0, 1, 2, 3 oder 4 gleiche oder verschiedene Ringheteroatome enthält, die aus N, O und S ausgewählt sind, steht,
wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist, mit der Maßgabe, daß R³ nicht für (C₁-C₆)-Alkyl stehen kann, wenn A für S steht;
R²¹ ausgewählt ist aus (C₁-C₄)-Alkyl, (C₃-C₇)-Cycloalkyl-C_{w}H_{2w} und Oxy, wobei w aus 0, 1 und 2 ausgewählt ist;
R²² ausgewählt ist aus Halogen, Hydroxy, (C₁-C₄)-Alkyl-, (C₁-C₄)-Alkyloxy, (C₁-C₄)-Alkyl-S(O)ₘ-, Amino, Nitro, Cyano, Hydroxycarbonyl, (C₁-C₄)-Alkyloxycarbonyl, Aminocarbonyl, Aminosulfonyl, R²³ und R²³-O-;
R²³ für einen Rest eines gesättigten, 3-gliedrigen bis 7-gliedrigen, monocyclischen oder bicyclischen Rings, der 0, 1, 2, 3 oder 4 gleiche oder verschiedene Ringheteroatome enthält, die aus N, O und S ausgewählt sind, steht, wobei ein oder zwei der Ringstickstoffatome ein Wasserstoffatom oder einen (C₁-C₄)-Alkylsubstituenten tragen können und eines der Ringschwefelatome eine oder zwei Oxogruppen tragen kann und wobei der Rest eines Rings gegebenenfalls an einem oder mehreren Ringkohlenstoffatomen durch gleiche oder verschiedene Substituenten R²⁴ substituiert ist;
R²⁴ ausgewählt ist aus Halogen, (C₁-C₄)-Alkyl, Hydroxy und Oxo;
R³¹ ausgewählt ist aus Halogen, (C₁-C₄)-Alkyl, (C₃-C₇)-Cycloalkyl, Hydroxy, (C₁-C₄)-Alkyloxy, Oxo, (C₁-C₄)-Alkyl-S(O)ₘ-, Amino, (C₁-C₄)-Alkylamino, Di((C₁-C₄)-alkyl)amino, (C₁-C₄)-Alkylcarbonylamino, (C₁-C₄)-Alkylsulfonylamino, Nitro, Cyano, (C₁-C₄)-Alkylcarbonyl, Aminosulfonyl, (C₁-C₄)-Alkylaminosulfonyl und Di((C₁-C₄)-alkyl)aminosulfonyl;
Het für einen Rest eines gesättigten, 4-gliedrigen bis 7-gliedrigen, monocyclischen Heterocyclus, der 1 oder 2 gleiche oder verschiedene Ringheteroatome enthält, die unter N, O und S ausgewählt sind, und der über ein Ringkohlenstoffatom gebunden ist, steht, wobei der Rest eines Heterocyclus gegebenenfalls durch einen oder mehrere gleiche oder verschiedene Substituenten substituiert ist, die unter Fluor und (C₁-C₄)-Alkyl ausgewählt sind;
m aus 0, 1 und 2 ausgewählt ist, wobei alle Zahlen m voneinander unabhängig sind;
wobei alle Cycloalkylgruppen unabhängig voneinander und unabhängig von anderen Substituenten gegebenenfalls durch einen oder mehrere gleiche oder verschiedene Substituenten substituiert sind, die unter Fluor und (C₁-C₄)-Alkyl ausgewählt sind;
wobei alle Alkyl-, CₜH₂ₜ-, CᵤH₂ᵤ-, CᵥH₂ᵥ-, C_{w}H_{2w}-, Alkenyl- und Alkinylgruppen unabhängig voneinander und unabhängig von anderen Substituenten gegebenenfalls durch einen oder mehrere Fluorsubstituenten substituiert sind.

2. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach Anspruch 1, worin R¹ ausgewählt ist aus (C₁-C₆)-Alkyl, (C₃-C₇)-Cycloalkyl-CₜH₂ₜ- und Het-CₜH₂ₜ-, worin t aus 0, 1 und 2 ausgewählt ist.

3. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach einem oder mehreren der Ansprüche 1 und 2, worin R² aus Phenyl und Pyridinyl ausgewählt ist, wobei das Ringstickstoffatom des Pyridinyls einen Oxysubstituenten tragen kann und wobei das Phenyl und das Pyridinyl gegebenenfalls an einem oder mehreren Ringkohlenstoffatomen durch gleiche oder verschiedene Substituenten R²² substituiert sind.

4. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach einem oder mehreren der Ansprüche 1 bis 3, worin R³ ausgewählt ist aus (C₃-C₇)-Cycloalkyl-CᵤH₂ᵤ- und Het-CᵥH₂ᵥ-, worin u und v aus 1 und 2 ausgewählt sind, oder R³ für einen Rest eines gesättigten oder ungesättigten, 3-gliedrigen bis 10-gliedrigen, monocyclischen oder bicyclischen Rings, der 0, 1, 2, 3 oder 4 gleiche oder verschiedene Ringheteroatome enthält, die unter N, O und S ausgewählt sind, steht, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist.

5. Verbindung der Formel I ihn einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach einem oder mehreren der Ansprüche 1 bis 4, worin
R¹ ausgewählt ist aus (C₁-C₆)-Alkyl, (C₃-C₇)-Cycloalkyl-CₜH₂ₜ- und Het-CₜH₂ₜ-, worin t aus 0, 1 und 2 ausgewählt ist;
R² ausgewählt ist aus Phenyl und Pyridinyl, worin das Ringstickstoffatom des Pyridinyls einen Oxysubstituenten tragen kann und wobei das Phenyl und das Pyridinyl gegebenenfalls an einem oder mehreren Ringkohlenstoffatomen durch gleiche oder verschiedene Substituenten R²² substituiert sind;
R³ ausgewählt ist aus (C₃-C₇)-Cycloalkyl-CᵤH₂ᵤ- und Het-CᵥH₂ᵥ-, worin u und v aus 1 und 2 ausgewählt sind, oder R³ für einen Rest eines gesättigten oder ungesättigten, 3-gliedrigen bis 1 0-gliedrigen, monocyclischen oder bicyclischen Rings, der 0, 1, 2, 3 oder 4 gleiche oder verschiedene Ringheteroatome enthält, die unter N, O und S ausgewählt sind, steht, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist.

6. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach einem oder mehreren der Ansprüche 1 bis 5, worin
A ausgewählt ist aus NH, O und S;
R¹ ausgewählt ist aus (C₁-C₆)-Alkyl, (C₃-C₇)-Cycloalkyl-CₜH₂ₜ- und Het-CₜH₂ₜ-, worin t aus 0, 1 und 2 ausgewählt ist;
R² ausgewählt ist aus Phenyl und Pyridinyl, wobei das Ringstickstoffatom des Pyridinyls einen Oxysubstituenten tragen kann und wobei das Phenyl und das Pyridinyl gegebenenfalls an einem oder mehreren Ringkohlenstoffatomen durch gleiche oder verschiedene Substituenten R²² substituiert sind;
R³ ausgewählt ist aus (C₃-C₇)-Cycloalkyl-CᵤH₂ᵤ- und Het-CᵥH₂ᵥ-, worin u und v unter 1 und 2 ausgewählt sind, oder R³ für einen Rest eines gesättigten oder ungesättigten, 3-gliedrigen bis 7-gliedrigen, monocyclischen Rings, der 0, 1 oder 2 gleiche oder verschiedene Ringheteroatome enthält, die unter N, O und S ausgewählt sind, steht, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist;
R²² ausgewählt ist aus Halogen, Hydroxy, (C₁-C₄)-Alkyl-, (C₁-C₄)-Alkyloxy, (C₁-C₄)-Alkyl-S(O)ₘ-, Amino, Cyano, Aminocarbonyl, Aminosulfonyl, R²³ und R²³-O-;
R²³ für einen Rest eines gesättigten 3-gliedrigen bis 6-gliedrigen, monocyclischen Rings, der 0,1 oder 2 gleiche oder verschiedene Ringheteroatome enthält, die aus N, O und S ausgewählt sind, steht, wobei ein oder zwei der Ringstickstoffatome ein Wasserstoffatom oder einen (C₁-C₄)-Alkylsubstituenten tragen können und eines der Ringschwefelatome eine oder zwei Oxogruppen tragen kann und wobei der Rest eines Rings gegebenenfalls an einem oder mehreren Ringkohlenstoffatomen durch gleiche oder verschiedene Substituenten R²⁴ substituiert ist;
R²⁴ ausgewählt ist aus Fluor, (C₁-C₄)-Alkyl und Hydroxy;
R³¹ ausgewählt ist aus Halogen, (C₁-C₄)-Alkyl, (C₃-C₇)-Cycloalkyl, Hydroxy, (C₁-C₄)-Alkyloxy, (C₁-C₄)-Alkyl-S(O)ₘ-, Amino, (C₁-C₄)-Alkylamino, Di((C₁-C₄)-alkyl)amino, (C₁-C₄)-Alkylcarbonylamino, (C₁-C₄)-Alkylsulfonylamino, Cyano, Aminosulfonyl, (C₁-C₄)-Alkylaminosulfonyl und Di((C₁-C₄)-alkyl)aminosulfonyl;
Het für einen Rest eines gesättigten, 4-gliedrigen bis 7-gliedrigen, monocyclischen Heterocyclus, der 1 oder 2 gleiche oder verschiedene Ringheteroatome enthält, die aus N, O und S ausgewählt sind, und der über ein Ringkohlenstoffatom gebunden ist, steht, wobei der Rest eines Heterocyclus gegebenenfalls durch einen oder mehrere gleiche oder verschiedene Substituenten substituiert ist, die aus Fluor und (C₁-C₄)-Alkyl ausgewählt sind;
m ausgewählt ist aus 0, 1 und 2, wobei alle Zahlen m voneinander unabhängig sind; wobei alle Cycloalkylgruppen unabhängig voneinander und unabhängig von anderen Substituenten gegebenenfalls durch einen oder mehrere gleiche oder verschiedene Substituenten substituiert sind, die aus Fluor und (C₁-C₄)-Alkyl ausgewählt sind;
wobei alle Alkyl-, CₜH₂ₜ-, CᵤH₂ᵤ- und CᵥH₂ᵥ-Gruppen unabhängig voneinander und unabhängig von anderen Substituenten gegebenenfalls durch einen oder mehrere Fluorsubstituenten substituiert sind.

7. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach einem oder mehreren der Ansprüche 1 bis 6, worin A für O steht.

8. Verfahren zur Herstellung einer Verbindung der Formel I nach einem der Ansprüche 1 bis 7, bei dem man eine Verbindung der Formel II mit einer Verbindung der Formel III umsetzt, 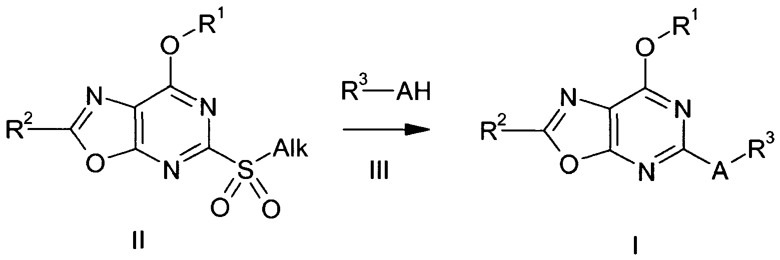 wobei die Gruppen A, R¹, R² und R³ in den Verbindungen der Formeln II und III wie in den Verbindungen der Formel I definiert sind und außerdem funktionelle Gruppen in geschützter Form oder in Form einer Vorläufergruppe vorliegen können und die Gruppe Alk für (C₁-C₄)-Alkyl steht.

9. Pharmazeutische Zusammensetzung, enthaltend mindestens eine Verbindung der Formel I nach einem der Ansprüche 1 bis 7 oder ein physiologisch akzeptables Salz davon oder ein physiologisch akzeptables Solvat einer derartigen Verbindung oder eines derartigen Salzes und einen pharmazeutisch akzeptablen Träger.

10. Verbindung der Formel I nach einem der Ansprüche 1 bis 7 oder physiologisch akzeptables Salz davon oder physiologisch akzeptables Solvat einer derartigen Verbindung oder eines derartigen Salzes zur Verwendung als Arzneimittel.

11. Verbindung der Formel I nach einem der Ansprüche 1 bis 7 oder eines physiologisch akzeptablen Salzes davon oder eines physiologisch akzeptablen Solvats einer derartigen Verbindung oder eines derartigen Salzes zur Behandlung von Wundheilungsstörungen.

12. Verbindung der Formel I nach einem der Ansprüche 1 bis 7 oder eines physiologisch akzeptablen Salzes davon oder eines physiologisch akzeptablen Solvats einer derartigen Verbindung oder eines derartigen Salzes zur Wundheilung.

13. Verbindung der Formel I nach einem der Ansprüche 1 bis 7 oder eines physiologisch akzeptablen Salzes davon oder eines physiologisch akzeptablen Solvats einer derartigen Verbindung oder eines derartigen Salzes zur Wundheilung bei Diabetikern.

14. Verbindung der Formel I nach einem der Ansprüche 1 bis 7 oder eines physiologisch akzeptablen Salzes davon oder eines physiologisch akzeptablen Solvats einer derartigen Verbindung oder eines derartigen Salzes zur Behandlung des Diabetischen Fußsyndroms.

## Claims

1. A compound of the formula I, in any of its stereoisomeric forms, or a mixture of stereoisomeric forms in any ratio, or a physiologically acceptable salt thereof, or a physiologically acceptable solvate of any of them, 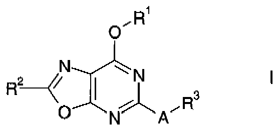 wherein
A is chosen from NH, O and S;
R¹ is chosen from (C₁-C₆)-alkyl, (C₂-C₆)-alkenyl, (C₂-C₆)-alkynyl, (C₃-C₇)-cycloalkyl-CₜH₂ₜ- and Het-CₜH₂ₜ-, wherein t is chosen from 0, 1, 2 and 3;
R² is chosen from phenyl and a residue of an aromatic, 5-membered to 6-membered monocyclic heterocycle which comprises 1, 2 or 3 identical or different ring heteroatoms chosen from N, O and S, wherein one of the ring nitrogen atoms can carry a hydrogen atom or a substituent R²¹, and wherein the phenyl and residue of an aromatic heterocycle are optionally substituted on one or more ring carbon atoms by identical or different substituents R²²;
R³ is chosen from (C₁-C₆)-alkyl, (C₂-C₆)-alkenyl, (C₂-C₆)-alkynyl, (C₃-C₇)-cycloalkyl-CᵤH₂ᵤ- and Het-CᵥH₂ᵥ-, wherein u and v are chosen from 1 and 2, or R³ is a residue of a saturated or unsaturated, 3-membered to 10-membered, monocyclic or bicyclic ring which comprises 0, 1, 2, 3 or 4 identical or different ring heteroatoms chosen from N, O and S, wherein one or two of the ring nitrogen atoms can carry a hydrogen atom or a (C₁-C₄)-alkyl substituent and one or two of the ring sulfur atoms can carry one or two oxo groups, and wherein the residue of a ring is optionally substituted on one or more ring carbon atoms by identical or different substituents R³¹, provided that R³ cannot be (C₁-C₆)-alkyl if A is S;
R²¹ is chosen from (C₁-C₄)-alkyl, (C₃-C₇)-cycloalkyl-C_{w}H_{2w}- and oxy, wherein w is chosen from 0, 1 and 2;
R²² is chosen from halogen, hydroxy, (C₁-C₄)-alkyl-, (C₁-C₄)-alkyloxy, (C₁-C₄)-alkyl-S(O)ₘ-, amino, nitro, cyano, hydroxycarbonyl, (C₁-C₄)-alkyloxycarbonyl, aminocarbonyl, aminosulfonyl, R²³ and R²³-O-;
R²³ is a residue of a saturated 3-membered to 7-membered, monocyclic or bicyclic ring which comprises 0, 1, 2, 3 or 4 identical or different ring heteroatoms chosen from N, O and S, wherein one or two of the ring nitrogen atoms can carry a hydrogen atom or a (C₁-C₄)-alkyl substituent and one of the ring sulfur atoms can carry one or two oxo groups, and wherein the residue of a ring is optionally substituted on one or more ring carbon atoms by identical or different substituents R²⁴;
R²⁴ is chosen from halogen, (C₁-C₄)-alkyl, hydroxy and oxo;
R³¹ is chosen from halogen, (C₁-C₄)-alkyl, (C₃-C₇)-cycloalkyl, hydroxy, (C₁-C₄)-alkyloxy, oxo, (C₁-C₄)-alkyl-S(O)ₘ-, amino, (C₁-C₄)-alkylamino, di((C₁-C₄)-alkyl)amino, (C₁-C₄)-alkylcarbonylamino, (C₁-C₄)-alkylsulfonylamino, nitro, cyano, (C₁-C₄)-alkylcarbonyl, aminosulfonyl, (C₁-C₄)-alkylaminosulfonyl and di((C₁-C₄)-alkyl)aminosulfonyl;
Het is a residue of a saturated, 4-membered to 7-membered, monocyclic heterocycle which comprises 1 or 2 identical or different ring heteroatoms chosen from N, O and S and which is bonded via a ring carbon atom, wherein the residue of a heterocycle is optionally substituted by one or more identical or different substituents chosen from fluorine and (C₁-C₄)-alkyl;
m is chosen from 0, 1 and 2, wherein all numbers m are independent of each other;
wherein all cycloalkyl groups, independently of each other and independently of any other substituents, are optionally substituted by one or more identical or different substituents chosen from fluorine and (C₁-C₄)-alkyl;
wherein all alkyl, CₜH₂ₜ, CᵤH₂ᵤ, CᵥH₂ᵥ, C_{w}H_{2w}, alkenyl and alkynyl groups, independently of each other and independently of any other substituents, are optionally substituted by one or more fluorine substituents.

2. A compound of the formula I, in any of its stereoisomeric forms, or a mixture of stereoisomeric forms in any ratio, or a physiologically acceptable salt thereof, or a physiologically acceptable solvate of any of them, as claimed in claim 1, wherein R¹ is chosen from (C₁-C₆)-alkyl, (C₃-C₇)-cycloalkyl-CₜH₂ₜ and Het-CₜH₂ₜ-, wherein t is chosen from 0, 1 and 2.

3. A compound of the formula I, in any of its stereoisomeric forms, or a mixture of stereoisomeric forms in any ratio, or a physiologically acceptable salt thereof, or a physiologically acceptable solvate of any of them, as claimed in one or more of claims 1 and 2, wherein R² is chosen from phonyl and pyridinyl, wherein the ring nitrogen atom of the pyridinyl can carry an oxy substituent, and wherein the phenyl and the pyridinyl are optionally substituted on one or more ring carbon atoms by identical or different substituents R²².

4. A compound of the formula I, in any of its stereoisomeric forms, or a mixture of stereoisomeric forms in any ratio, or a physiologically acceptable salt thereof, or a physiologically acceptable solvate of any of them, as claimed in one or more of claims 1 to 3, wherein R³ is chosen from (C₃-C₇)-cycloalkyl-CᵤH₂ᵤ- and Het-CᵥH₂ᵥ-, wherein u and v are chosen from 1 and 2, or R³ is a residue of a saturated or unsaturated, 3-membered to 10-membered, monocyclic or bicyclic ring which comprises 0, 1, 2, 3 or 4 identical or different ring heteroatoms chosen from N, O and S, wherein one or two of the ring nitrogen atoms can carry a hydrogen atom or a (C₁-C₄)-alkyl substituent and one or two of the ring sulfur atoms can carry one or two oxo groups, and wherein the residue of a ring is optionally substituted on one or more ring carbon atoms by identical or different substituents R³¹.

5. A compound of the formula I, in any of its stereoisomeric forms, or a mixture of stereoisomeric forms in any ratio, or a physiologically acceptable salt thereof, or a physiologically acceptable solvate of any of them, as claimed in one or more of claims 1 to 4, wherein
R¹ is chosen from (C₁-C₆)-alkyl, (C₃-C₇)-cycloalkyl-CₜH₂ₜ- and Het-CₜH₂ₜ-, wherein t is chosen from 0, 1 and 2;
R² is chosen from phenyl and pyridinyl, wherein the ring nitrogen atom of the pyridinyl can carry an oxy substituent, and wherein the phenyl and the pyridinyl are optionally substituted on one or more ring carbon atoms by identical or different substituents R²²;
R³ is chosen from (C₃-C₇)-cycloalkyl-CᵤH₂ᵤ- and Het-CᵥH_{2v-,} wherein u and v are chosen from 1 and 2, or R³ is a residue of a saturated or unsaturated, 3-membered to 10-membered, monocyclic or bicyclic ring which comprises 0, 1, 2, 3 or 4 identical or different ring heteroatoms chosen from N, O and S, wherein one or two of the ring nitrogen atoms can carry a hydrogen atom or a (C₁-C₄)-alkyl substituent and one or two of the ring sulfur atoms can carry one or two oxo groups, and wherein the residue of a ring is optionally substituted on one or more ring carbon atoms by identical or different substituents R³¹.

6. A compound of the formula I, in any of its stereoisomeric forms, or a mixture of stereoisomeric forms in any ratio, or a physiologically acceptable salt thereof, or a physiologically acceptable solvate of any of them, as claimed in one or more of claims 1 to 5, wherein
A is chosen from NH, O and S;
R¹ is chosen from (C₁-C₆)-alkyl, (C₃-C₇)-cycloalkyl-CₜH₂ₜ- and Het-CₜH₂ₜ-, wherein t is chosen from 0, 1 and 2;
R² is chosen from phenyl and pyridinyl, wherein the ring nitrogen atom of the pyridinyl can carry an oxy substituent, and wherein the phenyl and the pyridinyl are optionally substituted on one or more ring carbon atoms by identical or different substituents R²²;
R³ is chosen from (C₃-C₇)-cycloalkyl-CᵤH₂ᵤ- and Het-CᵥH₂ᵥ-, wherein u and v are chosen from 1 and 2, or R³ is a residue of a saturated or unsaturated, 3-membered to 7-membered, monocyclic ring which comprises 0, 1 or 2 identical or different ring heteroatoms chosen from N, O and S, wherein one or two of the ring nitrogen atoms can carry a hydrogen atom or a (C₁-C₄)-alkyl substituent and one or two of the ring sulfur atoms can carry one or two oxo groups, and wherein the residue of a ring is optionally substituted on one or more ring carbon atoms by identical or different substituents R³¹;
R²² is chosen from halogen, hydroxy, (C₁-C₄)-alkyl-, (C₁-C₄)-alkyloxy, (C₁-C₄)-alkyl-S(O)ₘ-, amino, cyano, aminocarbonyl, aminosulfonyl, R²³ and R²³-O-;
R²³ is a residue of a saturated 3-membered to 6-membered, monocyclic ring which comprises 0, 1 or 2 identical or different ring heteroatoms chosen from N, O and S, wherein one or two of the ring nitrogen atoms can carry a hydrogen atom or a (C₁-C₄)-alkyl substituent and one of the ring sulfur atoms can carry one or two oxo groups, and wherein the residue of a ring is optionally substituted on one or more ring carbon atoms by identical or different substituents R²⁴;
R²⁴ is chosen from fluorine, (C₁-C₄)-alkyl and hydroxy;
R³¹ is chosen from halogen, (C₁-C₄)-alkyl, (C₃-C-₇)cycloalkyl, hydroxy, (C₁-C₄)-alkyloxy, (C₁-C₄)-alkyl-S(O)ₘ-, amino, (C₁-C₄)-alkylamino, di((C₁-C₄)-alkyl)amino, (C₁-C₄)-alkylcarbonylamino, (C₁-C₄)-alkylsulfonylamino, cyano, aminosulfonyl, (C₁-C₄)-alkylaminosulfonyl and di((C₁-C₄)-alkyl)aminosulfonyl;
Het is a residue of a saturated, 4-membered to 7-membered, monocyclic heterocycle which comprises 1 or 2 identical or different ring heteroatoms chosen from N, O and S and which is bonded via a ring carbon atom, wherein the residue of a heterocycle is optionally substituted by one or more identical or different substituents chosen from fluorine and (C₁-C₄)-alkyl;
m is chosen from 0, 1 and 2, wherein all numbers m are independent of each other;
wherein all cycloalkyl groups, independently of each other and independently of any other substituents, are optionally substituted by one or more identical or different substituents chosen from fluorine and (C₁-C₄)-alkyl;
wherein all alkyl, CₜH₂ₜ, CᵤH₂ᵤ and CᵥH₂ᵥ groups, independently of each other and independently of any other substituents, are optionally substituted by one or more fluorine substituents.

7. A compound of the formula I, in any of its stereoisomeric forms, or a mixture of stereoisomeric forms in any ratio, or a physiologically acceptable salt thereof, or a physiologically acceptable solvate of any of them, as claimed in one or more of claims 1 to 6, wherein A is O.

8. A process for the preparation of a compound of the formula I as claimed in any of claims 1 to 7, comprising reacting a compound of the formula II with a compound of the formula III, 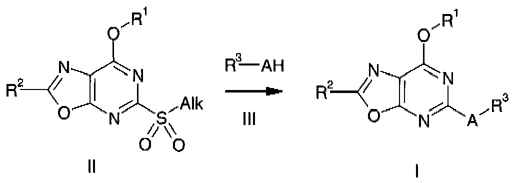 wherein the groups A, R¹, R² and R³ in the compounds of the formulae II and III are defined as in the compounds of the formula I and additionally functional groups can be present in protected form or in the form of a precursor group, and the group Alk is (C₁-C₄)-alkyl.

9. A pharmaceutical composition, comprising at least one compound of the formula I as claimed in any of claims 1 to 7 or a physiologically acceptable salt thereof, or a physiologically acceptable solvate of any of them, and a pharmaceutically acceptable carrier.

10. A compound of the formula I as claimed in any of claims 1 to 7 or a physiologically acceptable salt thereof, or a physiologically acceptable solvate of any of them, for use as a pharmaceutical.

11. A compound of the formula I as claimed in any of claims 1 to 7 or a physiologically acceptable salt thereof, or a physiologically acceptable solvate of any of them, for the treatment of wound healing disorders.

12. A compound of the formula I as claimed in any of claims 1 to 7 or a physiologically acceptable salt thereof, or a physiologically acceptable solvate of any of them for wound healing.

13. A compound of the formula I as claimed in any of claims 1 to 7 or a physiologically acceptable salt thereof, or a physiologically acceptable solvate of any of them for wound healing in diabetics.

14. A compound of the formula I as claimed in any of claims 1 to 7 or a physiologically acceptable salt thereof, or a physiologically acceptable solvate of any of them, for the treatment of diabetic foot syndrome.

## Revendications

1. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel, 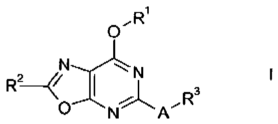 dans lequel
A est choisi parmi NH, O et S ;
R¹ est choisi parmi alkyle en (C₁-C₆), alcényle en (C₂-C₆), alcynyle en (C₂-C₆), cycloalkyle en (C₃-C₇)-CₜH₂ₜ- et Het-CₜH₂ₜ-, t étant choisi parmi 0, 1, 2 et 3;
R² est choisi parmi phényle et un radical d'un hétérocycle monocyclique aromatique de 5 éléments à 6 éléments, qui contient 1, 2 ou 3 hétéroatomes de cycle identiques ou différents choisis parmi N, O et S, un des atomes d'azote de cycle pouvant porter un atome d'hydrogène ou un substituant R²¹, et le phényle et le radical d'un hétérocycle aromatique étant éventuellement substitués sur un ou plusieurs atomes de carbone de cycle par des substituants R²² identiques ou différents ;
R³ est choisi parmi alkyle en (C₁-C₆), alcényle en (C₂-C₆), alcynyle en (C₂-C₆), cycloalkyle en (C₃-C₇)-CᵤH₂ᵤ- et Het-CᵥH₂v-, u et v étant choisis parmi 1 et 2, ou R³ représente un radical d'un cycle monocyclique ou bicyclique, saturé ou insaturé, de 3 éléments à 10 éléments, qui contient 0, 1, 2, 3 ou 4 hétéroatomes de cycle identiques ou différents choisis parmi N, O et S, un ou deux des atomes d'azote de cycle pouvant porter un atome d'hydrogène ou un substituant alkyle en (C₁-C₄), et un ou deux des atomes de soufre de cycle pouvant porter un ou deux groupes oxo, et le radical d'un cycle étant éventuellement substitué sur un ou plusieurs atomes de carbone de cycle par des substituants R³¹ identiques ou différents à condition que R³ ne puisse pas représenter alkyle en (C₁-C₆) lorsque A représente S ;
R²¹ est choisi parmi alkyle en (C₁-C₄), cycloalkyle en (C₃-C₇)-C_{w}H_{2w}- et oxy, w étant choisi parmi 0, 1 et 2 ;
R²² est choisi parmi halogène, hydroxy, alkyle en (C₁-C₄), alkyloxy en (C₁-C₄), alkyl-S(O)ₘ en (C₁-C₄), amino, nitro, cyano, hydroxycarbonyle, alkyloxycarbonyle en (C₁-C₄), aminocarbonyle, aminosulfonyle, R²³ et R²³. O-;
R²³ représente un radical d'un cycle saturé, de 3 éléments à 7 éléments, monocyclique ou bicyclique, qui contient 0, 1, 2, 3 ou 4 hétéroatomes de cycle identiques ou différents choisis parmi N, O et S, un ou deux des atomes d'azote de cycle pouvant porter un atome d'hydrogène ou un substituant alkyle en (C₁-C₄) et un des atomes de soufre de cycle pouvant porter un ou deux groupes oxo, et le radical d'un cycle étant éventuellement substitué sur un ou plusieurs atomes de carbone de cycle par des substituants R²⁴ identiques ou différents ;
R²⁴ est choisi parmi halogène, alkyle en (C₁-C₄), hydroxy et oxo ;
R³¹ est choisi parmi halogène, alkyle en (C₁-C₄), cycloalkyle en (C₃-C₇), hydroxy, alkyloxy en (C₁-C₄), oxo, alkyl-S(O)ₘ en (C₁-C₄), amino, alkylamino en (C₁-C₄), di(alkyle en (C₁-C₄))-amino, alkylcarbonylamino en (C₁-C₄), alkylsulfonylamino en (C₁-C₄), nitro, cyano, alkylcarbonyle en (C₁-C₄), aminosulfonyle, alkylaminosulfonyle en (C₁-C₄) et di(alkyle en (C₁-C₄))-aminosulfonyle ;
Het représente un radical d'un hétérocycle monocyclique saturé de 4 éléments à 7 éléments, qui contient 1 ou 2 hétéroatomes de cycle identiques ou différents choisis parmi N, O et S, et qui est relié par un atome de carbone de cycle, le radical d'un hétérocycle étant éventuellement substitué par un ou plusieurs substituants identiques ou différents choisis parmi fluor et alkyle en (C₁-C₄) ;
m est choisi parmi 0, 1 et 2, tous les nombres m étant indépendants les uns des autres ;
tous les groupes cycloalkyle étant éventuellement substitués indépendamment les uns des autres et indépendamment des autres substituants par un ou plusieurs substituants identiques ou différents choisis parmi fluor et alkyle en (C₁-C₄);
tous les groupes alkyle, CₜH₂ₜ, CᵤH₂ᵤ, CᵥH₂ᵥ, C_{w}H_{2w}, alcényle et alcynyle étant éventuellement substitués indépendamment les uns des autres et indépendamment des autres substituants par un ou plusieurs substituants fluor.

2. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon la revendication 1, dans lequel R¹ est choisi parmi alkyle en (C₁-C₆), cycloalkyle en (C₃-C₇)-CₜH₂ₜ- et Het-CₜH₂ₜ-, t étant choisi parmi 0, 1 et 2.

3. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon une ou plusieurs des revendications 1 et 2, dans lequel
R² est choisi parmi phényle et pyridinyle, l'atome d'azote de cycle du pyridinyle pouvant porter un substituant oxy et le phényle et le pyridinyle étant éventuellement substitués sur un ou plusieurs atomes de carbone de cycle par des substituants R²² identiques ou différents.

4. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon une ou plusieurs des revendications 1 à 3, dans lequel R³ est choisi parmi cycloalkyle en (C₃-C₇)-CᵤH₂ᵤ- et Het-CᵥH₂ᵥ-, u et v étant choisis parmi 1 et 2, ou R³ représente un radical d'un cycle monocyclique ou bicyclique, saturé ou insaturé, de 3 éléments à 10 éléments, qui contient 0, 1, 2, 3 ou 4 hétéroatomes de cycle identiques ou différents choisis parmi N, O et S, un ou deux des atomes d'azote de cycle pouvant porter un atome d'hydrogène ou un substituant alkyle en (C₁-C₄), et un ou deux des atomes de soufre de cycle pouvant porter un ou deux groupes oxo, et le radical d'un cycle étant éventuellement substitué sur un ou plusieurs atomes de carbone de cycle par des substituants R³¹ identiques ou différents.

5. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon une ou plusieurs des revendications 1 à 4, dans lequel
R¹ est choisi parmi alkyle en (C₁-C₆), cycloalkyle en (C₃-C₇)-CₜH₂ₜ- et Het-CₜH₂ₜ-, t étant choisi parmi 0, 1 et 2 ;
R² est choisi parmi phényle et pyridinyle, l'atome d'azote de cycle du pyridinyle pouvant porter un substituant oxy et le phényle et le pyridinyle étant éventuellement substitués sur un ou plusieurs atomes de carbone de cycle par des substituants R²² identiques ou différents ;
R³ est choisi parmi cycloalkyle en (C₃-C₇)-CᵤH₂ᵤ- et Het-CᵥH₂ᵥ-, u et v étant choisis parmi 1 et 2, ou R³ représente un radical d'un cycle monocyclique ou bicyclique, saturé ou insaturé, de 3 éléments à 10 éléments, qui contient 0, 1, 2, 3 ou 4 hétéroatomes de cycle identiques ou différents choisis parmi N, O et S, un ou deux des atomes d'azote de cycle pouvant porter un atome d'hydrogène ou un substituant alkyle en (C₁-C₄), et un ou deux des atomes de soufre de cycle pouvant porter un ou deux groupes oxo, et le radical d'un cycle étant éventuellement substitué sur un ou plusieurs atomes de carbone de cycle par des substituants R³¹ identiques ou différents.

6. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon une ou plusieurs des revendications 1 à 5, dans lequel
A est choisi parmi NH, O et S ;
R¹ est choisi parmi alkyle en (C₁-C₆), cycloalkyle en (C₃-C₇)-CₜH₂ₜ- et Het-CₜH₂ₜ-, t étant choisi parmi 0, 1 et 2 ;
R² est choisi parmi phényle et pyridinyle, l'atome d'azote de cycle du pyridinyle pouvant porter un substituant oxy, et le phényle et le pyridinyle étant éventuellement substitués sur un ou plusieurs atomes de carbone de cycle par des substituants R²² identiques ou différents ;
R³ est choisi parmi cycloalkyle en (C₃-C₇)-CᵤH₂ᵤ- et Het-CᵥH₂ᵥ-, u et v étant choisis parmi 1 et 2, ou R³ représente un radical d'un cycle monocyclique, saturé ou insaturé, de 3 éléments à 7 éléments, qui contient 0, 1 ou 2 hétéroatomes de cycle identiques ou différents choisis parmi N, O et S, un ou deux des atomes d'azote de cycle pouvant porter un atome d'hydrogène ou un substituant alkyle en (C₁-C₄), et un ou deux des atomes de soufre de cycle pouvant porter un ou deux groupes oxo, et le radical d'un cycle étant éventuellement substitué sur un ou plusieurs atomes de carbone de cycle par des substituants R³¹ identiques ou différents ;
R²² est choisi parmi halogène, hydroxy, alkyle en (C₁-C₄), alkyloxy en (C₁-C₄), alkyle en (C₁-C₄)-S(O)ₘ⁻, amino, cyano, aminocarbonyle, aminosulfonyle, R²³ et R²³-O- ;
R²³ représente un radical d'un cycle saturé, de 3 éléments à 6 éléments, monocyclique, qui contient 0, 1 ou 2 hétéroatomes de cycle identiques ou différents choisis parmi N, O et S, un ou deux des atomes d'azote de cycle pouvant porter un atome d'hydrogène ou un substituant alkyle en (C₁-C₄) et un des atomes de soufre de cycle pouvant porter un ou deux groupes oxo, et le radical d'un cycle étant éventuellement substitué sur un ou plusieurs atomes de carbone de cycle par des substituants R²⁴ identiques ou différents ;
R²⁴ est choisi parmi fluor, alkyle en (C₁-C₄) et hydroxy ;
R³¹ est choisi parmi halogène, alkyle en (C₁-C₄), cycloalkyle en (C₃-C₇), hydroxy, alkyloxy en (C₁-C₄), alkyl-S(O)ₘ en (C₁-C₄), amino, alkylamino en (C₁-C₄), di(alkyle en (C₁-C₄))amino, alkylcarbonylamino en (C₁-C₄), alkylsulfonylamino en (C₁-C₄), cyano, aminosulfonyle, alkylaminosulfonyle en (C₁-C₄) et di(alkyle en (C₁-C₄))aminosulfonyle ;
Het représente un radical d'un hétérocycle monocyclique saturé de 4 éléments à 7 éléments, qui contient 1 ou 2 hétéroatomes de cycle identiques ou différents choisis parmi N, O et S, et qui est relié par un atome de carbone de cycle, le radical d'un hétérocycle étant éventuellement substitué par un ou plusieurs substituants identiques ou différents choisis parmi fluor et alkyle en (C₁-C₄) ;
m est choisi parmi 0, 1 et 2, tous les nombres m étant indépendants les uns des autres ;
tous les groupes cycloalkyle étant éventuellement substitués indépendamment les uns des autres et indépendamment des autres substituants par un ou plusieurs substituants identiques ou différents choisis parmi fluor et alkyle en (C₁-C₄) ;
tous les groupes alkyle, CₜH₂ₜ, CᵤH₂ᵤ et CᵥH₂ᵥ étant éventuellement substitués indépendamment les uns des autres et indépendamment des autres substituants par un ou plusieurs substituants fluor.

7. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon une ou plusieurs des revendications 1 à 6, dans lequel A représente O.

8. Procédé de fabrication d'un composé de formule I selon l'une quelconque des revendications 1 à 7, selon lequel un composé de formule II est mis en réaction avec un composé de formule III 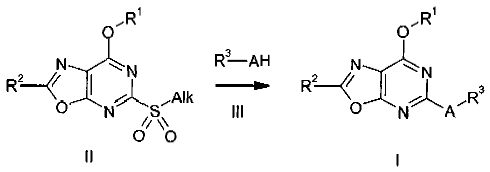 les groupes A, R¹, R² et R³ dans les composés de formules II et III étant tels que définis dans les composés de formule I et des groupes fonctionnels pouvant également être présents sous forme protégée ou sous la forme d'un groupe précurseur, et le groupe Alk représentant alkyle en (C₁-C₄).

9. Composition pharmaceutique, contenant au moins un composé de formule I selon l'une quelconque des revendications 1 à 7 ou un sel physiologiquement acceptable de celui-ci ou un solvate physiologiquement acceptable d'un tel composé ou d'un tel sel, et un véhicule pharmaceutiquement acceptable.

10. Composé de formule I selon l'une quelconque des revendications 1 à 7 ou sel physiologiquement acceptable de celui-ci ou solvate physiologiquement acceptable d'un tel composé ou d'un tel sel destiné à être utilisé en tant que médicament.

11. Composé de formule I selon l'une quelconque des revendications 1 à 7 ou sel physiologiquement acceptable de celui-ci ou solvate physiologiquement acceptable d'un tel composé ou d'un tel sel pour le traitement de troubles de la cicatrisation.

12. Composé de formule I selon l'une quelconque des revendications 1 à 7 ou sel physiologiquement acceptable de celui-ci ou solvate physiologiquement acceptable d'un tel composé ou d'un tel sel pour la cicatrisation.

13. Composé de formule I selon l'une quelconque des revendications 1 à 7 ou sel physiologiquement acceptable de celui-ci ou solvate physiologiquement acceptable d'un tel composé ou d'un tel sel pour la cicatrisation chez les diabétiques.

14. Composé de formule I selon l'une quelconque des revendications 1 à 7 ou sel physiologiquement acceptable de celui-ci ou solvate physiologiquement acceptable d'un tel composé ou d'un tel sel pour le traitement du syndrome du pied diabétique.
